# EUROPEAN PATENT APPLICATION

(11) **EP 4 019 636 A1**
(43) Date of publication of application: **29.06.2022**
(21) Application number: 21197081.9
(22) Date of filing: 21.12.2012
(51) Int. Cl.: C12N 15/113, C07K 14/005, C12N 7/04

(54) **PROCESSES USING VLPS WITH CAPSIDS RESISTANT TO HYDROLASES**

(30) Priority: 21.12.2011 US 201161578706 P; 07.03.2012 US 201261607900 P; 19.06.2012 US 201261661688 P
(62) Divisional of application: 12860313.1
(71) Applicant: APSE LLC, St. Louis, MO 63108 (US)
(72) Inventor: ARHANCET, Juan, Pedro, Humberto, St. Louis, MO 63108 (US); ARHANCET, Juan, P., St. Louis, MO 63108 (US); DELANEY, Kimberly, St. Louis, MO 63108 (US); HALL, Kathleen, B., St. Louis, MO 63108 (US); SUMMERS, Neena, St. Louis, MO 63108 (US)
(74) Representative: Nederlandsch Octrooibureau

(57) **Abstract**

Novel processes and compositions are described which use viral capsid proteins resistant to hydrolases to prepare virus-like particles to enclose and subsequently isolate and purify target cargo molecules of interest including nucleic acids such as siRNA's and shRNA's, and small peptides.

## Description

### CROSS REFERENCE TO RELATED APPLICATIONS

This application claims the priority of U.S. provisional application number 61/578,706, filed December 21, 2011, U.S. provisional application number 61/607,900, filed March 7, 2012, and U.S. provisional application number 61/661,688, filed June 19, 2012, the entire disclosures of which are hereby incorporated by reference.

### INCORPORATION OF SEQUENCE LISTING

The entire contents of a paper copy of the "Sequence Listing" and a computer readable form of the sequence listing on diskette, containing the file named 450061_SequenceListing_ST25.txt, which is 77 kilobytes in size and was created on December 20, 2012, are herein incorporated by reference.

### TECHNICAL FIELD

The invention relates to virus-like particles, and in particular to methods and compositions using viral capsids as nanocontainers for producing, isolating and purifying heterologous nucleic acids and proteins.

### BACKGROUND OF THE INVENTION

Virus-like particles (VLPs) are particles derived in part from viruses through the expression of certain viral structural proteins which make up the viral envelope and/or capsid, but VLPs do not contain the viral genome and are non-infectious. VLPs have been derived for example from the Hepatitis B virus and certain other viruses, and have been used to study viral assembly and in vaccine development.

Viral capsids are composed of at least one protein, several copies of which assemble to form the capsid. In some viruses, the viral capsid is covered by the viral envelope. Such viral envelopes are comprised of viral glycoproteins and portions of the infected host's cell membranes, and shield the viral capsids from large molecules that would otherwise interact with them. The capsid is typically said to encapsidate the nucleic acids which encode the viral genome and sometimes also proteins necessary for the virus' persistence in the natural environment. For the viral genome of a virus to enter a new host, the capsid must be disassembled. Such disassembly happens under conditions normally used by the host to degrade its own as well as foreign components, and most often involves proteolysis. Viruses take advantage of normal host processes such as proteolytic degradation to enable that critical part of their cycle, *i.e.* capsid disassembly and genome release.

It is therefore unsurprising that the literature has not previously described capsids resistant to hydrolases that act on peptide bonds. A very limited number of certain specific peptide sequences which are part of larger proteins are known to be somewhat resistant to certain proteases, but the vast majority of peptide sequences are not. Viruses that resist proteolysis have been reported, but these are all enveloped viruses, in which the capsid is shielded by the viral envelope. In such viruses the capsids are not in contact with, *i.e.* they are shielded from, the proteases described. Thus the role, if any, of the proteolytical stability of the virus capsid in such cases is unknown.

In large-scale manufacturing of recombinant molecules such as proteins, ultrafiltration is often used to remove molecules smaller than the target protein in the purification steps leading to its isolation. Purification methods also often involve precipitation, solvent extraction, and crystallization techniques. These separation techniques are inherently simple and low cost because, in contrast to chromatography, they are not based on surface but on bulk interactions. However, these techniques are typically limited to applications to simple systems, and by the need to specify a different set of conditions for each protein and expression system. Yet each target recombinant protein presents a unique set of binding interactions, thereby making its isolation process unique and complex. The separation efficiency for recombinant proteins using these simple isolation processes is therefore low.

Nucleic acids, including siRNA and miRNA, have for the most part been manufactured using chemical synthesis methods. These methods are generally complex and high cost because of the large number of steps needed and the complexity of the reactions which predispose to technical difficulties, and the cost of the manufacturing systems. In addition, the synthetic reagents involved are costly and so economy of scale is not easily obtained by simply increasing batch size.

### BRIEF SUMMARY OF THE INVENTION

In one aspect the present disclosure provides a virus-like particle (VLP) comprising a capsid enclosing at least one heterologous cargo molecule and a packing sequence. A VLP may further comprise at least one ribozyme enclosed by the capsid. The heterologous cargo molecule may comprise an oligonucleotide, or an oligoribonucleotide. A VLP may comprise a one or more ribozymes, and a ribozyme may be flanked by the packing sequence and the oligoribonucleotide to form a nucleic acid construct. A VLP may comprise a plurality of the nucleic acid constructs. In a VLP comprising an oligoribonucleotide, oligoribonucleotide may be a short RNA selected from siRNA, shRNA, sshRNA, IshRNA and miRNA. A VLP may comprise at least two ribozymes, wherein each ribozyme is selected to cut one end of the short RNA. A VLP may further comprise a linker consisting of at least 1 to 100 nucleotides, the linker comprising at least 40% A's or at least 40% U's, wherein the linker links the oligoribonucleotide and the packing sequence, or the oligoribonucleotide and the ribozyme. Ribozymes may be selected for example from a Hammerhead ribozyme and a Hepatitis Delta V ribozyme. A Hammerhead ribozyme may be a Hammerhead ribozyme variant having a contiguous set of nucleotides complementary to at least 6 contiguous nucleotides of the oligoribonucleotide. Alternatively, the ribozyme may be a mutant Hepatitis Delta V ribozyme capable of cleaving its connection with the oligoribonucleotide at a rate at most about 50% the rate of a wildtype Hepatitis Delta V ribozyme. Such a mutant HDV ribozyme may have for example a nucleic acid sequence selected from SEQ ID Nos: 10-18.

VLP's according to the present disclosure may comprise a capsid which comprises a wild type viral capsid which is resistant to hydrolysis catalyzed by a peptide bond hydrolase category EC 3.4, or a capsid protein having at least 15%, at least 16%, at least 21%, at least 40%, at least 41%, at least 45%, at least 52%, at least 53%, at least 56%, at least 59% or at least 86% sequence identity with the amino acid sequence of wild type Enterobacteria phage MS2 capsid (SEQ ID NO : 3) and is resistant to hydrolysis catalyzed by a peptide bond hydrolase category EC 3.4. The capsid may comprise a wild type Enterobacteria phage MS2 capsid protein having the amino acid sequence of SEQ ID NO: 3.

VLP's according to the present disclosure may comprise a heterologous cargo molecule comprising a peptide or polypeptide. A VLP may further comprise an oligonucleotide linker coupling the heterologous cargo peptide or polypeptide molecule and the viral capsid. The oligonucleotide linker may be an oligoribonucleotide comprising a ribozyme sequence. Alternatively, the heterologous cargo molecule may comprise a bi-molecular cargo molecule comprising a bifunctional polynucleotide comprising a first aptamer sequence which specifically binds a bioactive small molecule having a molecular weight of about 1,500 Da or less and a second aptamer sequence for binding a packing sequence of the capsid. The VLP may further comprise the bioactive small molecule bound to the first aptameric sequence. The bioactive small molecule may comprise and herbicide or a pesticide, which may selected for example from atrazine, acetamipridphorate, profenofos, isocarbophos and omethoateas.

In another aspect, the present disclosure provides a nucleic acid construct comprising a nucleotide sequence that encodes a short RNA, a ribozyme and a packing sequence. The short RNA may be for example an siRNA or an shRNA. The nucleic acid construct may further comprise a linking nucleotide sequence of 4 to 100 nucleotides of which at least 40% are A's or at least 40% are T's, wherein the linking nucleotide sequence is flanked by the packing-coding sequence and by the short RNA-coding sequence. The nucleic acid construct may further comprise a linking nucleotide sequence of 4 to 100 nucleotides of which at least 40% are A's or at least 40% are U's, wherein the linking nucleotide sequence is flanked by the ribozyme and the short RNA-encoding sequence. The ribozyme sequence may be flanked by the short RNA and the packing sequence. The present disclosure also encompasses a vector comprising any such nucleic acid constructs, and host cells comprising such a vector , as well as host cell stably transformed with such a vector. Host cells may be a bacterial cell, such as but not limited to an *Escherichia coli* cell, a plant cell, a mammalian cell, an insect cell, a fungal cell or a yeast cell. A host cell may further be stably transfected with a second vector comprising a second nucleic acid sequence encoding a viral capsid which is resistant to hydrolysis catalyzed by a peptide bond hydrolase category EC 3.4. The second nucleic acid sequence may encode for example a viral protein encoding a viral capsid having at least 40% sequence identity with the amino acid sequence of wild type Enterobacteria phage MS2 capsid protein (SEQ ID NO: 3) and is resistant to hydrolysis catalyzed by a peptide bond hydrolase category EC 3.4. A nucleic acid construct as described herein may also encode a wild type Enterobacteria phage MS2 capsid protein (SEQ ID NO: 3). The ribozyme in such a nucleic acid construct may be for example a Hammerhead ribozyme, .a Hammerhead ribozyme variant having a contiguous set of nucleotides complementary to at least 6 contiguous nucleotides of the short RNA, a Hepatitis Delta V ribozyme, or a mutant Hepatitis Delta V ribozyme capable of cleaving its connection with the short RNA at a rate at most 50% the rate of a wildtype Hepatitis Delta V ribozyme. An non-limiting but exemplary mutant HDV ribozyme has a nucleic acid sequence selected from SEQ ID NOs: 10-18. The present disclosure also encompasses a plant or plant tissue transformed to contain a nucleic acid construct described herein, and seed or progeny of such a plant or plant tissue, wherein the seed or progeny comprises the nucleic acid construct.

In another aspect, the present disclosure provides a composition comprising: a) a plurality of virus-like particles each comprising a viral capsid enclosing at least one heterologous cargo molecule; and b) one or more cell lysis products present in an amount of less than 4 grams for every 100 grams of capsid present in the composition, wherein the cell lysis products are selected from proteins, polypeptides, peptides and any combination thereof. In the composition, the capsid is for example resistant to hydrolysis catalyzed by a peptide bond hydrolase category EC 3.4. The capsid may comprise a capsid protein having at least 15%, at least 16%, at least 21%, at least 40%, at least 41%, at least 45%, at least 52%, at least 53%, at least 56%, at least 59% or at least 86% sequence identity with the amino acid sequence of wild type Enterobacteria phage MS2 capsid (SEQ ID NO: 3) and is resistant to hydrolysis catalyzed by a peptide bond hydrolase category EC 3.4. The capsid may comprises a wild type Enterobacteria phage MS2 capsid protein (SEQ ID NO: 3). In the composition, the heterologous cargo molecule may comprise an oligonucleotide which may be an oligoribonucleotide. An oligoribonucleotide may be selected for example from siRNA, shRNA, sshRNA, IshRNA and miRNA. In the composition, each virus-like particle may further comprise at least one ribozyme, wherein the ribozyme is flanked by the packing sequence and the oligoribonucleotide to form a nucleic acid construct, and each virus-like particle may comprise a plurality of the nucleic acid constructs. In the VLP's of such a composition, the ribozyme may be for example a Hammerhead ribozyme, .a Hammerhead ribozyme variant having a contiguous set of nucleotides complementary to at least 6 contiguous nucleotides of the short RNA, a Hepatitis Delta V ribozyme, or a mutant Hepatitis Delta V ribozyme capable of cleaving its connection with the short RNA at a rate at most 50% the rate of a wildtype Hepatitis Delta V ribozyme. An non-limiting but exemplary mutant HDV ribozyme has a nucleic acid sequence selected from SEQ ID NOs: 10-18. The VLP's in such a composition may further comprise a linking nucleotide sequence of 4 to 100 nucleotides of which at least 40% are A's or at least 40% are T's, wherein the linking nucleotide sequence is flanked by the packing-coding sequence and by the short RNA-coding sequence, or a linking nucleotide sequence of 4 to 100 nucleotides of which at least 40% are A's or at least 40% are U's, wherein the linking nucleotide sequence is flanked by the ribozyme and the short RNA-encoding sequence. The ribozyme sequence may be flanked by the short RNA and the packing sequence. The VLP's in such a composition may f comprise a heterologous cargo molecule comprising a peptide or polypeptide. Such VLP's in a composition may further comprise an oligonucleotide linker coupling the heterologous cargo molecule and the viral capsid. The oligonucleotide linker may be an oligoribonucleotide comprising a ribozyme sequence. In such a composition, the cell lysis products may be present in an amount of less than 0.5 grams, less than 0.2 grams or less than 0.1 grams.

In another aspect, the present disclosure provides method for isolating and purifying a target cargo molecule, the method comprising: (a) obtaining a whole cell lysate comprising a plurality of virus-like particles (VLPs) each comprising a capsid enclosing at least one target cargo molecule, wherein the capsids are resistant to hydrolysis catalyzed by a peptide bond hydrolase category EC 3.4; (b) subjecting the VLP's to hydrolysis using a peptide bond hydrolase category EC 3.4, for a time and under conditions sufficient for at least 60, at least 70, at least 80, or at least 90 of every 100 individual polypeptides present in the whole cell lysate but not enclosed by the capsids to be cleaved, while at least 60, at least 70, at least 80, or at least 90 of every 100 capsids present in the whole cell lysate before such hydrolysis remain intact following the hydrolysis. In the method, the capsids may each comprises a viral capsid protein having at least 15%, at least 16%, at least 21%, at least 40%, at least 41%, at least 45%, at least 52%, at least 53%, at least 56%, at least 59% or at least 86% sequence identity with the amino acid sequence of wild type Enterobacteria phage MS2 capsid protein (SEQ ID NO: 3) and is resistant to hydrolysis catalyzed by a peptide bond hydrolase category EC 3.4. The capsids may each comprise a wild type Enterobacteria phage MS2 capsid protein (SEQ ID NO: 3). In the method, the heterologous cargo molecule may comprise an oligonucleotide which may be an oligoribonucleotide, or a peptide or a polypeptide. An oligoribonucleotide may be selected for example from siRNA, shRNA, sshRNA, IshRNA and miRNA. In the method, each virus-like particle may further comprise a ribozyme, wherein the ribozyme is flanked by the packing sequence and the oligoribonucleotide to form a nucleic acid construct. The method may further comprise purification of the capsids following hydrolysis. Purification may include at least one of a liquid-liquid extraction step, a crystallization step, a fractional precipitation step, and an ultra filtration step. The present disclosure also encompasses a composition produced by such a method.

In another aspect, the present disclosure provides a method for protecting a target molecule from hydrolysis in a whole cell lyste following intracellular production of the target molecule in a host cell, the method comprising: (a) selecting a viral capsid which is resistant to hydrolysis catalyzed by a peptide bond hydrolase category EC 3.4; (b) stably transfecting the host cell with a first vector comprising a nucleic acid sequence encoding a viral protein forming the viral capsid, and a second vector comprising a nucleic acid sequence comprising a ribozyme flanked by a packing sequence and an siRNA sequence; and (c) maintaining the cells for a time and under conditions sufficient for the transformed cells to express and assemble capsids encapsidating the ribozyme flanked by the packing sequence and the siRNA sequence. In the process, the capsids may each comprises a viral capsid protein having at least 15%, at least 16%, at least 21%, at least 40%, at least 41%, at least 45%, at least 52%, at least 53%, at least 56%, at least 59% or at least 86% sequence identity with the amino acid sequence of wild type Enterobacteria phage MS2 capsid protein (SEQ ID NO: 3) and is resistant to hydrolysis catalyzed by a peptide bond hydrolase category EC 3.4.

In another aspect, the present disclosure provides a process for purifying VLP's enclosing at least one heterologous cargo molecule, the process comprising: (a) obtaining a cell lysate comprising a plurality of the VLP's; (b) contacting the cell lysate with a protease for a time and under conditions sufficient to hydrolyze cell lysis products other than the VLP's to form a hydrolysate; and (c) isolating the VLP's from the hydrolsyate. Step (c) may comprise (i) performing a first precipitation with ammonium sulfate followed by a first centrifugation to obtain a first precipitate and a first supernatant; and (ii) performing a second precipitation on the first supernatant with ammonium sulfate followed by a second centrifugation to obtain a second precipitate, wherein the second precipitate comprises at least about 70%, 80% or 90% by weight of the VLP's. Step (c) may comprise (i) performing a first precipitation with ethanol followed by a first centrifugation to obtain a first precipitate and a first supernatant; and (ii) performing a second precipitation on the first supernatant with ammonium sulfate followed by a second centrifugation to obtain a second precipitate, wherein the second precipitate comprises at least about 70%, 80% or 90% by weight of the VLP's. Step (c) may comprise ultracentrifuging the hydrolysate to obtain a precipitate comprising at least about 70%, 80% or 90% by weight of the VLP's. In the process, the VLP's may each comprise a capsid which is resistant to hydrolysis catalyzed by a peptide bond hydrolase category EC 3.4., which can comprise a capsid protein having at least 15%, at least 16%, at least 21%, at least 40%, at least 41%, at least 45%, at least 52%, at least 53%, at least 56%, at least 59% or at least 86% sequence identity with the amino acid sequence of wild type Enterobacteria phage MS2 capsid (SEQ ID NO: 3) and is resistant to hydrolysis catalyzed by a peptide bond hydrolase category EC 3.4. Te VLP's may each comprise a wild type Enterobacteria phage MS2 capsid protein (SEQ ID NO: 3). In the process, step (b) can be performed for at least about 30 minutes at about 37°C. The process may further comprise, before step (b), contacting the cell lysate with at least one of a nuclease, an amylase and a lypase for at least about 30 minutes at about 37°C. In the process, the protease can be fore example a peptide bond hydrolase category EC 3.4, which can be selected for example from Proteinase K, Protease from *Streptomyces griseus,* Protease from *Bacillus lichenformis,* pepsin and papain. In the process, the heterologous cargo molecule enclosed by the VLP's may comprise an oligonucleotide which may be an oligoribonucleotide, or a peptide or a polypeptide. An oligoribonucleotide may be selected for example from siRNA, shRNA, sshRNA, IshRNA and miRNA. In the process, the VLP's may each further comprise a ribozyme as described herein, flanked by a packing sequence and the oligoribonucleotide to form a nucleic acid construct. The oligoribonucleotide and the packing sequence may be linked by a linker sequence of at least 1 to 100 nucleotides, and comprising more than 40% A's, more than 40% of U's, or more than 40% T's. The process may further comprise preparing the cell lyaste before step (a) by centrifuging cells following expression of the VLPs in the cells; resuspending the cells; lysing the cells and centrifuging the cell lysate to obtain a supernatant, wherein the supernatant is used as the cell lysate for step (a).

In another aspect, the present disclosure provides VLP's comprising a capsid enclosing at least one heterologous cargo molecule and a packing sequence wherein the capsid comprises a capsid protein which is a variant of wild type Enterobacteria phage MS2 capsid (SEQ ID NO: 3). The capsid protein may be one which has the amino acid sequence of wild type Enterobacteria phage MS2 capsid (SEQ ID NO: 3) except that the A residue at position 1 is deleted, and is resistant to hydrolysis catalyzed by a peptide bond hydrolase category EC 3.4. The capsid protein may be one which has the amino acid sequence of wild type Enterobacteria phage MS2 capsid (SEQ ID NO:3) except that the A residue at position 1 is deleted and the S residue at position 2 is deleted, and is resistant to hydrolysis catalyzed by a peptide bond hydrolase category EC 3.4. The capsid protein may be one which has the amino acid sequence of wild type Enterobacteria phage MS2 capsid (SEQ ID NO: 3) except that the A residue at position 1 is deleted, the S residue at position 2 is deleted and the N residue at position 3 is deleted, and is resistant to hydrolysis catalyzed by a peptide bond hydrolase category EC 3.4. The capsid protein may be one which has the amino acid sequence of wild type Enterobacteria phage MS2 capsid (SEQ ID NO: 3) except that the Y reside at position 129 is deleted, and is resistant to hydrolysis catalyzed by a peptide bond hydrolase category EC 3.4. The capsid protein may be one which has the amino acid sequence of wild type Enterobacteria phage MS2 capsid (SEQ ID NO:3) but having a single (1) amino acid deletion in the 112-117 segment and is resistant to hydrolysis catalyzed by a peptide bond hydrolase category EC 3.4. The capsid protein may be one which has the amino acid sequence of wild type Enterobacteria phage MS2 capsid (SEQ ID NO:3) but having a single (1) amino acid deletion in the 112-117 segment and is resistant to hydrolysis catalyzed by a peptide bond hydrolase category EC 3.4. The capsid protein may be one which has the amino acid sequence of wild type Enterobacteria phage MS2 capsid (SEQ ID NO:3) but having a 1-2 residue insertion in the 65-83 segment and is resistant to hydrolysis catalyzed by a peptide bond hydrolase category EC 3.4. The capsid protein may be one which has the amino acid sequence of wild type Enterobacteria phage MS2 capsid (SEQ ID NO:3) but having a 1-2 residue insertion in the 44-55 segment and is resistant to hydrolysis catalyzed by a peptide bond hydrolase category EC 3.4. The capsid protein may be one which has the amino acid sequence of wild type Enterobacteria phage MS2 capsid (SEQ ID NO:3) but having a single (1) residue insertion in the 33-43 segment and is resistant to hydrolysis catalyzed by a peptide bond hydrolase category EC 3.4. The capsid protein may be one which has the amino acid sequence of wild type Enterobacteria phage MS2 capsid (SEQ ID NO:3) but having a 1-2 residue insertion in the 24-30 segment and is resistant to hydrolysis catalyzed by a peptide bond hydrolase category EC 3.4. The capsid protein may be one which has the amino acid sequence of wild type Enterobacteria phage MS2 capsid (SEQ ID NO :3) but having a single (1) residue insertion in the 10-18 segment and is resistant to hydrolysis catalyzed by a peptide bond hydrolase category EC 3.4. The capsid may comprise a capsid protein monomer sequence concatenated with a second capsid monomer sequence which assembles into a capsid which resistant to hydrolysis catalyzed by a peptide bond hydrolase category EC 3.4. The capsid may comprise a capsid protein monomer sequence whose C-terminus is extended with a 0-6 residue linker segment whose C-terminus is concatenated with a second capsid monomer sequence, all of which assembles into a capsid which resistant to hydrolysis catalyzed by a peptide bond hydrolase category EC 3.4. A linker segment may have a sequence such as, for example, - (Gly)ₓ, wherein x=0-6. including -Gly-; -Gly-Gly-; and -Gly-Gly-Gly-. A linker segment may be a Gly-Ser linker selected from -Gly-Gly-Ser-Gly-Gly-, -Gly-Gly-Ser and -Gly-Ser-Gly- The capsid may comprise the capsid protein concatenated with a third capsid monomer sequence which assembles into a capsid which resistant to hydrolysis catalyzed by a peptide bond hydrolase category EC 3.4. The capsid may comprise a capsid protein wherein the C-terminus is extended with a 0-6 residue linker segment whose C-terminus s concatenated with a third capsid monomer sequence, all of which assembles into a capsid which resistant to hydrolysis catalyzed by a peptide bond hydrolase category EC 3.4. The capsid may comprise a capsid protein wherein the capsid comprises a capsid protein in which one or both linker sequences is - (Gly)ₓ, wherein x=0-6, including -Gly-; -Gly-Gly-; and -Gly-Gly-Gly-. A linker segment may be a Gly-Ser linker selected from -Gly-Gly-Ser-Gly-Gly-, -Gly-Gly-Ser and -Gly-Ser-Gly-.

Such a capsid protein assembles for example into a capsid which is resistant to hydrolysis catalyzed by a peptide bond hydrolase category EC 3.4. For example, the capsid may comprise a capsid protein in which one or both linker sequences is -(Gly)x-, x=1, which assembles into a capsid which is resistant to hydrolysis catalyzed by a peptide bond hydrolase category EC 3.4. The capsid may comprise a capsid protein in which one or both linker sequences is -(Gly)x-, x=2, which assembles into a capsid which is resistant to hydrolysis catalyzed by a peptide bond hydrolase category EC 3.4. The capsid may comprise a capsid protein in which one or both linker sequences is -(Gly)x-, x=3, which assembles into a capsid which is resistant to hydrolysis catalyzed by a peptide bond hydrolase category EC 3.4. The capsid may comprise one or more coat protein sequences which is N-terminally truncated by 1-3 residues and a linker segment as described herein is lengthened by the number of residues deleted, and which is resistant to hydrolysis catalyzed by a peptide bond hydrolase category EC 3.4. The capsid may comprise one or more coat protein sequences which is C-terminally truncated by 1 residue, and linker segments as described herein are lengthened by the one residue, wherein the capsid is resistant to hydrolysis catalyzed by a peptide bond hydrolase category EC 3.4. The capsid may comprise a first coat protein sequence in a concatenated dimer which is C-terminally truncated by 1 residue and the linker segments lengthened by the one residue or wherein the first and/or second coat protein sequence in a concatenated trimer is C-terminally truncated by 1 residues and which is resistant to hydrolysis catalyzed by a peptide bond hydrolase category EC 3.4. The capsid may comprise a capsid protein having N- and C-terminal truncations and which is resistant to hydrolysis catalyzed by peptide bond hydrolase category EC 3.4.

### REFERENCE TO COLOR FIGURES

The application file contains at least one photograph executed in color. Copies of this patent application publication with color photographs will be provided by the Office upon request and payment of the necessary fee.

### BRIEF DESCRIPTION OF THE DRAWINGS

**Figure 1** is a plot of Optical Density (OD; filled diamonds) and pH (open squares) over time, showing propagation of wild type MS2 bacteriophage (ATCC No.15597-B1, from American Type Culture Collection, Rockville, MD) in its *E. Coli* host (ATCC No. 15669).
**Figure 2** is a gel showing results of SDS-PAGE analysis of MS2 bacteriophage samples obtained following propagation in *E. Coli* and purified using Proteinase K and ultrafiltration, showing that Proteinase K purification yields phage purified to higher than 99% (band at 14 kDa corresponds to MS2 bacteriophage coat protein).
**Figure 3** is a gel showing results of SDS-PAGE analysis of partially purified MS2, showing complete degradation of the phage and results obtained after 1x or 2x ultrafiltration of the lysate (Lanes 4 and 6).
**Figure 4** is a gel showing results of SDS-PAGE analysis of MS2 samples purified using ultrafiltration and Proteinase K treatment.
**Figure 5** is a gel showing results of SDS-PAGE analysis of MS2 samples purified using Proteinase K treatment, precipitation at acidic conditions, precipitation using ethanol at basic and acidic conditions, and ultrafiltration.
**Figure 6** is a graph showing the UV spectrum of MS2 samples purified using Proteinase K treatment, precipitation at acidic conditions, precipitation using ethanol at basic and acidic conditions, and ultrafiltration.
**Figure 7** is a chromatograph of PCR products obtained from an MS2 sample following purification described for Figures 5 and 6, chromatographed in 1.5% agarose gel stained with Ethidium Bromide (1.2 kbp for primers F1201_1223-R1979_2001 in Lane 1, 800 bp for primers F1201_1223-R1979_2001 in Lane 2, and 304 bp for primers F1401_1426-R1680_1705 in Lane 3), showing consistency with an intact MS2 bacteriophage genome.
**Figure 8** is a plot of Optical Density (OD; filled diamonds) over time, obtained with a control sample (open diamonds) and an MS2 sample following purification described for Figures 5 and 6 (filled squares), showing that the purified sample contained phage that retained high infectivity.
**Figure 9** is a gel showing results of SDS-PAGE analysis of MS2 samples following expression of MS2 capsids encapsidating RNA coding for the coat protein attached to a coat- specific 19-mer RNA hairpin.
**Figure 10** is a chromatograph of PCR products from PCR interrogation of an MS2 sample for presence or absence of a section of the MS2 capsid following purification, chromatographed in 2% agarose gel stained with Ethidium Bromide (304 bp in Lane 1; the leftmost Lane corresponds to 1 kb plus ladder from Life Technologies), showing consistency with an intact MS2 coat gene.
**Figure 11** is a gel showing results of SDS-PAGE analysis of MS2 samples following simple precipitation with ethanol for purification of MS2 Virus-Like Particles (VLPs).
**Figure 12** is a gel showing results of SDS-PAGE analysis of MS2 samples following use of Proteinase K (PK) and simple precipitation with ethanol for purification of MS2 VLPs.
**Figure 13** is a gel showing results of SDS-PAGE analysis of MS2 samples following use of constitutive hydrolases (CH), fractional precipitation with ethanol, and ultrafiltration for purification of MS2 VLPs.
**Figure 14** is a gel showing results of SDS-PAGE analysis of MS2 samples following use of various hydrolases, and factional precipitation with ammonium sulfate for purification of MS2 VLPs.
**Figure 15** is a gel showing results of PAGE analysis of RNA obtained from RNA encapsidated in MS2 capsids.
**Figure 16** is a gel showing results of PAGE analysis of RNA products produced following *in vitro* transcriptions using Hepatitis Delta Virus (HDV) ribozyme.
**Figure 17** is a gel showing results of PAGE analysis of siRNA products obtained during *in vitro* transcriptions using long flanking Hammerhead ribozymes.
**Figure 18** is a gel showing results of PAGE analysis of RNA products obtained from RNA encapsidated in VLPs, following purification of the VLP's and isolation of the RNA from the VLPs.
**Figure 19** is a series of gels showing results of SDS-PAGE analyses of VLP's comprising MS2 capsids, following purification and suspension of the VLPs, and exposure to various proteases for 1 hour and 4 hours of incubation.
**Figure 20** is an alignment of selected enterobacteria phage MS2 capsid proteins.
**Figure 21** is an alignment of complete leviviridae viral coat protein sequences retrieved from the UniProt database and aligned using their BLAST multiple alignment with default values for weighting array choice, gap penalties, etc.
**Figure 22** is a graphic illustration of backbone superposition of 1AQ3 chain B (leviviridae coat protein monomer) with 1QBE chain C (alloleviridae coat protein monomer).
**Figure 23** is a graphic illustration of an alternative view of the backbone superposition of 1AQ3 chain B (leviviridae coat protein monomer) with 1QBE chain C (alloleviridae coat protein monomer) shown in Figure 22.
**Figure 24** is a graphic illustration of another alternative view of the backbone superposition of 1AQ3 chain B (leviviridae coat protein monomer) with 1QBE chain C (alloleviridae coat protein monomer) shown in Figure 22.
**Figure 25** is a graphic illustration of another alternative view of the backbone superposition of 1AQ3 chain B (leviviridae coat protein monomer) with 1QBE chain C (alloleviridae coat protein monomer) shown in Figure 22.
**Figure 26** is a structural sequence alignment of 1AQ3, 2VTU and 1QBE.using jFATCAT rigid.
**Figure 27** is an alignment of complete alloleviviridae viral coat protein sequences retrieved from the UniProt database and aligned using their BLAST multiple alignment with default values for weighting array choice, gap penalties, etc.
**Figure 28** is a graphic illustration showing another 60 of the 180 monomers forming the isosahedral levi- and alloleviviridae capsid. The backbone of each monomer of represented by a ribbon of a different color. Backbone hydrogen bonds are represented by cyan lines. The isosahedral three-fold axis is in the center of the figure. Monomer-monomer contacts do not fill the central circle outlined by hydrogen bonds connecting the tips of flexible loops 67-81.

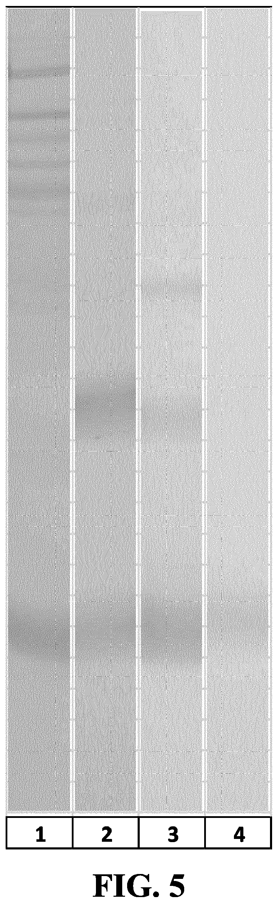

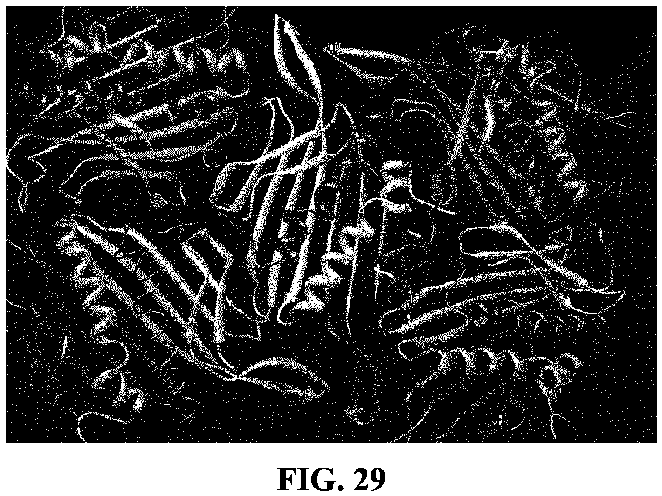

**Figure 29** is a graphic illustration showing 2 MS2 monomers (ribbons representing backbone colored dark and pale blue) surrounded by monomers in contact in the isosahedral capsid (ribbons representing monomer backbones in brown and navy). The alloleviviridae Qbeta has a two residue deletion with respect to leviviridae between residues 72 & 73 (red, bottom center). The central void is immediately below this deletion site (Figure 5). The deletion causes it to slightly expand. The Qbeta deletion at 126 (red central left) removes the excursion from the segment but extensive contacts between the sheets of neighboring monomers essentially holds the monomers in place. MS2 sequence numbering is used.

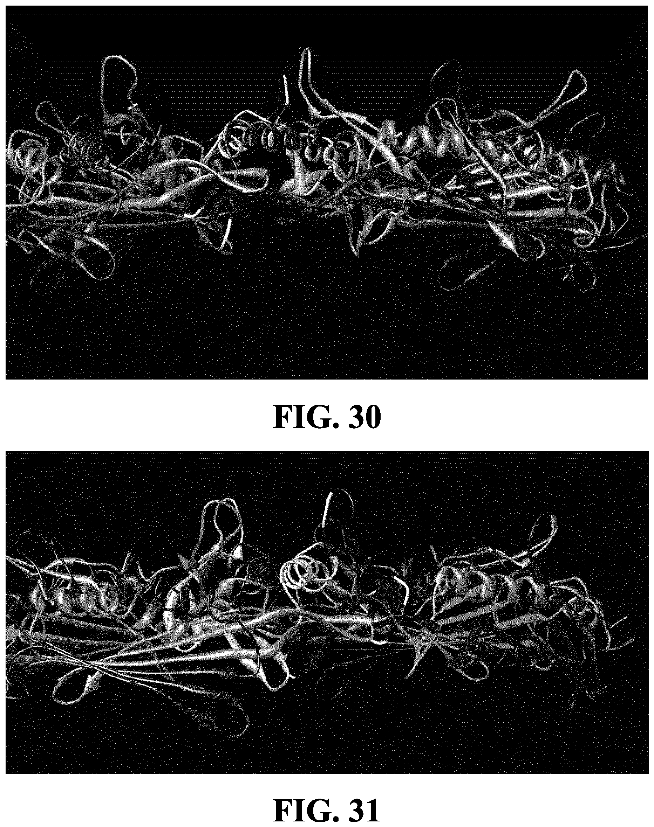

**Figure 30** is a graphic illustration of 2 MS2 monomers (ribbons representing backbone colored dark and pale blue) surrounded by monomers in contact in the isosahedral capsid (ribbons representing monomer backbones in brown and navy). The alloleviviridae Qbeta has a one residue insertion with respect to leviviridae between residues 12 & 13 (yellow, top left center), a flexible loop that extends from the outer capsid surface into solvent; a two residue insertion between residues 53 & 54 (yellow, lower left central) at the end a stand connection extending into the interior cargo; a one-residue insertion between residues 27 & 28 is also at the end of a beta-strand connector extending into the cpsid cargo space. None of these insertions require movement in the monomer fold or between neighbors
**Figure 31** is a graphic illustration of 2 MS2 monomers (ribbons representing backbone colored dark and pale blue) surrounded by monomers in contact in the isosahedral capsid (ribbons representing monomer backbones in brown and navy). The alloleviviridae Qbeta has a one residue insertion with respect to leviviridae between residues 36 & 37 (yellow, center right). The loop packs against the end of the adjacent helix but inserted residues can extend into the central space above the flexible loop immediately below.
**Figure 32** is a graphic illustration of backbone ribbons of 3 noncovalent Enterobacteria phage MS2 noncovalent dimers packed around a symmetry point in the assembled capsid, with all of the N-termini colored green, the C-termini red.

### DETAILED DESCRIPTION OF THE INVENTION

Section headings as used in this section and the entire disclosure herein are not intended to be limiting.

### A. Definitions

As used herein, the singular forms "a," "an" and "the" include plural referents unless the context clearly dictates otherwise. For the recitation of numeric ranges herein, each intervening number there between with the same degree of precision is explicitly contemplated. For example, for the range 6-9, the numbers 7 and 8 are contemplated in addition to 6 and 9, and for the range 6.0-7.0, the numbers 6.0, 6.1, 6.2, 6.3, 6.4, 6.5, 6.6, 6.7, 6.8, 6.9 and 7.0 are explicitly contemplated.

The use of "or" means "and/or" unless stated otherwise. Furthermore, the use of the term "including", as well as other forms, such as "includes" and "included", is not limiting.

Unless otherwise defined herein, scientific and technical terms used in connection with the present disclosure shall have the meanings that are commonly understood by those of ordinary skill in the art. For example, any nomenclatures used in connection with, and techniques of, animal and cellular anatomy, cell and tissue culture, biochemistry, molecular biology, immunology, and microbiology described herein are those that are well known and commonly used in the art. The meaning and scope of the terms should be clear; in the event however of any latent ambiguity, definitions provided herein take precedent over any dictionary or extrinsic definition. Further, unless otherwise required by context, singular terms shall include pluralities and plural terms shall include the singular.

A wide variety of conventional techniques and tools in chemistry, biochemistry, molecular biology, and immunology are employed and available for practicing the methods and compositions described herein, are within the capabilities of a person of ordinary skill in the art and well described in the literature. Such techniques and tools include those for generating and purifying VLP's including those with a wild type or a recombinant capsid together with the cargo molecule(s), and for transforming host organisms and expressing recombinant proteins and nucleic acids as described herein. *See, e.g.,* MOLECULAR CLONING, A LABORATORY MANUAL 2nd ed. 1989 (Sambrook et al., Cold Spring Harbor Laboratory Press); and CURRENT PROTOCOLS IN MOLECULAR BIOLOGY (Eds. Ausubel et al., Greene Publ. Assoc., Wiley-Interscience, NY) 1995. The disclosures in each of these are herein incorporated by reference.

As used herein, the term "cargo molecule" refers to an oligonucleotide, polypeptide or peptide molecule, which is or may be enclosed by a capsid.

As used herein, the term "oligonucleotide" refers to a short polymer of at least two, and no more than about 70 nucleotides, preferably no more than about 55 nucleotides linked by phosphodiester bonds. An oligonucleotide may be an oligodeoxyribonucleotide (DNA) or a oligoribonucleotide (RNA), and encompasses short RNA molecules such as but not limited to siRNA, shRNA, sshRNA, and miRNA.

As used herein, the term "peptide" refers to a polymeric molecule which minimally includes at least two amino acid monomers linked by peptide bond, and preferably has at least about 10, and more preferably at least about 20 amino acid monomers, and no more than about 60 amino acid monomers, preferably no more than about 50 amino acid monomers linked by peptide bonds. For example, the term encompasses polymers having about 10, about 20, about 30, about 40, about 50, or about 60 amino acid residues.

As used herein, the term "polypeptide" refers to a polymeric molecule including at least one chain of amino acid monomers linked by peptide bonds, wherein the chain includes at least about 70 amino acid residues, preferably at least about 80, more preferably at least about 90, and still more preferably at least about 100 amino acid residues. As used herein the term encompasses proteins, which may include one or more linked polypeptide chains, which may or may not be further bound to cofactors or other proteins. The term "protein" as used herein is used interchangeably with the term "polypeptide."

As used herein, the term "variant" with reference to a molecule is a sequence that is substantially similar to the sequence of a native or wild type molecule. With respect to nucleotide sequences, variants include those sequences that may vary as to one or more bases, but because of the degeneracy of the genetic code, still encode the identical amino acid sequence of the native protein. Variants include naturally occurring alleles, and nucleotide sequences which are engineered using well-known techniques in molecular biology, such as for example site-directed mutagenesis, and which encode the native protein, as well as those that encode a polypeptide having amino acid substitutions. Generally, nucleotide sequence variants of the invention have at least 40%, at least 50%, at least 60%, at least 70% or at least 80% sequence identity to the native (endogenous) nucleotide sequence. The present disclosure also encompasses nucleotide sequence variants having at least about 85% sequence identity, at least about 90% sequence identity, at least about 85%, 86%, 87%, 88%, 89%, 90% 91%, 92%, 93%, 94%, 95%, 96%, 97%, 98%, 99%.

As used herein with respect to a given nucleotide sequence, the term "conservative variant" refers to a nucleotide sequence that encodes an identical or essentially identical amino acid sequence as that of a reference sequence. Due to the degeneracy of the genetic code, whereby almost always more than one codon may code for each amino acid, nucleotide sequences encoding very closely related proteins may not share a high level of sequence identity. Moreover, different organisms have preferred codons for many amino acids, and different organisms or even different strains of the same organism, *e.g.*, *E coli* strains, can have different preferred codons for the same amino acid. Thus, a first nucleotide acid sequence which encodes essentially the same polypeptide as a second nucleotide acid sequence is considered substantially identical to the second nucleotide sequence, even if they do not share a minimum percentage sequence identity, or would not hybridize to one another under stringent conditions. Additionally, it should be understood that with the limited exception of ATG, which is usually the sole codon for methionine, any sequence can be modified to yield a functionally identical molecule by standard techniques, and such modifications are encompassed by the present disclosure. As described herein below, the present disclosure specifically contemplates protein variants of a native protein, which have amino acid sequences having at least 15%, at least 16%, at least 21%, at least 40%, at least 41%, at least 52%, at least 53%, at least 56%, at least 59% or at least 86% sequence identity to a native nucleotide sequence.

Sequence identity of amino acid sequences or nucleotide sequences, within defined regions of the molecule or across the full-length sequence, can be readily determined using conventional tools and methods known in the art and as described herein. For example, the degree of sequence identity of two amino acid sequences, or two nucleotide sequences, is readily determined using alignment tools such as the NCBI Basic Local Alignment Search Tool (BLAST) (Altschul et al., 1990), which are readily available from multiple online sources. Algorithms for optimal sequence alignment are well known and described in the art, including for example in Smith and Waterman, Adv. Appl. Math. 2:482 (1981); Pearson and Lipman Proc. Natl. Acad. Sci. (U.S.A.) 85: 2444 (1988). Algorithms for sequence analysis are also readily available in programs such as blastp, blastn, blastx, tblastn and tblastx. For the purposes of the present disclosure, two nucleotide sequences may be also considered "substantially identical" when they hybridize to each other under stringent conditions. Stringent conditions including a high hybridization temperature and low salt in hybridization buffers which permit hybridization only between nucleic acid sequences that are highly similar. Stringent conditions are sequence-dependent and will be different in different circumstance, but typically include a temperature at least about 60° , which is about 10° C to about 15° C lower than the thermal melting point (Tm) for the specific sequence at a defined ionic strength and pH. Salt concentration is typically about 0.02 molar at pH 7.

The degree of sequence identity between two amino acid sequences may be determined using the BLASTp algorithm of Karlin and Altschul (Proc. Natl. Acad. Sci. USA 87:2264-2268, 1993). The percentage of sequence identity is determined by comparing two optimally aligned sequences over a comparison window, wherein the portion of the amino acid sequence in the comparison window may comprise additions or deletions (i.e., gaps) as compared to the reference sequence (which does not comprise additions or deletions) for optimal alignment of the two sequences. The percentage is calculated by determining the number of positions at which an identical amino acid occurs in both sequences to yield the number of matched positions, dividing the number of matched positions by the total number of positions in the window of comparison and multiplying the result by 100 to yield the percentage of sequence identity.

One of skill will recognize that polypeptides may be "substantially similar" in that an amino acid may be substituted with a similar amino acid residue without affecting the function of the mature protein. Polypeptide sequences which are "substantially similar" share sequences as noted above except that residue positions, which are not identical, may have conservative amino acid changes. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acid substitution groups include: valine-leucine-isoleucine, phenylalanine-tyrosine, lysine-arginine, alanine-valine, and asparagine-glutamine.

A nucleic acid encoding a peptide, polypeptide or protein may be obtained by screening selected cDNA or genomic libraries using a deduced amino acid sequence for a given protein. Conventional procedures using primer extension procedures, as described for example in Sambrook et al., can be used to detect precursors and processing intermediates.

### Virus-Like Particles (VLP's) Composed of a Capsid enclosing a Cargo Molecule

The methods and compositions described herein are the result in part of the appreciation that certain viral capsids can be prepared and/or used in novel manufacturing and purification methods to improve commercialization procedures for nucleic acids. The methods described herein use recombinant viral capsids which are resistant to readily available hydrolases, to enclose heterologous cargo molecules such as nucleic acids, peptides, or polypeptides including proteins.

The capsid may be a wild type capsid or a mutant capsid derived from a wild type capsid, provided that the capsid exhibits resistance to hydrolysis catalyzed by at least one hydrolase acting on peptide bonds when the capsids are contacted with the hydrolase. As used interchangeably herein, the phrases "resistance to hydrolysis" and "hydrolase resistant" refer to any capsid which, when present in a whole cell lysate also containing polypeptides which are cell lysis products and not enclosed in the capsids, and subjected to hydrolysis using a peptide bond hydrolase category EC 3.4 for a time and under conditions sufficient for at least 60, at least 70, at least 80, or at least 90 of every 100 individual polypeptides present in the lysate (which are cell lysis products and not enclosed in the capsids) to be cleaved (*i.e.* at least 60%, at least 70%, at least 80%, or at least 90% of all individual unenclosed polypeptides are cleaved), yet at least 60, at least 70, at least 80, or at least 90 of every 100 capsids present before such hydrolysis remain intact following the hydrolysis. Hydrolysis may be conducted for a period of time and under conditions sufficient for the average molecular weight of cell proteins remaining from the cell line following hydrolysis is less than about two thirds, less than about one half, less than about one third, less than about one fourth, or less than about one fifth, of the average molecular weight of the cell proteins before the hydrolysis is conducted. Methods may further comprise purifying the intact capsid remaining after hydrolysis, and measuring the weight of capsids and the weight of total dry cell matter before and after hydrolysis and purification, wherein the weight of capsids divided by the weight of total dry cell matter after hydrolysis and purification is at least twice the weight of capsids divided by the weight of total dry cell matter measured before the hydrolysis and purification. The weight of capsids divided by the weight of total dry cell matter after hydrolysis and purification may be at least 10 times more than, preferably 100 times more than, more preferably 1,000 times more than, and most preferably 10,000 times more than the weight of capsids divided by the weight of total dry cell matter measured before such hydrolysis and purification.

Hydrolases are enzymes that catalyze hydrolysis reactions classified under the identity number EC 3 by the European Commission. For example, enzymes that catalyze hydrolysis of ester bonds have identity numbers starting with EC 3.1. Enzymes that catalyze hydrolysis of glycosidic bonds have identity numbers starting with EC 3.2. Enzymes that catalyze hydrolysis of peptide bonds have identity numbers starting with EC 3.4. Proteases, which are enzymes that catalyze hydrolysis of proteins, are classified using identity numbers starting with EC 3.4, including but not limited to Proteinase K and subtilisin. For example, Proteinase K has identity number EC 3.4.21.64. The present disclosure encompasses VLP's which are resistant, in non-limiting example, Proteinase K, Protease from *Streptomyces griseus,* Protease from *Bacillus lichenformis,* pepsin and papain, and methods and processes of using such VLP's.

The Nomenclature Committee of the International Union of biochemistry and Molecular Biology also recommends naming and classification of enzymes by the reactions they catalyze. Their complete recommendations are freely and widely available, and for example can be accessed online at http:// enzyme.expasy.org and, www.chem.qmul.ac.uk/iubmb/enzyme/, among others. The IUBMB developed a shorthand for describing what sites each enzyme is active against. Enzymes that indescriminately cut are referred to as broadly specific. Cleavage patterns for the other enzymes are described as Xaa|Yaa, where | represents the cleavage site, Xaa ={set of residues preferred by the enzyme on the N-terminal side of the cleavage}, and Yaa={set of residues preferred by the enzyme on the C-terminal side of the cleavage}. Some enzymes have more binding requirements than this so the description can become more complicated. For an enzyme that catalyzes a very specific reaction, for example an enzyme that processes prothrombin to active thrombin, then that activity is the basis of the cleavage description. In certain instances the precise activity of an enzyme may not be clear, and in such cases, cleavage results against standard test proteins like B-chain insulin are reported. As an alternative to using enzymes that catalyze hydrolysis of peptide bonds which have identity numbers starting with EC 3.4, broadly specific enzymes can be used which have Xaa|Yaa preferences where the enzyme has reported PI pocket binding preferences Xaa but no preference for binding the P1' pocket Yaa and conversely, where the enzyme has reported P1' pocket binding preferences Yaa but no preference for binding the PI pocket Xaa.

The capsids can be further selected and/or prepared such that they can be isolated and purified using simple isolation and purification procedures, as described in further detail herein. For example, the capsids can be selected or genetically modified to have significantly higher hydrophobicity than a surrounding matrix as described herein, so as to selectively partition into a non-polar water-immiscible phase into which they are simply extracted. Alternatively, a capsid may be selected or genetically modified for improved ability to selectively crystallize from solution.

Use of simple and effective purification processes using the capsids is enabled by the choice of certain wild type capsids, or modifications to the amino acid sequence of proteins comprising the wild type capsids, such that the capsid exhibits resistance to hydrolysis catalyzed by at least one hydrolase acting on peptide bonds as described herein above. Such wild type capsids, such as the wild type MS2 capsid, can be used in a purification process in which certain inexpensive enzymes such as Proteinase K or subtilisin are used for proteolysis. A non-limiting example is the Enterobacteria phage MS2 (SEQ ID NO: 1, whole MS2 wild type genome; SEQ ID NO: 2, MS2 wild type coat protein, DNA sequence; and SEQ ID NO: 3, MS2 wild type coat protein, amino acid sequence.
(SEQ. ID NO: 1) Whole MS2 Genome, wildtype, coat protein sequence in bold: 
GGGTGGGACCCCTTTCGGGGTCCTGCTCAACTTCCTGTCGAGCTAATGCCATTTTA
ATGTCTTTAGCGAGACGCTACCATGGCTATCGCTGTAGGTAGCCGGAATTCCATTCC
TAGGAGGTTTGACCTGTGCGAGCTTTTAGTACCCTTGATAGGGAGAACGAGACCTTC
GTCCCCTCCGTTCGCGTTTACGCGGACGGTGAGACTGAAGATAACTCATTCTCTTTA
AAATATCGTTCGAACTGGACTCCCGGTCGTTTTAACTCGACTGGGGCCAAAACGAAA
CAGTGGCACTACCCCTCTCCGTATTCACGGGGGCGTTAAGTGTCACATCGATAGAT
CAAGGTGCCTACAAGCGAAGTGGGTCATCGTGGGGTCGCCCGTACGAGGAGAAAGC
CGGTTTCGGCTTCTCCCTCGACGCACGCTCCTGCTACAGCCTCTTCCCTGTAAGCCAA
AACTTGACTTACATCGAAGTGCCGCAGAACGTTGCGAACCGGGCGTCGACCGAAGT
CCTGCAAAAGGTCACCCAGGGTAATTTTAACCTTGGTGTTGCTTTAGCAGAGGCCAG
GTCGACAGCCTCACAACTCGCGACGCAAACCATTGCGCTCGTGAAGGCGTACACTG
CCGCTCGTCGCGGTAATTGGCGCCAGGCGCTCCGCTACCTTGCCCTAAACGAAGATC
GAAAGTTTCGATCAAAACACGTGGCCGGCAGGTGGTTGGAGTTGCAGTTCGGTTGG
TTACCACTAATGAGTGATATCCAGGGTGCATATGAGATGCTTACGAAGGTTCACCTT
 
CAAGAGTTTCTTCCTATGAGAGCCGTACGTCAGGTCGGTACTAACATCAAGTTAGAT
GGCCGTCTGTCGTATCCAGCTGCAAACTTCCAGACAACGTGCAACATATCGCGACGT
ATCGTGATATGGTTTACATAAACGATGCACGTTTGGCATGGTTGTCGTCTCTAGGT
ATCTTGAACCCACTAGGTATAGTGTGGAAAAGGTGCCTTTCTCATTCGTTGTCGAC
TGGCTCCTACCTGTAGGTAACATGCTCGAGGGCCTTACGGCCCCCGTGGATGCTCC
TACATGTCAGGAACAGTTACTGACGTAATAACGGGTGAGTCCATCATAAGCGTTGA
CGCTCCCTACGGGTGGACTGTGGAGAGACAGGGCACTGCTAAGGCCCAAATCTCAG
CCATGCATCGAGGGGTACAATCCGTATGGCCAACAACTGGCGCGTACGTAAAGTCT
CCTTTCTCGATGGTCCATACCTTAGATGCGTTAGCATTAATCAGGCAACGGCTCTCT
AGATAGAGCCCTCAACCGGAGTTTGAAGCATGGCTTCTAACTTTACTCAGTTCGT
TCTCGTCGACAATGGCGGAACTGGCGACGTGACTGTCGCCCCAAGCAACTTCG
CTAACGGGGTCGCTGAATGGATCAGCTCTAACTCGCGTTCACAGGCTTACAAA
GTAACCTGTAGCGTTCGTCAGAGCTCTGCGCAGAATCGCAAATACACCATCAA
AGTCGAGGTGCCTAAAGTGGCAACCCAGACTGTTGGTGGTGTAGAGCTTCCTG
TAGCCGCATGGCGTTCGTACTTAAATATGGAACTAACCATTCCAATTTTCGCTA
CGAATTCCGACTGCGAGCTTATTGTTAAGGCAATGCAAGGTCTCCTAAAAGATG
GAAACCCGATTCCCTCAGCAATCGCAGCAAACTCCGGCATCTACTAATAGACGC
CGGCCATTCAAACATGAGGATTACCCATGTCGAAGACAACAAAGAAGTTCAACTCT
TTATGTATTGATCTTCCTCGCGATCTTTCTCTCGAAATTTACCAATCAATTGCTTCTGT
CGCTACTGGAAGCGGTGATCCGCACAGTGACGACTTTACAGCAATTGCTTACTTAAG
GGACGAATTGCTCACAAAGCATCCGACCTTAGGTTCTGGTAATGACGAGGCGACCC
GTCGTACCTTAGCTATCGCTAAGCTACGGGAGGCGAATGGTGATCGCGGTCAGATA
AATAGAGAAGGTTTCTTACATGACAAATCCTTGTCATGGGATCCGGATGTTTTACAA
ACCAGCATCCGTAGCCTTATTGGCAACCTCCTCTCTGGCTACCGATCGTCGTTGTTTG
GGCAATGCACGTTCTCCAACGGTGCTCCTATGGGGCACAAGTTGCAGGATGCAGCG
CCTTACAAGAAGTTCGCTGAACAAGCAACCGTTACCCCCCGCGCTCTGAGAGCGGCT
CTATTGGTCCGAGACCAATGTGCGCCGTGGATCAGACACGCGGTCCGCTATAACGA
GTCATATGAATTTAGGCTCGTTGTAGGGAACGGAGTGTTTACAGTTCCGAAGAATAA
TAAAATAGATCGGGCTGCCTGTAAGGAGCCTGATATGAATATGTACCTCCAGAAAG
GGGTCGGTGCTTTCATCAGACGCCGGCTCAAATCCGTTGGTATAGACCTGAATGATC
AATCGATCAACCAGCGTCTGGCTCAGCAGGGCAGCGTAGATGGTTCGCTTGCGACG
ATAGACTTATCGTCTGCATCCGATTCCATCTCCGATCGCCTGGTGTGGAGTTTTCTCC
CACCAGAGCTATATTCATATCTCGATCGTATCCGCTCACACTACGGAATCGTAGATG
GCGAGACGATACGATGGGAACTATTTTCCACAATGGGAAATGGGTTCACATTTGAG
CTAGAGTCCATGATATTCTGGGCAATAGTCAAAGCGACCCAAATCCATTTGGTAAC
GCCGGAACCATAGGCATCTACGGGGACGATATTATATGTCCCAGTGAGATTGCACC
CCGTGTGCTAGAGGCACTTGCCTACTACGGTTTTAAACCGAATCTTCGTAAAACGTT
CGTGTCCGGGCTCTTTCGCGAGAGCTGCGGCGCGCACTTTTACCGTGGTGTCGATGT
CAAACCGTTTTACATCAAGAAACCTGTTGACAATCTCTTCGCCCTGATGCTGATATT
AAATCGGCTACGGGGTTGGGGAGTTGTCGGAGGTATGTCAGATCCACGCCTCTATA
 
AGGTGTGGGTACGGCTCTCCTCCCAGGTGCCTTCGATGTTCTTCGGTGGGACGGACC
TCGCTGCCGACTACTACGTAGTCAGCCCGCCTACGGCAGTCTCGGTATACACCAAGA
CTCCGTACGGGCGGCTGCTCGCGGATACCCGTACCTCGGGTTTCCGTCTTGCTCGTA
TCGCTCGAGAACGCAAGTTCTTCAGCGAAAAGCACGACAGTGGTCGCTACATAGCG
TGGTTCCATACTGGAGGTGAAATCACCGACAGCATGAAGTCCGCCGGCGTGCGCGT
TATACGCACTTCGGAGTGGCTAACGCCGGTTCCCACATTCCCTCAGGAGTGTGGGCC
AGCGAGCTCTCCTCGGTAGCTGACCGAGGGACCCCCGTAAACGGGGTGGGTGTGCT
CGAAAGAGCACGGGTGCGAAAGCGGTCCGGCTCCACCGAAAGGTGGGCGGGCTTCG
GCCCAGGGACCTCCCCCTAAAGAGAGGACCCGGGATTCTCCCGATTTGGTAACTAG
CTGCTTGGCTAGTTACCACCCA
(SEQ. ID NO: 2) MS2 coat protein DNA sequence, wild type: 
ATGGCTTCTAACTTTACTCAGTTCGTTCTCGTCGACAATGGCGGAACTGGCGACGTG
ACTGTCGCCCCAAGCAACTTCGCTAACGGGGTCGCTGAATGGATCAGCTCTAACTCG
CGTTCACAGGCTTACAAAGTAACCTGTAGCGTTCGTCAGAGCTCTGCGCAGAATCGC
AAATACACCATCAAAGTCGAGGTGCCTAAAGTGGCAACCCAGACTGTTGGTGGTGT
AGAGCTTCCTGTAGCCGCATGGCGTTCGTACTTAAATATGGAACTAACCATTCCAAT
TTTCGCTACGAATTCCGACTGCGAGCTTATTGTTAAGGCAATGCAAGGTCTCCTAAA
AGATGGAAACCCGATTCCCTCAGCAATCGCAGCAAACTCCGGCATCTACTAATAG
(SEQ. ID NO: 3) MS2 wild type coat protein, amino acid sequence:

MASNFTQFVLVDNGGTGDVTVAPSNFANGVAEWISSNSRSQAYKVTCSVRQSSAQNRK
YTIKVEVPKVATQTVGGVELPVAAWRSYLNMELTIPIFATNSDCELIVKAMQGLLKDGN
PIPSAIAANSGIY

Surprisingly, the unmodified, wild type MS2 capsid though lacking an envelope is resistant to a variety of category EC 3.4 hydrolases, including but not limited to Proteinase K and subtilisin, such that a highly purified composition of the capsid, which may contain a cargo molecule, can be prepared from a whole cell lysate. Accordingly, the present disclosure provides VLPs comprising viral capsids comprising the wild type MS2 capsid protein, and/or capsid proteins sharing homology with wild type MS2 capsid proteins, which viral capsids encapsidate the cargo molecule. The cargo molecule may comprise one or more heterologous nucleic acids, peptides, polypeptides or proteins. These VLP's can then be isolated and purified from a whole cell lysate after a hydrolysis step using a category EC 3.4 hydrolase, to produce a composition of VLP's of high purity, for example at least 60%, at least 70%, a least 80%, or at least 85% by weight VLP's. Compositions having a purity of at least 90%, 91%, 92%, 93%, 94%, 95%, 96%, 97%, and 98% by weight of VLP's are expressly contemplated.

The present disclosure encompasses a composition comprising: a) a plurality of virus-like particles each comprising a wild type viral capsid and at least one target heterologous cargo molecule enclosed in the wild type viral capsid; and b) one or more cell lysis products present in an amount of less than 40 grams, less than 30 grams, less than 20 grams, less than 15 grams, less than 10 grams, and preferably less than 9, 8, 7, 6, 5, 4, 3 , more preferably less than 2 grams, and still more preferably less than 1 gram, for every 100 grams of capsid present in the composition, wherein the cell lysis products are selected from proteins, polypeptides, peptides and any combination thereof. Subsequently the cargo molecules can be readily harvested from the capsids. Accordingly, such compositions are highly desirable for all applications where high purity and/or high production efficiency is required.

Hydrolase resistant capsids as described herein may be used to enclose different types of cargo molecules to form a virus-like particle. The cargo molecule can be but is not limited to any one or more oligonucleotide or oligoribonucleotide (DNA, RNA, LNA, PNA, siRNA, shRNA , sshRNA, IshRNA or miRNA, or any oligonucleotide comprising any type of non-naturally occurring nucleic acid), any peptide, polypeptide or protein. A cargo molecule which is an oligonucleotide or oligoribonucleotide may be enclosed in a capsid with or without the use of a linker. A capsid can be triggered for example to self-assemble from capsid protein in the presence of nucleotide cargo, such as an oligoribonucleotide. In non-limiting example, a capsid as described herein may enclose a target heterologous RNA strand, such as for example a target heterologous RNA strand containing a total of between 1,800 and 2,248 ribonucleotides, including the 19-mer pack site from Enterobacteria phage MS2, such RNA strand transcribed from a plasmid separate from a plasmid coding for the capsid proteins, as described by Wei, Y. et al. (2008) J. Clin. Microbiol. 46:1734-1740.

RNA interference (RNAi) is a phenomenon mediated by short RNA molecules such as siRNA molecules, which can be used for selective suppression of a target gene of interest, and has multiple applications in biotechnology and medicine. For example, short RNA molecules can be employed to target a specific gene of interest in an organism to obtain a desirable phenotype. Short RNA molecules, including siRNA, are however easily degraded by ubiquitous enzymes called RNAses. Capsids, such as those described herein, protect encapsidated RNA from enzymatic degradation. A capsid as described herein may however enclose an RNA strand containing one or more ribozymes, either self-cleaving ribozymes (cis-acting), or in certain cases capable of cleaving bonds in other RNA (trans-acting). One or more ribozymes may be included for example to specifically cut RNA sequence(s) to produce a specifically tailored RNA molecule, such as for example but not limited to an siRNA molecule. For example, variants of Hammerhead and Hepatitis Delta Virus ribozymes are known and can be used to cut long RNA sequences. The present disclosure describes novel VLPs comprising a capsid encapsidating one or more ribozymes attached to pack sequences as described above (*i.e.,* RNA sequences with strong affinity to the interior wall of a capsid), and the ribozymes used to cut short RNA sequences from packing sequences attached to the ribozymes.

The present disclosure thus also encompasses the novel use of ribozymes to isolate short or small" RNA sequences such as siRNA, shRNA, sshRNA, and miRNA sequences from the packing sequence(s) used to encapsidate them. It should be understood that, unless expressly indicated otherwise, the term short RNA encompasses short single stranded and short hairpin (stem loop) RNA sequences having a double stranded stem and a single-stranded loop or hairpin. A short RNA is any RNA single strand having no more than 30 nucleotides, preferably no more than 25 nucleotides, and more preferably no more than 22 nucleotides; or a hairpin RNA having a stem of no more than 30 nucleotides base pairs, preferably no more than 25 nucleotide base pairs, and more preferably no more than 22 nucleotide base pairs in the stem.

A challenge in using a ribozyme which is highly active to isolate such short RNA sequences from packing sequences, is that the ribozyme may works so fast as to liberate the short RNA from the packing sequence before encapsidation of the RNA is achieved. Additionally, it has been discovered that the three dimensional structures of short RNA such as an siRNA, or the hairpin packing sequences, can interfere with the proper functioning of the ribozyme. These problems can be overcome by 1) using ribozyme mutants which demonstrate a slower rate of activity, to avoid liberation of the short RNA from the packing sequence before encapsidation of the short RNA is achieved, and/or 2) increasing the number of nucleotides in the ribozyme that form Watson-Crick pairs with the short RNA. Additionally, trans-acting ribozymes can be used advantageously to increase the percentage of RNA encapsidated into VLPs as short RNA, if the short RNA sequence(s) are flanked not by complete ribozymes but rather shorter sequences that are targets of trans-acting ribozymes, also encapsidated into the same VLP.

One or more short RNA sequences can also be encapsidated into a viral capsid, either wild type or genetically modified, which has been modified to insert an external peptide tag, to deliver a protein or drug molecule to a specific class of cell. Wild type capsids may also be genetically modified to insert external peptide sequences acting as ligands for certain surface protein cell receptors can be advantageously used to encapsidate short RNA sequences aimed at inducing RNAi in specific target cells. Such compositions are much simpler, less expensive and more reliably manufactured than current alternatives for short RNA delivery.

Non-limiting examples of useful VLP's which can be prepared include a capsid enclosing an RNA strand comprising:
(i) at least one packing sequence and from 1 to 100 identical or different siRNAs flanked by one single stranded (non-hybridyzing) RNA spacer, where every single stranded RNA spacer has between 4 and 40 nucleotides (SEQ ID NO: 30);
(ii) one (1) ribozyme and one single stranded (non-hybridizing) RNA sequence per siRNA, where every single stranded RNA sequence has between 4 and 40 nucleotides (SEQ ID NO: 28; HDV ribozymes);
(iii) two (2) ribozymes per siRNA;
(iv) one (1) T7 start site, one (1) ribozyme, one (1) packing site and one (1) transcription termination site; or
(v) one (1) T7 start site, one (1) packing site, and one (1) transcription termination site.
(vi) four (4) ribozymes per siRNA (SEQ ID NO: 29)

In VLP's which include one or more ribozymes, the disclosure further contemplates VLPs containing the resulting products after the ribozymes have cut the RNA. VLP's which include a transcription terminator can use for example the T7 transcription terminator as described in Studier FW, Moffatt BA. (1986) J. Mol Biol. May5; 189(1):113-30; and R Sousa, D Patra, EM Lafer (1992) J. Mol. Biol. 224:319-334. For example, the following two sequences are suitable transcription terminators for T7 RNA polymerase:
CTAGCATAACCCCTTGGGGCCTCTAAACGGGTCTTG (SEQ ID NO: 4)
or
   TAGCATAACCCCTTGGGGCCTCTAAACGGGTCTTGAGGGGTTTTTTG (SEQ ID NO: 5).

VLPs as described herein may alternatively enclose at least one target peptide, polypeptide or protein. When the target heterologous cargo molecule is a peptide, polypeptide or protein, an oligonucleotide linker can be used to couple the target heterologous cargo molecule and the viral capsid. A cargo molecule which is a peptide, polypeptide or protein, preferably is packaged in a capsid using a linker. The packaging process is promoted by the linker, consisting of a short RNA aptamer sequence, which forms a link between the coat protein and a peptide tag fused to the target cargo molecule. (*See* Fiedler, J. et al., RNA-Directed Packaging of Enzymes within Virus-like Particles, Angew. Chem. Int. Ed. 49: 9648 -9651 (2010)). The oligonucleotide linker may consist of DNA, RNA, LNA, PNA, and the like. The linker is for example a 50- to 100- mer having a short sequence, for example about 20 nt long, at a first end with binding specificity for the inside of the capsid coat, and another sequence, for example about 70 nt long, at the second, opposite end which has a binding specificity for the cargo peptide, polypeptide or protein. Additionally, a slow ribozyme may be incorporated into a linker consisting of RNA. For example, a slow ribozyme can be incorporated between the packing sequence (binding to the coat protein) and the aptamer (binding to the tag of target protein). Upon activation, the ribozyme will separate the coat protein from the target protein. Alternatively, a capsid as described herein may enclose at least one target protein N-terminally tagged with a peptide able to non-covalently bind to an aptamer- and capsid pack sequence-containing RNA strand, for example an N-terminal tag and aptamer- and pack sequence-containing RNA strand as described by Fiedler, J. et al. (2010).

A cargo molecule can be a bi-molecular cargo molecule, and capsids described herein may also encapsidate a bi-molecular cargo molecule, which may or may not include one or more more ribozymes. A bi-molecular cargo molecule may comprise an aptamer linked to a bifunctional polynucleotide. The aptamer may have a sequence specifically selected using SELEX to exhibit specific binding to a bioactive small molecule, *i.e.,* a molecule having a low molecular weight, preferably lower than 1,500 Da. The bifunctional polynucleotide has both a first aptameric activity for binding the low-molecular weight bioactive cargo molecule, and a second aptameric activity for binding a packing sequence of a capsid. The bifunctional polynucleotide linked to the bioactive cargo molecule forms the bi-molecular cargo molecule which can then be linked to the capsid. Such a cargo molecule can be used to bind the bioactive small molecule, and thus load the VLP with the small molecule. The present disclosure thus also encompasses a VLP comprising a capsid linked to such a synthetic bi-molecular cargo molecule. Examples of low molecular weight bioactives which can be loaded into a VLP by binding to an RNA aptamer include: atrazine (herbicide), acetamipridphorate, profenofos, isocarbophos and omethoateas (insecticides), as described by Sett et al. (2012) Open Journal of Applied Biosensor, 1:p. 9-19.

Examples of low molecular weight bioactives which can be loaded into a VLP by binding to an RNA aptamer include herbicides such as 2,4-D (2,4-Dichlorophenoxyacetic acid), Dicamba ((3,6-dichloro-2-methoxybenzoic acid), Paraquat (N,N'-dimethyl-4,4'-bipyridinium dichloride), Oryzalin (4-(dipropylamino)-3,5-dinitrobenzenesulfonamide), DCMU (3-(3,4-dichlorophenyl)-1,1-dimethylurea), Trifluralin (2,6-Dinitro-N,N-dipropyl-4-(trifluoromethyl)aniline), Imazapic (-methyl-2-[4-methyl-5-oxo-4-(propan-2-yl)-4,5-dihydro-1H-imidazol-2-yl]pyridine-3-carboxylic acid), Aminopyralid (4-amino-3,6-dichloropyridine-2-carboxylic acid), Clopyralid (3,6-dichloro-2-pyridinecarboxylic acid), Metolachlor ((RS)-2-Chloro-N-(2-ethyl-6-methyl-phenyl)-N-(1-methoxypropan-2-yl)acetamide), Pendimethalin (3,4-Dimethyl-2,6-dinitro-N-pentan-3-yl-aniline), Picloram (4-Amino-3,5,6-trichloro-2-pyridinecarboxylic acid), Propanil (N-(3,4-Dichlorophenyl)propanamide), Triclopyr ([(3,5,6-Trichloro-2-pyridinyl)oxy]acetic acid), and Atrazine (2-chloro-4-(ethylamino)-6-(isopropylamino)-s-triazine), among other listed for example by Roberts et al. (1998) Metabolic Pathways of Agrochemicals: Part 1: Herbicides and Plant Growth Regulators. Published by Royal Society of Chemistry (Great Britain) ISBN 978-1-84755-138-2. For example, an RNA aptamer binding Atrazine was described by Sinha et al. (2010) Nature Chemical Biology, 6:p.464-470.

RNA aptamers can also be used to bind insecticides such as, Propargite (2-(4-tert-butylphenoxy)cyclohexyl prop-2-yne-1-sulfonate), Chlorpyrifos (O,O-diethyl O-3,5,6-trichloropyridin-2-yl phosphorothioate), Cypermethrin, Phosmet (2-Dimethoxyphosphinothioylthiomethyl)isoindoline-1,3-dione), Permethrin (3-Phenoxybenzyl (1RS)-cis,trans-3-(2,2-dichlorovinyl)-2,2-dimethylcyclopropanecarboxylate), Diazinon (O,O-Diethyl O-[4-methyl-6-(propan-2-yl)pyrimidin-2-yl] phosphorothioate), Methylparathion (O,O-Dimethyl O-(4-nitrophenyl) phosphorothioate), and Acetamiprid (N-[(6-chloro-3-pyridyl)methyl]-N'-cyano-N-methyl-acetamidine), and fungicides such as Chlorothalonil (2,4,5,6-tetrachloroisophthalonitrile), Captan ((3aR,7aS)-2-[(trichloromethyl)sulfanyl]-3a,4,7,7a-tetrahydro-1H-isoindole-1,3(2H)-dione), Boscalid (2-chloro-N-(4'-chlorobiphenyl-2-yl)nicotinamide), Iprodione (3-(3,5-dichlorophenyl)-N-isopropyl-2,4-dioxoimidazolidine-1-carboxamide), Azoxystrobin (Methyl (2E)-2-(2-{[6-(2-cyanophenoxy)pyrimidin-4-yl]oxy}phenyl)-3-methoxyacrylate), Pyraclostrobin, (methyl 2-[1-(4-chlorophenyl)pyrazol-3-yloxymethyl]-N-methoxycarbanilate), Cyprodinil (4-cyclopropyl-6-methyl-N-phenylpyrimidin-2-amine), among other listed for example by Roberts et al. (1999) Metabolic Pathways of Agrochemicals: Part 2: Insecticides and Fungicides. Published by Royal Society of Chemistry (Great Britain) ISBN 978-1-84755-137-5. For example, aptamers have been described to bind acetamiprid, phorate, profenofos, isocarbophos and omethoate, as exemplified by Sett et al. (2012) Open Journal of Applied Biosensor, 1:p. 9-19 using DNA aptamers built in a similar manner as RNA aptamers are built using SELEX.

These herbicides, insecticides or fungicides are bioactive small molecules, *i.e.*, molecules having a low molecular weight, preferably lower than 1,500 Da. Due to their small size they can permeate capsids forming VLPs of the current disclosure, as exemplified by Wu et al. (2005) Delivery of antisense oligonucleotides to leukemia cells by RNA bacteriophage capsids, Nanomedicine: Nanotechnology, Biology and Medicine, 1:p.67-76. These small bioactive molecules are added to VLPs of the current disclosure which encapsidate aptamers designed using SELEX to bind the small bioactive molecules, after such VLPs have been formed, either before or after purification. The addition of these small bioactive molecules is done, for example by adding them to a solution of the VLPs and incubation, for example at room temperature for a time between 30 minutes and 10 hours. These small bioactive molecules enter the VLPs by diffusion through the pores at the particle symmetry axes and are retained inside due to their binding to the enclosed aptamers. Suitable solvents used for loading the small bioactive molecules into the VLPs range from polar such as water and water-ethanol blends to non-polar such as, for example, isooctane, toluene, dichloromethane, or chloroform. Using non-polar solvents for the dissolution of VLPs is done, for example, as described by Johnson et al. (2006), Solubilization and stabilization of bacteriophage MS2 in organic solvents, Biotechnology and bioengineering, 2007. 97(2): p. 224-34, with the help of surfactants like Aerosol OT. Use of non-polar solvents for loading small bioactive molecules is preferred since their solubility in polar solvents is, in most cases, poor.

VLPs encapsidating both siRNA and small bioactive molecules are preferred in applications where a synergistic effect is achieved between the two bioactive ingredients, for example in those cases where the targeted plant, insect or fungus is resistant to the small bioactive molecule. In such cases, the siRNA is designed to target the biologic pathway that confers the plant, insect or fungus resistance to the small bioactive molecule, as exemplified by Sammons et al., Polynucleotide molecules for gene regulation in plants, US 2011/0296556.

Alternatively, the bi-functional polynucleotide may encode at least one siRNA, shRNA , sshRNA, IshRNA or miRNA, and the cargo molecule can be a small (low molecular weight) protein or peptide. Accordingly, a bi-molecular cargo molecule can be capable of binding a low molecular bioactive protein or peptide. Such a bi-molecular cargo molecule may comprise a biologically active protein or peptide, coupled to a polynucleotide encoding at least one siRNA or shRNA or sshRNA or IshRNA of miRNA, and having a first aptameric activity for binding the bioactive protein or peptide cargo molecule and a second aptameric activity for binding a packing sequence of a capsid. The polynucleotide is linked to the protein or peptide cargo molecule and is capable of linking to packing sequence of a capsid.

A bifunctional polynucleotide as described above may optionally include one or more ribozyme sequences. A VLP including a bi-molecular cargo molecule including a bifunctional polynucleotide as described above may optionally include one or more ribozymes. The present disclosure also encompasses a VLP comprising a capsid and reaction products of the the bi-molecular cargo molecule after at least one ribozyme has reacted with bimolecular cargo molecule to cut the cargo molecule into constituent parts including the aptamer.

VLPs as described herein may be assembled by any available method(s) which produces a VLP with an assembled, hydrolase resistant capsid encapsidating one or more cargo molecule(s), and optionally any linker, packing sequence, one or more ribozymes, or tags. For example, capsids and cargo molecules may be co-expressed in any expression system. Recombinant DNA encoding one or more capsid proteins, one or more cargo molecule(s), and optionally any linker, packing sequence, ribozyme(s) or tags can be readily introduced into the host cells, *e.g*., bacterial cells, plant cells, yeast cells, fungal cells, and animal cells (including insect and mammalian) by transfection with one or more expression vectors by any procedure useful for introducing such a vector into a particular cell, and stably transfecting the cell to yield a cell which expresses the recombinant sequence(s).

The host cell is preferably of eukaryotic origin, *e.g.*, plant, mammalian, insect, yeast or fungal sources, but non-eukaryotic host cells may also be used. Suitable expression systems include but are not limited to microorganisms such as bacteria (*e.g.*, *E. coli*,) transformed with recombinant bacteriophage DNA, plasmid DNA or cosmid DNA expression vectors containing the coding sequences for the VLP elements. In non-limiting example, for VLPs using the MS2 capsid protein, expression in *E. coli* is a suitable expression system.

The present disclosure expressly contemplates plant cells which have been transformed using a nucleic acid construct as described herein, and which expresses a capsid coat protein, cargo molecule and a and optionally any linker, packing sequence, one or more ribozymes, or tags. Means for transforming cells including plant cells and preparing transgenic cells are well known in the art. Vectors, plasmids, cosmids, YACs (yeast artificial chromosomes) and DNA segments can be used to transform cells and will as generally recognized include promoters, enhancers, and/or polylinkers. Transgenic cells specifically contemplated include transgenic plant cells including but not limited to cells obtained from corn, soybean, wheat, vegetables, grains, legumes, fruit trees, and so on, or any plant which would benefit from introduction of a VLP as described herein. Also contemplated are plants, plant tissue obtained from cells transformed as described herein, and the seed or progeny of the plant or plant tissue.

Expression of assembled VLPs can be obtained for example by constructing at least one expression vector including sequences encoding all elements of the VLP. Sometimes two vectors are used, a first which includes a sequence encoding the cargo molecule(s) and optionally any linker, packing sequence, one or more ribozymes, or tags; and a second vector which includes a sequence encoding the capsid protein. In an exemplary process for generating exemplary VLPs including siRNA, two vectors may be co-expressed in the host cell for generation of the VLP, as further detailed in the Examples. Methods and tools for constructing such expression vectors containing the coding sequences and transcriptional and translational control sequences are well known in the art. Vector(s) once constructed are transferred to the host cells also using techniques well known in the art, and the cells then maintained under culture conditions for a time sufficient for expression and assembling of the VLP's to occur, all using conventional techniques. The present disclosure thus encompasses host cells containing any such vectors, and cells which have been transformed by such vectors, as well as cells containing the VLP's.

When the VLP's have been expressed and assembled in the host cells, they may be isolated and purified using any method known in the art for virus purification. For example, the cells can be lysed using conventional cell lysis techniques and agents, and the cell lysate subjected to hydrolysis using at least one peptide bond hydrolase category EC 3.4 such as but not limited to Proteinase K or subtilisin. Intact capsids remaining in the cell lysate following hydrolysis can be removed and purified using conventional protein isolation techniques.

Purification of capsids, VLPs or proteins may also include methods generally known in the art. For example, following capsid expression and cell lysis, the resulting lysate can be subjected to one or more isolation or purification steps. Such steps may include for example enzymatic lipolysis, DNA hydrolysis, and proteolysis steps. A proteolysis step may be performed for example using a blend of endo- and exo-proteases. For example, after cell lysis and hydrolytic disassembly of most cell components, such capsids with their cargo molecules can be separated from surrounding matrix by extraction, for example into a suitable non-polar water-immiscible solvent, or by crystallization from a suitable solvent. For example, hydrolysis and/or proteolysis steps transform contaminants from the capsid that are contained in the lysate matrix into small, water soluble molecules. Hydrophobic capsids may then be extracted into an organic phase such as 1,3-bis(trifluoromethyl)benzene. Purification of capsids, VLPs or proteins may include for example at least one liquid-liquid extraction step, at least one fractional precipitation step, at least one ultrafiltration step, or at least one crystallization step. A liquid-liquid extraction may comprise for example use of an immiscible non-aqueous non-polar solvent, such as but not limited to benzene, toluene, hexane, heptane, octane, chloroform, dichloromethane, or carbon tetrachloride. Purifying may include at least one crystallization step. Use of one or more hydrolytic steps, and especially of one or more proteolytic steps, eliminates certain problems observed with current separation processes used for cargo molecules, which are mainly result from the large number and varying degree of binding interactions which take place between cargo molecules and components derived from the cell culture in which they are produced. The capsids described herein resist hydrolytic steps such that the matrix which results after hydrolysis includes intact capsids which safely partition any cargo molecules from the surrounding matrix, thereby interrupting the troublesome binding interactions which interfere with current purification processes.

Following purification, the capsid can be opened to obtain the cargo molecule, which maybe a protein or polypeptide, a peptide, or a nucleic acid molecule as described herein. Capsids can be opened using any one of several possible procedures known in the art, including for example heating in an aqueous solution above 50°C; repeated freeze-thawing; incubating with denaturing agents such as formamide; by incubating with one or more proteases; or by a combination of any of these procedures.

Capsid proteins which are resistant to hydrolases and useful in the VLPs and methods according to the present disclosure can also be variants of, or derived from the wild type MS2 capsid protein. Capsid proteins may comprise, for example, at least one substitution, deletion or insertion of an amino acid residue relative to the wild type MS2 capsid amino acid sequence. Such capsid proteins may be naturally occurring variants or can be obtained by genetically modifying the MS2 capsid protein using conventional techniques, provided that the variant or modified capsid protein forms a non-enveloped capsid which is resistant to hydolysis catalyzed by a peptide bond hydrolase category EC 3.4 as described herein.

Genetically modified MS2 capsid proteins which can assemble into capsids which are resistant to hydrolysis as described herein can be engineered by making select modifications in the amino acid sequence according to conventional and well-known principles in physical chemistry and biochemistry to produce a protein which retains resistance to hydrolysis as described herein and in the Examples herein below.

It is common knowledge for example that the shape or global fold of a functional protein is determined by the amino acid sequence of the protein, and that the fold defines the protein's function. The global fold is comprised of one or more folding domains. When more than one folding domain exists in the global fold, the domains generally bind together, loosely or tightly along a domain interface. The domain fold can be broken down into a folding core of tightly packed, well-defined secondary structure elements which is primarily responsible for the domain's shape and a more mobile outer layer typically comprised of turns and loops whose conformations are influenced by interactions with the folding core as well as interactions with nearby domains and other molecules, including solvent and other proteins. An extensive public domain database of protein folds, the Structural Classification of Proteins (SCOP) database (Alexey G Murzin, Curr Opin Struct Biol (1996) 6, 386-394) of solved protein structures in the public domain is maintained online at http://scop.berkeley.edu and regularly expanded as new solved structures enter the public domain (Protein Data Bank (F.C.Bernstein, T.F.Koetzle, G.J.Williams, E.E.Meyer Jr., M.D.Brice, J.R.Rodgers, O.Kennard, T.Shimanouchi, M. Tasumi, "The Protein Data Bank: A Computer-based Archival File For Macromolecular Structures," J. of. Mol. Biol., 112 (1977): 535), http://www.rcsb.org) database. Members of a family which are evolutionarily distant, yet have the same shape and very similar function, commonly retain as few as 30% identical residues at topologically and/or functionally equivalent positions. In some families, sequences of distant members have as few as 20% of their residues unchanged with respect to each other, *e.g.* levi- and alloleviviridae capsid proteins. Further, the fold and function of a protein is remarkably tolerant to change via directed or random mutation, even of core residues (Peter O. Olins‡, S. Christopher Bauer, Sarah Braford-Goldberg, Kris Sterbenz, Joseph O. Polazzi, Maire H. Caparon, Barbara K. Klein, Alan M. Easton, Kumnan Paik, Jon A. Klover, Barrett R. Thiele, and John P. McKearn (1995) J Biol Chem 270, 23754-23760; Yiqing Feng, Barbara K. Klein and Charles A. McWherter (1996), J Mol Biol 259, 524-541; Dale Rennell, Suzanne E. Bouvier, Larry W. Hardy and Anthony R. Poteetel (1991) J Mol Biol 222, 67-87), insertion/deletion of one or more residues (Yiqing Feng, Barbara K. Klein and Charles A. McWherter (1996), J Mol Biol 259, 524-541), permutation of the sequence (Multi-functional chimeric hematopoietic fusion proteins between sequence rearranged c-mpl receptor agonists and other hematopoietic factors, US 6066318), concatenation via the N- or C-terminus or both (to copies of itself or other peptides or proteins) (Multi-functional chimeric hematopoietic fusion proteins between sequence rearranged g-csf receptor agonists and other hematopoietic factors , US20040171115; Plevka, P., Tars, K., Liljas, L. (2008) Protein Sci. 17: 173) or covalent modification, e.g., glycosylation, pegylation, SUMOylation or the addition of peptidyl or nonpeptidyl affinity tags as long as the residues critical to maintaining the fold and/or function are spared.

VLPs according to the present disclosure and as used in any of the methods and processes, thus encompass those comprising a capsid protein having at least 15%, 16%, 21%, 40%, 41%, 52%, 53%, 56%, 59% or at least 86% sequence identity with the amino acid sequence of wild type Enterobacteria phage MS2 capsid protein (SEQ ID NO: 3) and is resistant to hydrolysis catalyzed by a peptide bond hydrolase category EC 3.4. Such VLPs include for example a VLP comprising a capsid protein having at least 52% sequence identity with SEQ ID NO: 3) as described above. Also included is a VLP comprising a capsid protein having at least 53% sequence identity to SEQ ID NO:3, which can be obtained substantially as described above but not disregarding the FR capsid sequence, representing 53% sequence identity to wild-type enterobacteria phage MS2 capsid protein (SEQ ID NO:3). Also included is a VLP comprising a capsid protein having at least 56% sequence identity to SEQ ID NO:3, when it is considered that when the structures identified as 1AQ3 (van den Worm, S.H., Stonehouse, N.J., Valegard, K., Murray, J.B., Walton, C., Fridborg, K., Stockley, P.G., Liljas, L. (1998) Nucleic Acids Res. 26: 1345-1351), 1GAV (Tars, K., Bundule, M., Fridborg, K., Liljas, L. (1997) J.Mol.Biol. 271: 759-773), 1FRS (Liljas, L., Fridborg, K., Valegard, K., Bundule, M., Pumpens, P. (1994) J.Mol.Biol. 244: 279-290) and 2VTU (Plevka, P., Tars, K., Liljas, L. (2008) Protein Sci. 17: 1731) (Protein Data Bank identifiers described above), only 56% of the sequence positions have identical sequence and topologically equivalent positions with respect to the backbone overlays when all three sequences are considered together. Also included is a VLP comprising a capsid protein having at least 59% sequence identity to SEQ ID NO:3, when it is considered that the sequence of the MS2 viral capsid protein compared to that of the GA viral capsid protein is 59%. Also included is a VLP comprising a capsid protein having at least 86% sequence identity to SEQ ID NO:3, when it is considered that the sequence of the MS2 viral capsid protein compared to that of the FR capsid protein is 86%. VLPs according to the present disclosure thus encompass those comprising a capsid protein having at least 15%, 16%, or 21% sequence identity with the amino acid sequence of wild type Enterobacteria phage MS2 capsid (SEQ ID NO:3) based on a valid structure anchored alignment and is resistant to hydrolysis catalyzed by a peptide bond hydrolase category EC 3.4.

A VLP may thus comprise any of the MS2 capsid protein variants as described herein. Genetically modified capsid proteins consistent with those described herein can be produced for example by constructing at least one DNA plasmid encoding at least one capsid protein having at least one amino acid substitution, deletion or insertion relative to the amino acid sequence of the wild type MS2 capsid protein, making multiple copies of each plasmid, transforming a cell line with the plasmids; maintaining the cells for a time and under conditions sufficient for the transformed cells to express and assemble capsids encapsidating nucleic acids; lysing the cells to form a cell lysate; subjecting the cell lysate to hydrolysis using at least one peptide bond hydrolase, category EC 3.4; and removing intact capsids remaining in the cell lysate following hydrolysis to obtain capsids having increased resistance to at least one hydrolase relative to the wild type capsid protein. Following purification of the resulting, intact capsids, an amino acid sequence for each capsid protein may be determined according to methods known in the art.

The specialized capsids described herein can be used in research and development and in industrial manufacturing facilities to provide improved yields, since the purification processes used in both settings have the same matrix composition. Having such same composition mainly depends on using the same cell line in both research and development and manufacturing processes. However, differences in matrix composition due to using different cell lines are greatly reduced after proteolytic steps used in both research and development and manufacturing stages. This feature enables use of different cell lines in both stages with a minimal manufacturing yield penalty.

Further aspects and embodiments of the invention are set out in the following numbered paragraphs 1-133 which form part of the description.
1. A virus-like particle (VLP) comprising a capsid enclosing at least one heterologous cargo molecule and a packing sequence.
2. A VLP according to paragraph 1, further comprising at least one ribozyme enclosed by the capsid.
3. A VLP according to paragraph 2, wherein the heterologous cargo molecule comprises an oligonucleotide.
4. A VLP according to paragraph 3, wherein the heterologous cargo molecule comprises an oligoribonucleotide.
5. A VLP according to paragraph 4, wherein the ribozyme is flanked by the packing sequence and the oligoribonucleotide to form a nucleic acid construct.
6. A VLP according to paragraph 5, comprising a plurality of the nucleic acid constructs.
7. A VLP according to paragraph 4, wherein the oligoribonucleotide is a short RNA selected from siRNA, shRNA, sshRNA, lshRNA and miRNA.
8. A VLP according to paragraph 7, comprising at least two ribozymes, wherein each ribozyme is selected to cut one end of the short RNA.
9. A VLP according to paragraph 4, further comprising a linker consisting of at least 1 to 100 nucleotides, the linker comprising at least 40% A's or at least 40% U's, wherein the linker links the oligoribonucleotide and the packing sequence, or the oligoribonucleotide and the ribozyme,
10. A VLP according to paragraph 2, wherein the ribozyme is selected from a Hammerhead ribozyme and a Hepatitis Delta V ribozyme.
11. A VLP according to paragraph 2, wherein the ribozyme is a Hammerhead ribozyme variant having a contiguous set of nucleotides complementary to at least 6 contiguous nucleotides of the oligoribonucleotide.
12. A VLP according to paragraph 2, wherein the ribozyme is a mutant Hepatitis Delta V ribozyme capable of cleaving its connection with the oligoribonucleotide at a rate at most about 50% the rate of a wildtype Hepatitis Delta V ribozyme.
13. A VLP according to paragraph 2, wherein the ribozyme is a mutant HDV ribozyme having a nucleic acid sequence selected from SEQ ID Nos: 10-18.
14. A VLP according to paragraph 1, wherein the capsid comprises a wild type viral capsid which is resistant to hydrolysis catalyzed by a peptide bond hydrolase category EC 3.4.
15. A VLP according to paragraph 1, wherein the capsid comprises a capsid protein having at least 40% sequence identity with the amino acid sequence of wild type Enterobacteria phage MS2 capsid (SEQ ID NO : 3) and is resistant to hydrolysis catalyzed by a peptide bond hydrolase category EC 3.4.
16. A VLP according to paragraph 1, wherein the capsid comprises a capsid protein having at least 41% sequence identity with the amino acid sequence of wild type Enterobacteria phage MS2 capsid (SEQ ID NO: 3) and is resistant to hydrolysis catalyzed by a peptide bond hydrolase category EC 3.4.
17. A VLP according to paragraph 1, wherein the capsid comprises a capsid protein having at least 45% sequence identity with the amino acid sequence of wild type Enterobacteria phage MS2 capsid (SEQ ID NO: 3) and is resistant to hydrolysis catalyzed by a peptide bond hydrolase category EC 3.4.
18. A VLP according to paragraph 1, wherein the capsid comprises a capsid protein having at least 52% sequence identity with the amino acid sequence of wild type Enterobacteria phage MS2 capsid (SEQ ID NO: 3) and is resistant to hydrolysis catalyzed by a peptide bond hydrolase category EC 3.4.
19. A VLP according to paragraph 1, wherein the capsid comprises a capsid protein having at least 53% sequence identity with the amino acid sequence of wild type Enterobacteria phage MS2 capsid (SEQ ID NO: 3) and is resistant to hydrolysis catalyzed by a peptide bond hydrolase category EC 3.4.
20. A VLP according to paragraph 1, wherein the capsid comprises a capsid protein having at least 56% sequence identity with the amino acid sequence of wild type Enterobacteria phage MS2 capsid (SEQ ID NO: 3) and is resistant to hydrolysis catalyzed by a peptide bond hydrolase category EC 3.4.
21. A VLP according to paragraph 1, wherein the capsid comprises a capsid protein having at least 59% sequence identity with the amino acid sequence of wild type Enterobacteria phage MS2 capsid (SEQ ID NO:3) and is resistant to hydrolysis catalyzed by a peptide bond hydrolase category EC 3.4.
22. A VLP according to paragraph 1, wherein the capsid comprises a capsid protein having at least 86% sequence identity with the amino acid sequence of wild type Enterobacteria phage MS2 capsid protein (SEQ ID NO: 3) and is resistant to hydrolysis catalyzed by a peptide bond hydrolase category EC 3.4.
23. A VLP according to paragraph 1, wherein the capsid comprises a wild type Enterobacteria phage MS2 capsid protein having the amino acid sequence of SEQ ID NO: 3.
24. A VLP according to paragraph 1, wherein the capsid comprises a capsid protein having at least 15% sequence identity with the amino acid sequence of wild type Enterobacteria phage MS2 capsid (SEQ ID NO:3) based on a valid structure anchored alignment and is resistant to hydrolysis catalyzed by a peptide bond hydrolase category EC 3.4.
25. A VLP according to paragraph 1, wherein the capsid comprises a capsid protein having at least 16% sequence identity with the amino acid sequence of wild type Enterobacteria phage MS2 capsid (SEQ ID NO:3) based on a valid structure anchored alignment and is resistant to hydrolysis catalyzed by a peptide bond hydrolase category EC 3.4.
26. A VLP according to paragraph 1, wherein the capsid comprises a capsid protein having at least 21% sequence identity with the amino acid sequence of wild type Enterobacteria phage MS2 capsid (SEQ ID NO:3) based on a valid structure anchored alignment and is resistant to hydrolysis catalyzed by a peptide bond hydrolase category EC 3.4.
27. A VLP according to paragraph 1, wherein the heterologous cargo molecule comprises a peptide or a polypeptide.
28. A VLP according to paragraph 27, further comprising an oligonucleotide linker coupling the heterologous cargo molecule and the viral capsid.
29. A VLP according to paragraph 28, wherein the oligonucleotide linker is an oligoribonucleotide comprising a ribozyme sequence.
30. A VLP according to paragraph 1, wherein the heterologous cargo molecule comprises a bi-molecular cargo molecule comprising a bifunctional polynucleotide comprisng a first apatamer sequence which specificlaly binds a bioactive small molecule having a molecular weight of about 1,500 Da or less and a second apatamer sequecne for binding a packing sequence of the capsid.
31. A VLP according to paragraph 30, further comprising the bioactive small molecule bound to the first aptameric sequence.
32. A VLP according to paragraph 31, wherein the bioactive small molecule comprises and herbicide or a pesticide.
33. A VLP according to paragraph 32, wherein the bioactive small molecule is sleceted from the rgoup consiting of: atrazine, acetamipridphorate, profenofos, isocarbophos and omethoateas.
34. A nucleic acid construct comprising a nucleotide sequence that encodes a short RNA, a ribozyme and a packing sequence.
35. A nucleic acid construct according to paragraph 34, wherein the short RNA is an siRNA or an shRNA.
36. A nucleic acid construct according to paragraph 34, further comprising a linking nucleotide sequence of 4 to 100 nucleotides of which at least 40% are A's, at least 40% are T's, or at least 40% are U's, wherein the linking nucleotide sequence is flanked by the packing-coding sequence and by the siRNA-coding sequence.
37. A nucleic acid construct according to paragraph 34, wherein the ribozyme is flanked by the short RNA and the packing sequence.
38. A nucleic acid construct according to paragraph 37, wherein the short RNA is an siRNA or an shRNA.
39. A vector comprising a nucleic acid construct according to paragraph 34.
40. A host cell comprising the vector according to paragraph 39.
41. A host cell according to paragraph 40, wherein the host cell is stably transfected with the vector.
42. A host cell according to paragraph 40, which is a bacterial cell.
43. A host cell according to paragraph 40, which is an *Escherichia coli* cell.
44. A host cell according to paragraph 40, which a plant cell.
45. A host cell according to paragraph 40, which is a mammalian cell.
46. A host cell according to paragraph 40, which a fungal cell.
47. A host cell according to paragraph 40, which a yeast cell.
48. A host cell according to paragraph 40, wherein the host cell is further stably transfected with a second vector comprising a second nucleic acid sequence encoding a viral capsid which is resistant to hydrolysis catalyzed by a peptide bond hydrolase category EC 3.4.
49. A host cell according to paragraph 40, wherein the second nucleic acid sequence encodes a viral protein encoding a viral capsid having at least 40% sequence identity with the amino acid sequence of wild type Enterobacteria phage MS2 capsid protein (SEQ ID NO: 3) and is resistant to hydrolysis catalyzed by a peptide bond hydrolase category EC 3.4.
50. A host cell according to paragraph 40, wherein the nucleic acid sequence encodes a wild type Enterobacteria phage MS2 capsid protein (SEQ ID NO: 3).
51. A nucleic acid construct according to paragraph 34, wherein the ribozyme is a Hammerhead ribozyme.
52. A nucleic acid construct according to paragraph 34, wherein the ribozyme is a Hammerhead ribozyme variant having a contiguous set of nucleotides complementary to at least 6 contiguous nucleotides of the short RNA.
53. A nucleic acid construct according to paragraph 34, wherein the ribozyme is a Hepatitis Delta V ribozyme.
54. A nucleic acid construct according to paragraph 34, wherein the ribozyme is a mutant Hepatitis Delta V ribozyme capable of cleaving its connection with the short RNA at a rate at most 50% the rate of a wildtype Hepatitis Delta V ribozyme, or a mutant HDV ribozyme having a nucleic acid sequence selected from SEQ ID NOs: 10-18.
55. A plant or plant tissue transformed to contain the nucleic acid construct according to paragraph 34.
56. A seed or progeny of the plant or plant tissue of paragraph 55, wherein the seed or progeny comprises the nucleic acid constuct.
57. A composition comprising: a) a plurality of virus-like particles each comprising a viral capsid enclosing at least one heterologous cargo molecule; and b) one or more cell lysis products present in an amount of less than 4 grams for every 100 grams of capsid present in the composition, wherein the cell lysis products are selected from proteins, polypeptides, peptides and any combination thereof.
58. A composition according to paragraph 57, wherein the capsid is resistant to hydrolysis catalyzed by a peptide bond hydrolase category EC 3.4.
59. A composition according to paragraph 57, wherein the capsid comprises a capsid protein having at least 40% sequence identity with the amino acid sequence of wild type Enterobacteria phage MS2 capsid (SEQ ID NO: 3) and is resistant to hydrolysis catalyzed by a peptide bond hydrolase category EC 3.4.
60. A composition according to paragraph 57, wherein the capsid comprises a wild type Enterobacteria phage MS2 capsid protein (SEQ ID NO: 3).
61. A composition according to paragraph 57, wherein the heterologous cargo molecule comprises an oligonucleotide.
62. A composition according to paragraph 57, wherein the heterologous cargo molecule comprises an oligoribonucleotide selected from siRNA, shRNA, sshRNA, IshRNA and miRNA.
63. A composition according to paragraph 62, wherein each virus-like particle further comprises at least one ribozyme, wherein the ribozyme is flanked by the packing sequence and the oligoribonucleotide to form a nucleic acid construct.
64. A composition according to paragraph 63, wherein each virus-like particle comprises a plurality of the nucleic acid constructs.
65. A composition according to paragraph 63, wherein the ribozyme is selected from a Hammerhead ribozyme and a Hepatitis Delta V ribozyme.
66. A composition according to paragraph 63, wherein the ribozyme is a Hammerhead ribozyme variant having a contiguous set of nucleotides complementary to at least 6 contiguous nucleotides of the oligoribonucleotide.
67. A composition according to paragraph 63, wherein the ribozyme is a mutant Hepatitis Delta V ribozyme capable of cleaving its connection with the oligoribonucleotide at a rate at most about 50% the rate of a wildtype Hepatitis Delta V ribozyme, or a mutant HDV ribozyme having a nucleic acid sequence selected from SEQ ID NOS: 10-18.
68. A composition according to paragraph 63, wherein the oligoribonucleotide and the packing sequence are linked by a nucleotide sequence of 1 to 100 nucleotides in length, such linking sequence comprising more than 40% A's or more than 40% of U's.
69. A composition according to paragraph 57, wherein the heterologous cargo molecule comprises a peptide or a polypeptide.
70. A composition according to paragraph 69, further comprising an oligonucleotide linker coupling the heterologous cargo molecule and the viral capsid.
71. A composition according to paragraph 70, wherein the oligonucleotide linker is an oligoribonucleotide comprising a ribozyme sequence.
72. A composition according to paragraph 57, wherein the cell lysis products are present in an amount of less than 0.5 grams.
73. A composition according to paragraph 57, wherein the cell lysis products are present in an amount of less than 0.2 grams.
74. A composition according to paragraph 57, wherein the cell lysis products are present in an amount of less than 0.1 grams.
75. A method for isolating and purifying a target cargo molecule, the method comprising: (a) obtaining a whole cell lysate comprising a plurality of virus-like particles (VLPs) each comprising a capsid enclosing at least one target cargo molecule, wherein the capsids are resistant to hydrolysis catalyzed by a peptide bond hydrolase category EC 3.4; (b) subjecting the VLP's to hydrolysis using a peptide bond hydrolase category EC 3.4, for a time and under conditions sufficient for at least 60, at least 70, at least 80, or at least 90 of every 100 individual polypeptides present in the whole cell lysate but not enclosed by the capsids to be cleaved, while at least 60, at least 70, at least 80, or at least 90 of every 100 capsids present in the whole cell lysate before such hydrolysis remain intact following the hydrolysis.
76. A method according to paragraph 75, wherein the capsids each comprises a viral capsid protein having at least 40% sequence identity with the amino acid sequence of wild type Enterobacteria phage MS2 capsid protein (SEQ ID NO: 3) and is resistant to hydrolysis catalyzed by a peptide bond hydrolase category EC 3.4.
77. A method according to paragraph 75, wherein the capsids each comprise a wild type Enterobacteria phage MS2 capsid protein (SEQ ID NO: 3).
78. A method according to paragraph 75, wherein the target cargo molecule comprises an oligonucleotide.
79. A method according to paragraph 75, wherein the target cargo molecule comprises an oligoribonucleotide selected from siRNA, shRNA, sshRNA, IshRNA and miRNA.
80. A method according to paragraph 75, wherein each virus-like particle further comprises a ribozyme, wherein the ribozyme is flanked by the packing sequence and the oligoribonucleotide to form a nucleic acid construct.
81. A method according to paragraph 75, further comprising purification of the capsids following hydrolysis.
82. A method according to paragraph 81, wherein purification includes at least one of a liquid-liquid extraction step, a crystallization step, a fractional precipitation step, and an ultrafiltration step.
83. A method according to paragraph 75, wherein the target cargo molecule comprises a peptide or a polypeptide.
84. A composition produced by the method according to paragraph 75.
85. A method for protecting a target molecule from hydrolysis in a whole cell lyste following intracellular production of the target molecule in a host cell, the method comprising: (a) selecting a viral capsid which is resistant to hydrolysis catalyzed by a peptide bond hydrolase category EC 3.4; (b) stably transfecting the host cell with a first vector comprising a nucleic acid sequence encoding a viral protein forming the viral capsid, and a second vector comprising a nucleic acid sequence comprising a ribozyme flanked by a packing sequence and an siRNA sequence; and (c) maintaining the cells for a time and under conditions sufficient for the transformed cells to express and assemble capsids encapsidating the ribozyme flanked by the packing sequence and the siRNA sequence.
86. A process for purifying VLP's enclosing at least one heterologous cargo molecule, the process comprising: (a) obtaining a cell lysate comprising a plurality of the VLP's; (b) contacting the cell lysate with a protease for a time and under conditions sufficient to hydrolyze cell lysis products other than the VLP's to form a hydrolysate; and (c) isolating the VLP's from the hydrolsyate.
87. The process accorfing to paragraph 86, wherein step (c) comprises (i) performing a first precipitation with ammonium sulfate followed by a first centrifugation to obtain a first precipitate and a first supernatant; and (ii) performing a second precipitation on the first supernatant with ammonium sulfate followed by a second centrifugation to obtain a second precipitate, wherein the second precipitate comprises at least about 90% by weight of the VLP's.
88. The process accorfing to paragraph 86, wherein step (c) comprises (i) performing a first precipitation with ethanol followed by a first centrifugation to obtain a first precipitate and a first supernatant; and (ii) performing a second precipitation on the first supernatant with ammonium sulfate followed by a second centrifugation to obtain a second precipitate, wherein the second precipitate comprises at least about 90% by weight of the VLP's.
89. The process accorfing to paragraph 86, wherein step (c) comprises ultracentrifuging the hydrolysate to obtain a precipitate comprising at least about 90% by weight of the VLP's.
90. The process according to paragraph 86, wherein the VLP's each comprise a capsid which is resistant to hydrolysis catalyzed by a peptide bond hydrolase category EC 3.4.
91. The process according to paragraph 86, wherein the VLP's each comprise a capsid which comprises a capsid protein having at least 40% sequence identity with the amino acid sequence of wild type Enterobacteria phage MS2 capsid (SEQ ID NO: 3) and is resistant to hydrolysis catalyzed by a peptide bond hydrolase category EC 3.4.
92. The process according to paragraph 86, wherein the VLP's each comprise a wild type Enterobacteria phage MS2 capsid protein (SEQ ID NO: 3).
93. The process according to paragraph 86, wherein step (b) is performed for at least about 30 minutes at about 37°C.
94. The process according to paragraph 86, further comprising before step (b), contacting the cell lysate with at least one of a nuclease, an amylase and a lypase for at least about 30 minutes at about 37°C.
95. The process according to paragraph 86, wherein the protease is a peptide bond hydrolase category EC 3.4.
96. The process according to paragraph 86, wherein the protease is selected from Proteinase K, Protease from *Streptomyces griseus,* and Protease from *Bacillus lichenformis,* pepsin and papain.
97. The process according to paragraph 86, wherein the heterologous cargo molecule comprises an oligonucleotide.
98. The process according to paragraph 86, wherein the heterologous cargo molecule comprises an oligoribonucleotide.
99. The process according to paragraph 98, wherein the the oligoribonucleotide is a short RNA selected from siRNA, shRNA, sshRNA, IshRNA and miRNA.
100. The process according to paragraph 86, wherein the VLP's each further comprise a ribozyme flanked by a packing sequence and the oligoribonucleotide to form a nucleic acid construct.
101. The process according to paragraph 100, wherein the ribozyme is selected from a Hammerhead ribozyme and a Hepatitis Delta V ribozyme.
102. The process according to paragraph 100, wherein the ribozyme is a Hammerhead ribozyme variant having a contiguous set of nucleotides complementary to at least 6 contiguous nucleotides of the oligoribonucleotide.
103. The process according to paragraph 100, wherein the ribozyme is a mutant Hepatitis Delta V ribozyme capable of cleaving its connection with the oligoribonucleotide at a rate at most about 50% the rate of a wildtype Hepatitis Delta V ribozyme.
104. The process according to paragraph 100, wherein the ribozyme is a mutant HDV ribozyme having a nucleic acid sequence selected from SEQ ID Nos: 10-18.
105. The process according to paragraph 100, wherein the oligoribonucleotide and the packing sequence are linked by a linker sequence of at least 1 to 100 nucleotides, and comprising more than 40% A's or more than 40% of U's.
106. The process according to paragraph 86, wherein the heterologous cargo molecule comprises a peptide or a polypeptide.
107. The process according to paragraph 106, wherein the VLP's each further comprise an oligonucleotide linker coupling the heterologous cargo molecule and the viral capsid.
108. The process according to paragraph 107, wherein the oligonucleotide linker is an oligoribonucleotide comprising a ribozyme sequence.
109. The process according to paragraph 86, further comprising preparing the cell lyaste before step (a) by centrifuging cells following expression of the VLPs in the cells; resuspending the cells; lysing the cells and centrifuging the cell lysate to obtain a supernatant, wherein the supernatant is used as the cell lysate for step (a).
110. A VLP according to paragraph 1, wherein the capsid comprises a capsid protein with the amino acid sequence of wild type Enterobacteria phage MS2 capsid (SEQ ID NO :3) except that the A residue at position 1 is deleted, and is resistant to hydrolysis catalyzed by a peptide bond hydrolase category EC 3.4.
111. A VLP according to paragraph 1, wherein the capsid comprises a capsid protein with the amino acid sequence of wild type Enterobacteria phage MS2 capsid (SEQ ID NO:3) except that the A residue at position 1 is deleted and the S residue at position 2 is deleted, and is resistant to hydrolysis catalyzed by a peptide bond hydrolase category EC 3.4.
112. A VLP according to paragraph 1, wherein the capsid comprises a capsid protein with the amino acid sequence of wild type Enterobacteria phage MS2 capsid (SEQ ID NO:3) except that the A residue at position 1 is deleted, the S residue at position 2 is deleted and the N residue at position 3 is deleted, and is resistant to hydrolysis catalyzed by a peptide bond hydrolase category EC 3.4.
113.A VLP according to paragraph 1, wherein the capsid comprises a capsid protein with the amino acid sequence of wild type Enterobacteria phage MS2 capsid (SEQ ID NO:3) except that the Y reside at position 129 is deleted, and is resistant to hydrolysis catalyzed by a peptide bond hydrolase category EC 3.4.
114. A VLP according to paragraph 1, wherein the capsid comprises a capsid protein with the amino acid sequence of wild type Enterobacteria phage MS2 capsid (SEQ ID NO:3) having a single (1) amino acid deletion in the 112-117 segment and is resistant to hydrolysis catalyzed by a peptide bond hydrolase category EC 3.4.
115. A VLP according to paragraph 1, wherein the capsid comprises a capsid protein with the amino acid sequence of wild type Enterobacteria phage MS2 capsid (SEQ ID NO:3) having a single (1) amino acid deletion in the 112-117 segment and is resistant to hydrolysis catalyzed by a peptide bond hydrolase category EC 3.4.
116.A VLP according to paragraph 1, wherein the capsid comprises a capsid protein with the amino acid sequence of wild type Enterobacteria phage MS2 capsid (SEQ ID NO:3) having a 1-2 residue insertion in the 65-83 segment and is resistant to hydrolysis catalyzed by a peptide bond hydrolase category EC 3.4.
117. A VLP according to paragraph 1, wherein the capsid comprises a capsid protein with the amino acid sequence of wild type Enterobacteria phage MS2 capsid (SEQ ID NO:3) having a 1-2 residue insertion in the 44-55 segment and is resistant to hydrolysis catalyzed by a peptide bond hydrolase category EC 3.4.
118. A VLP according to paragraph 1, wherein the capsid comprises a capsid protein with the amino acid sequence of wild type Enterobacteria phage MS2 capsid (SEQ ID NO:3) having a single (1) residue insertion in the 33-43 segment and is resistant to hydrolysis catalyzed by a peptide bond hydrolase category EC 3.4.
119.A VLP according to paragraph 1, wherein the capsid comprises a capsid protein with the amino acid sequence of wild type Enterobacteria phage MS2 capsid (SEQ ID NO:3) having a 1-2 residue insertion in the 24-30 segment and is resistant to hydrolysis catalyzed by a peptide bond hydrolase category EC 3.4.
120.A VLP according to paragraph 1, wherein the capsid comprises a capsid protein with the amino acid sequence of wild type Enterobacteria phage MS2 capsid (SEQ ID NO :3) having a single (1) residue insertion in the 10-18 segment and is resistant to hydrolysis catalyzed by a peptide bond hydrolase category EC 3.4.
121. A VLP according to paragraph 1, wherein the capsid comprises a capsid protein monomer sequence concatenated with a second capsid monomer sequence which assembles into a capsid which resistant to hydrolysis catalyzed by a peptide bond hydrolase category EC 3.4.
122. A VLP according to paragraph 1, wherein the capsid comprises a capsid protein monomer sequence whose C-terminus is extended with a 0-6 residue linker segment whose C-terminus is concatenated with a second capsid monomer sequence, all of which which assembles into a capsid which resistant to hydrolysis catalyzed by a peptide bond hydrolase category EC 3.4.
123.A VLP according to paragraph 122, wherein the linker sequence is -(Gly)ₓ-, wherein x=0-6, or a Gly-Ser linker selected from -Gly-Gly-Ser-Gly-Gly-, -Gly-Gly-Ser and -Gly-Ser-Gly- .
124.A VLP according to paragraph 122, wherein the capsid comprises a capsid protein concatenated with a third capsid monomer sequence which assembles into a capsid which resistant to hydrolysis catalyzed by a peptide bond hydrolase category EC 3.4.
125.A VLP according to paragraph 122, wherein the capsid comprises a capsid protein wherein the C-terminus is extended with a 0-6 residue linker segment whose C-terminus s concatenated with a third capsid monomer sequence, all of which which assembles into a capsid which resistant to hydrolysis catalyzed by a peptide bond hydrolase category EC 3.4.
126.A VLP according to paragraph 125, wherein the capsid comprises a capsid protein in which one or both linker sequences is -(Gly)ₓ-, wherein x=0-6, or a Gly-Ser linker selected from - Gly-Gly-Ser-Gly-Gly-, -Gly-Gly-Ser and -Gly-Ser-Gly- which assembles into a capsid which is resistant to hydrolysis catalyzed by a peptide bond hydrolase category EC 3.4.
127.A VLP according to paragraph 125, wherein the capsid comprises a capsid protein of in which one or both linker sequences is -(Gly)x-, x=1. which assembles into a capsid which is resistant to hydrolysis catalyzed by a peptide bond hydrolase category EC 3.4.
128.A VLP according to paragraph 125, wherein the capsid comprises a capsid protein of in which one or both linker sequences is -(Gly)x-, x=2. which assembles into a capsid which is resistant to hydrolysis catalyzed by a peptide bond hydrolase category EC 3.4.
129.A VLP according to paragraph 134, wherein the capsid comprises a capsid protein of in which one or both linker sequences is -(Gly)x-, x=3. which assembles into a capsid which is resistant to hydrolysis catalyzed by a peptide bond hydrolase category EC 3.4.
130.A VLP according paragraph 1, wherein one or more coat protein sequences is N-terminally truncated by 1-3 residues and a linker sequence is lengthened by the number of residues deleted, and which is resistant to hydrolysis catalyzed by a peptide bond hydrolase category EC 3.4, wherein the linker sequence is -(Gly)ₓ-, wherein x=0-6.
131.A VLP according paragraph 1, wherein one or more coat protein sequences is C-terminally truncated by 1 residue and a linker sequence is lengthened by the one residue and which is resistant to hydrolysis catalyzed by a peptide bond hydrolase category EC 3.4, wherein the linker sequence is -(Gly)ₓ-, wherein x=0-6.
132.A VLP according paragraph 1, wherein the first coat protein sequence in a concatenated dimer is C-terminally truncated by 1 residue and a linker sequence is lengthened by the one residue or wherein the first and/or second coat protein sequence in a concatenated trimer is C-terminally truncated by 1 residues and which is resistant to hydrolysis catalyzed by a peptide bond hydrolase category EC 3.4, wherein the linker sequence is -(Gly)ₓ-, wherein x=0-6.
133.A VLP according paragraph 1, containing N- and C-terminal truncations and which is resistant to hydrolysis catalyzed by peptide bond hydrolase category EC 3.4.

### EXAMPLES

The following non-limiting examples are included to illustrate various aspects of the present disclosure. It will be appreciated by those of skill in the art that the techniques disclosed in the following examples represent techniques discovered by the Applicants to function well in the practice of the invention, and thus can be considered to constitute preferred modes for its practice. However, those of skill in the art should, in light of the instant disclosure, appreciate that many changes can be made in the specific examples described, while still obtaining like or similar results, without departing from the scope of the invention. Thus, the examples are exemplary only and should not be construed to limit the invention in any way. To the extent necessary to enable and describe the instant invention, all references cited are herein incorporated by reference.

### Example A: Propagation of MS2 bacteriophage

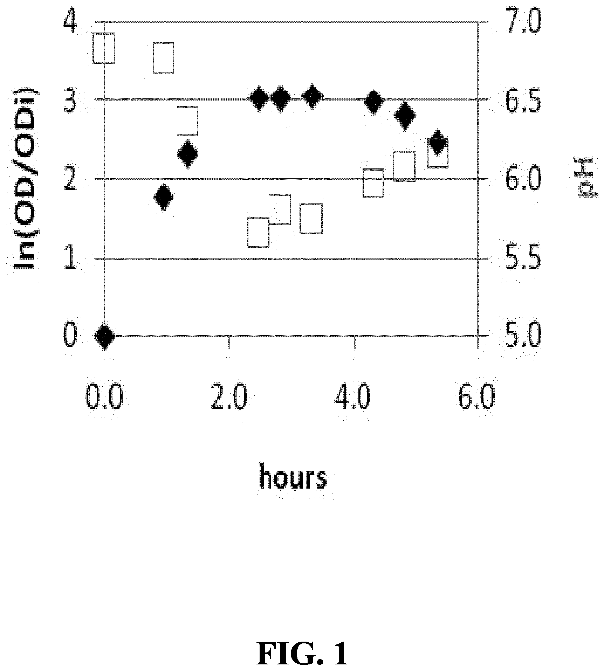

MS2 bacteriophage (ATCC No.15597-B1, from American Type Culture Collection, Rockville, MD) and its *E. Coli* host (ATCC No. 15669) were obtained from ATCC and propagated using the procedure described by Strauss and Sinsheimer (1963) J. Mol. Biol 7:43-54 J. Mol. Biol 7:43-54. Results are plotted in Figure 1. Optical Density (OD) at 600 nm and pH were followed during the reaction. ODi represents OD immediately after inoculation with host. Infection was done at 2.3 hours. Ln(OD/ODi) was plotted on the left axis (full diamonds) and pH was plotted on the right axis (open squares). This experiment was ended 5.3 hours after inoculation with host. Lysate obtained was centrifuged at 2,000 g and filtered through a 0.2µm membrane to eliminate remaining bacteria and bacterial debris.

### Example B: Purification of MS2 bacteriophage using Proteinase K and ultrafiltration

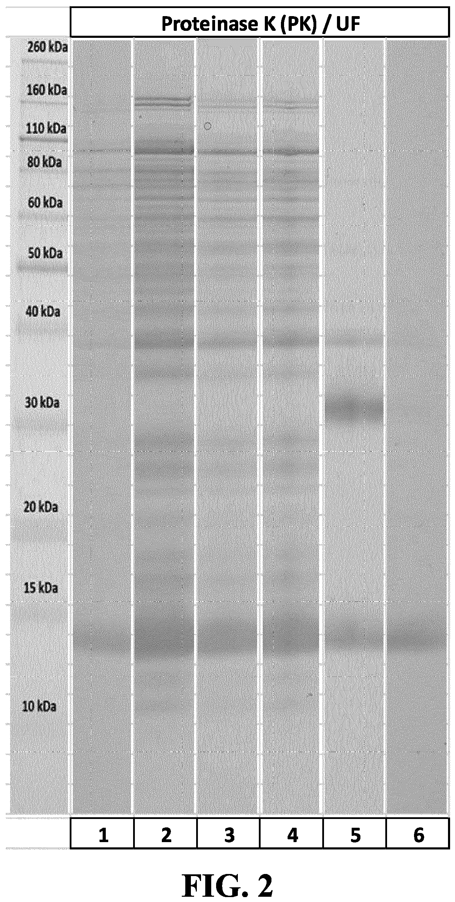

Purification of MS2 bacteriophage was conducted as follows. Samples were taken during purification and SDS PAGE analysis was run on the samples. Results obtained are shown in Figure 2.

8 mL lysate obtained at end of Example A (sample in Lane 1, Figure 2) was filtered through a 300 kDa membrane (Vivaspin 2, from Sartorius Stedim, Bohemia, NY) and the filtrate was filtered through a 100 kDa membrane, from which 1 mL ofretentate was obtained (sample in Lane 2, Figure 2). This retentate was divided in two equal parts. To one half (control) 206µL 20mM CaCl₂ aqueous solution at pH = 7.5 was added. To the second half (Proteinase) 0.15 mg Proteinase K (Sigma Aldrich, St. Louis, MO) dissolved in 206µL 20mM CaCl₂ aqueous solution at pH = 7.5 and was added. Both tubes were incubated at 37°C and after 1 hour they were placed in an ice-water bath. Samples were then taken and analyzed: control sample in Lane 3, Figure 2, and Proteinase sample in Lane 5, Figure 2. Each product was then diluted to 2 mL with deionized (DI) water and filtered through a 100 kDa membrane. Each retentate (150 µL) was diluted to 2 mL with DI water and filtered again through the same membrane. Dilution and ultrafiltration was repeated one more time for each product. Samples of each retentate were then taken and analyzed: control sample in Lane 4, Figure 2, and Proteinase sample in Lane 6, Figure 2. Band at 14 kDa corresponds to MS2 bacteriophage's coat protein. Band at 30 kDa corresponds to Proteinase K. Product from control experiment yields a highly impure phage. Product from Proteinase experiment yields a product containing phage with purity higher than 99%.

### Example C: Degradation of MS2 bacteriophage

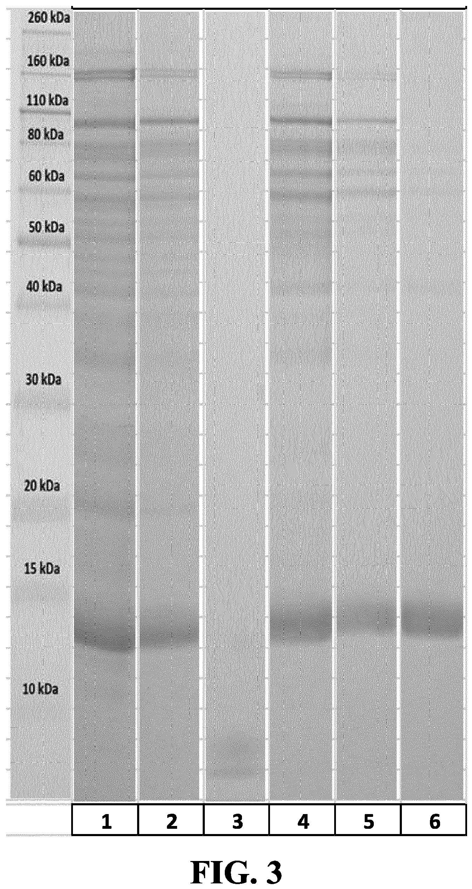

Treatment of MS2 bacteriophage was conducted as follows. Samples were taken during treatment and SDS PAGE analysis was run on the samples. Results obtained are shown in Figure 3. 4 mL lysate obtained at end of Example A was partially purified by precipitation using ammonium sulfate and extraction using trichlorofluoromethane (Freon 11) as described by Strauss & Sinsheimer (1963) J. Mol. Biol 7:43-54. A sample of the aqueous solution after extraction with Freon 11 was taken and analyzed (sample in Lane 1, Figure 3). To the partially purified phage solution (130 µL) 370 µL of 20 mMCaCl₂ aqueous solution was added. The mixture was incubated at 37°C and after 1 hour it was placed in an ice-water bath. A sample was then taken and analyzed: sample in Lane 2, Figure 3. The incubation product was diluted to 2 mL with deionized (DI) water and filtered through 100 kDa membrane. The retentate (150 µL) was diluted to 2 mL with DI water and filtered again through the same membrane. Dilution and ultrafiltration of the retentate was repeated one more time. A sample of the retentate was then taken and analyzed: sample in Lane 3, Figure 3. Only weak bands at lower than 10 kDa were observed, indicating complete degradation of phage.

### Example D: Purification of MS2 bacteriophage using ultrafiltration.

Purification of MS2 bacteriophage was conducted as follows. Samples were taken during purification and SDS PAGE analysis was run on the samples. Results obtained are shown in Figure 3. 4 mL lysate obtained at end of Example A was partially purified by precipitation using ammonium sulfate and extraction using trichlorofluoromethane (Freon 11) as described by Strauss & Sinsheimer (1963) J. Mol. Biol 7:43-54. The aqueous solution containing partially purified phage was diluted to 2 mL with deionized water, filtered through a 300 kDa membrane and the filtrate was filtered through a 100 kDa membrane, from which 150µL of retentate was obtained. The retentate was then diluted to 2 mL with deionized (DI) water and filtered through the same 100 kDa membrane. Dilution and ultrafiltration of the retentate (150µL) was repeated one more time. A sample of the retentate was then taken and analyzed: sample in Lane 4, Figure 3.370 µL of 20 mMCaCl₂ aqueous solution was added to the retentate (130µL). The mixture was incubated at 37°C and after 1 hour it was placed in an ice-water bath. A sample was then taken and analyzed: sample in Lane 5, Figure 3. The product was then diluted to 2 mL with deionized (DI) water and filtered through a 100 kDa membrane. The retentate (150 µL) was diluted to 2 mL with DI water and filtered again through the same membrane. Dilution and ultrafiltration of the retentate was repeated one more time. A sample of the retentate was then taken and analyzed: sample in Lane 6, Figure 3. MS2's coat protein, of 14 kDa, retained by a membrane through which permeate proteins with less than 100 kDa molecular weight, is clearly visible, indicating the presence of intact MS2 capsids. The product obtained contained phage with purity higher than 99%.

### Example E: Purification of MS2 bacteriophage using Proteinase K, and ultrafiltration.

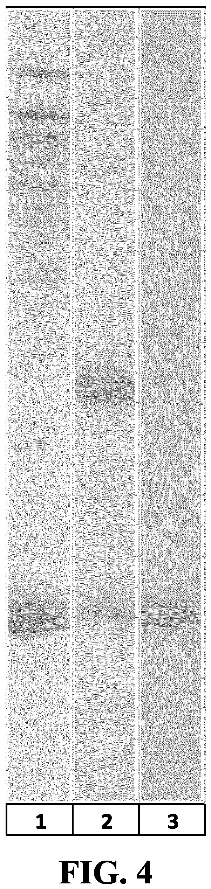

Purification of MS2 bacteriophage was conducted as follows. Samples were taken during purification and SDS PAGE analysis was run on the samples. Results obtained are shown in Figure 4.

4 mL lysate obtained at end of Example A was partially purified by precipitation using ammonium sulfate and extraction using trichlorofluoromethane (Freon 11) as described by Strauss & Sinsheimer (1963) J. Mol. Biol 7:43-54. The aqueous solution containing partially purified phage was diluted to 2 mL with deionized water, filtered through a 100 kDa membrane, from which 150µL of retentate was obtained. The retentate was then diluted to 2 mL with deionized (DI) water and filtered through the same 100 kDa membrane. Dilution and ultrafiltration of the retentate (150µL) was repeated one more time. A sample of the retentate was then taken and analyzed: sample in Lane 1, Figure 4. 0.15 mg of Proteinase K dissolved in 370 µL of 20 mMCaCl₂ aqueous solution was added to the retentate (130µL). The mixture was incubated at 37°C and after 1 hour it was placed in an ice-water bath. A sample was then taken and analyzed: sample in Lane 2, Figure 4. The product was then diluted to 2 mL with deionized (DI) water and filtered through a 100 kDa membrane. The retentate (150 µL) was diluted to 2 mL with DI water and filtered again through the same membrane. Dilution and ultrafiltration of the retentate was repeated one more time. A sample of the retentate was then taken and analyzed: sample in Lane 3, Figure 4. The product obtained contained phage with purity higher than 99%.

### Example F: Purification of MS2 bacteriophage using Proteinase K, precipitation at acidic conditions, precipitation using ethanol at basic and acidic conditions, and ultrafiltration.

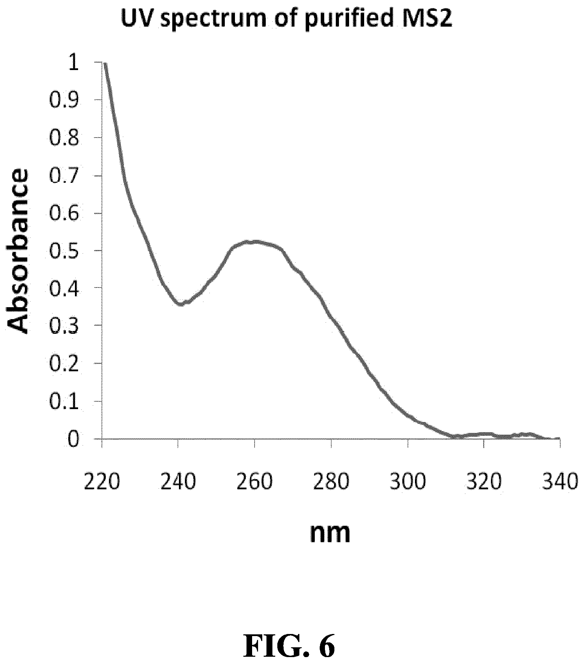

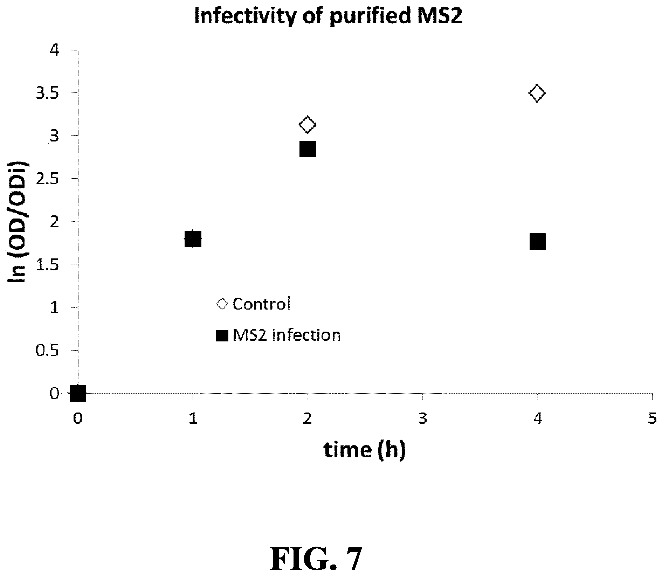

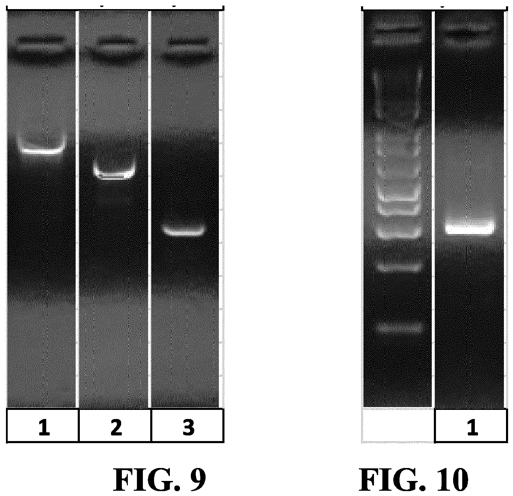

Purification of MS2 bacteriophage was conducted as follows. Samples were taken during purification and SDS PAGE analysis was run on the samples. Results obtained are shown in Figure 5. 50 mL lysate obtained at end of Example A was partially purified by precipitation using ammonium sulfate and extraction using trichlorofluoromethane (Freon 11) as described by Strauss & Sinsheimer (1963) J. Mol. Biol 7:43-54. A sample of the aqueous solution after extraction with Freon 11 was taken and analyzed (sample in Lane 1, Figure 5). To the partially purified phage solution (1.2 mL) 0.9 mg of Proteinase K dissolved in 1.24 mL of 20 mMCaCl₂ aqueous solution was added. The mixture was incubated at 37°C and after 1 hour60 µL of 0.2M Phenylmethanesulfonyl fluoride (PMSF) solution in ethanol was added to inactivate Proteinase K. The mixture was then placed in an ice-water bath. A sample was taken and analyzed: sample in Lane 2, Figure 5.0.68 mL of 0.1% phosphoric acid aqueous solution was slowly added with vigorous agitation in an ice/water bath to bring the pH of the liquid to 4. The liquid was kept at 0°C for 30 minutes and centrifuged at 16,000 g at 4°C for 30 min. The supernatant was allowed to reach room temperature and 130 µL of 1% NaOH was added to bring the pH of the liquid to 8. 0.81 mL of ethanol at room temperature was slowly added with vigorous agitation to bring the ethanol concentration in the liquid to 20%. The liquid was kept at room temperature for 30 min and centrifuged at 16,000 g at room temperature for 30 min. The supernatant was placed in an ice/water bath for 15 min and 1.3 mL of 1% acetic acid was slowly added at 0°C with vigorous agitation to bring the pH of the liquid to 4. 1.5 mL of ethanol at 0°C was slowly added with vigorous agitation to bring the ethanol concentration in the liquid to 34%.The liquid was kept at 0°C for 30 minutes and centrifuged at 16,000 g at 4°C for 30 min. The pellet was resuspended in 200 µL of DI water and a 20 µL sample was taken and analyzed: Lane 3, Figure5. The rest (180µL) was diluted with DI water to 2 mL and filtered through 100 kDa membrane. The retentate (150 µL) was diluted to 2 mL with DI water and filtered again through the same membrane. Dilution and ultrafiltration of the retentate was repeated one more time. A sample of the retentate was then taken and analyzed by SDS PAGE: sample in Lane 4, Figure 5. MS2's coat protein, of 14 kDa, retained by a membrane through which proteins with less than 100 kDa molecular weight are able to permeate, is clearly visible, consistent with the presence of intact MS2 capsids.A UV spectrum on the same retentate is shown in Figure 6, which is consistent with results published by G. F. Rohrmann and R. G. Krueger, (1970) J. Virol., 6(3):26 for pure MS2 phage. A Superdex 200 (GE Healthcare, Piscataway, NJ) size exclusion chromatography was run on the same retentate using Tris-buffered saline at pH 7.4 and 150 mM NaCl. It showed 280 nm absorbance only at the void volume of the column. There was no absorbance in the elution volume for proteins of 600 kD to 2 kD. This test is consistent with intact phage particles. RNA was isolated from another sample of the same retentate using a QIAamp Viral RNA Mini Kit (Qiagen, Valencia, CA) and a DNA-free kit (Life Technologies, Grand Island, NY), and reverse transcribed using a High Capacity cDNA Reverse Transcription Kit (Life Technologies). The presence or absence of three different sections of the MS2 genome was then interrogated in PCR experiments. The following pairs of primers were used, each primer named for the position of its first and last base in the MS2 genome, forward (F) and reverse (R) respectively:F1001_1021-R2180_2201, F1201_1223-R1979_2001, F1401_1426-R1680_1705. Platinum Taq DNA Polymerase High Fidelity (Life Technologies) was used for amplification. PCR products, chromatographed in 1.5% agarose gel stained with Ethidium Bromide, as shown in Figure 9 (1.2 kbp for primers F1201_1223-R1979_2001 in Lane 1, 800 bp for primers F1201_1223-R1979_2001 in Lane 2, and 304 bp for primers F1401_1426-R1680_1705 in Lane 3),were consistent with an intact MS2 bacteriophage genome. An infectivity test was also run on the same retentate as follows. 5 µL of retentate were used to infect 1 mL of bacterial culture as described in Example A at the point it reached OD(600nm) = 0.22. OD(600nm) was 0.621 hour after of infection and dropped to 0.21 after 2 additional hours, while during the same time a control sample attained OD(600nm) of 0.82 1 hour after infection and 1.2 after 2 additional hours, as shown in Figure 7. This test showed a highly infectious phage in the retentate and therefore demonstrated that the purification processes used to isolate it did not compromise its integrity. In conclusion, the product obtained contained MS2 bacteriophage with purity higher than 99%.

### Example G: Purification of MS2 bacteriophage using different exogenous proteases, and ultrafiltration.

Purification of MS2 bacteriophage using different exogenous proteases was attempted substantially as described in Example E, with the exception that proteases other than Proteinase K were used. MS2 bacteriophage was successfully purified after proteolysis promoted by Protease from *Bacillus licheniformis* (P5380, Sigma Aldrich). However, a proteolysis reaction using Pepsin from porcine gastric mucosa (P6887, Sigma Aldrich) at pH of 1.6 was found to significantly degrade MS2 bacteriophage. On the other hand, proteolysis reactions using Papain from papaya latex (P3125, Sigma Aldrich) at pH 6 did not extensively degrade MS2 bacteriophage.

### Example H: Production of MS2 Capsids encapsidating RNA coding for its coat protein attached to its specific 19-mer RNA hairpin.

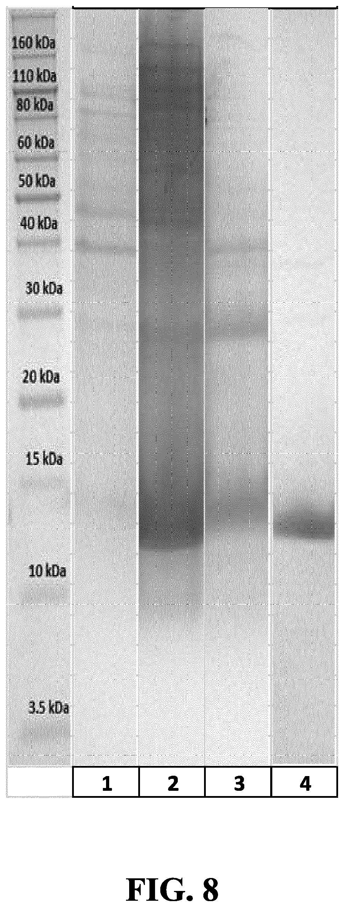

Production of MS2 capsids was conducted as follows. Samples were taken during the course of expression and SDS PAGE analysis was run on the samples to monitor capsid production. Results obtained are shown in Figure 8. The following DNA sequence, encoding MS2's coat protein and its specific RNA 19-mer PAC site was cloned into pDEST14_A252 plasmid (Life Technologies):

ACAAGTTTGTACAAAAAAGCAGGCTAAGAAGGAGATATACATATGGCTTCTAACTT
TACTCAGTTCGTTCTCGTCGACAATGGCGGAACTGGCGACGTGACTGTCGCCCCAAG
CAACTTCGCTAACGGGGTCGCTGAATGGATCAGCTCTAACTCGCGTTCACAGGCTTA
CAAAGTAACCTGTAGCGTTCGTCAGAGCTCTGCGCAGAATCGCAAATACACCATCA
AAGTCGAGGTGCCTAAAGTGGCAACCCAGACTGTTGGTGGTGTAGAGCTTCCTGTA
GCCGCATGGCGTTCGTACTTAAATATGGAACTAACCATTCCAATTTTCGCTACGAAT
TCCGACTGCGAGCTTATTGTTAAGGCAATGCAAGGTCTCCTAAAAGATGGAAACCC
GATTCCCTCAGCAATCGCAGCAAACTCCGGCATCTACTAATAGACGCCGGCCATTCA
AACATGAGGATTACCCATGTACCCAGCT (SEQ ID NO: 6)

One Shot BL21(DE3) Chemically Competent E. coli (Life Technologies) cells were transformed using such plasmid. BL21(DE3) containing the plasmid were grown in 750 mL of LB medium containing ampicillin at 37°C, to OD(600nm) equal to 0.8. A pre-induction sample was then taken and analyzed: sample in Lane 1, Figure 8. Isopropyl β-D-1-thiogalactopyranoside (Sigma-Aldrich) was then added to a final concentration of 1 mM. Four hours post-induction cells were harvested by centrifugation at 3,000g and 4°C for 40 min. A sample was then taken and analyzed: sample in Lane 2, Figure 8.

### Example I: Purification and characterization of MS2 Capsids encapsidating RNA coding for its coat protein attached to its specific 19-mer RNA hairpin.

Purification of MS2 capsids was conducted as follows. Samples were taken during purification and SDS PAGE analysis was run on the samples. Results obtained are shown in Figure 8. A fraction of the pellet from Example H equivalent to 115 mL of culture was resuspended in 20 mM Tris-HCl, pH 7.5, containing 10 mM MgCl2 and sonicated to lyse cells. Cell debris was removed by centrifugation at 16,000 g. The cell lysate obtained was partially purified by precipitation using ammonium sulfate and extraction using trichlorofluoromethane (Freon 11) as described by Strauss & Sinsheimer (1963) J. Mol. Biol 7:43-54. To the partially purified MS2 capsid solution (1.05 mL) 0.3 mg of Proteinase K dissolved in 1.05 mL of 20 mM CaCl2 aqueous solution was added. The mixture was incubated at 37°C and after 2.5 hours it was placed in an ice-water bath. A sample was then taken and analyzed: sample in Lane 3, Figure 8. Fifteen minutes afterwards, 0.14 mL of 1% phosphoric acid aqueous solution was slowly added with vigorous agitation in an ice/water bath to bring the pH of the liquid to 4.1. The liquid was kept at 0°C for 30 minutes and centrifuged at 16,000 g at 4°C for 20 min. To the supernatant, kept at 0°C, 100 µL of 1% NaOH was added to bring the pH of the liquid to 7.9. 0.5 mL of ethanol at 0°C was then slowly added with vigorous agitation to bring the ethanol concentration in the liquid to 20%. The liquid was kept at 0°C for 30 minutes and centrifuged at 16,000 g at 4°C for 20 min. After adding 1% acetic acid to adjust the pH of the solution to 7, the supernatant was filtered through a Vivaspin 2 (Sartorius) 300 kDa membrane and the filtrate was filtered through a 100 kDa membrane, from which 150µL of retentate was obtained. The retentate was then diluted to 2 mL with phosphate buffered saline and filtered through the same 100 kDa membrane. Dilution and ultrafiltration of the retentate (150µL) was repeated four more times. A sample of the retentate was then taken and analyzed by SDS PAGE: sample in Lane 4, Figure 8.MS2's coat protein, of 14 kDa, retained by a membrane through which proteins with less than 100 kDa molecular weight are able to permeate, is clearly visible, consistent with the presence of intact MS2 capsids. RNA was isolated from another sample of the same retentate using a QIAamp Viral RNA Mini Kit (Qiagen, Valencia, CA) and a DNA-free kit (Life Technologies, Grand Island, NY), and reverse transcribed using a High Capacity cDNA Reverse Transcription Kit (Life Technologies). The presence or absence of a section of the MS2 capsid was then interrogated in PCR experiments. The following pair of primers was used, each primer named for the position of its first and last base in the MS2 genome, forward (F) and reverse (R) respectively: F1401_1426-R1680_1705. Platinum Taq DNA Polymerase High Fidelity (Life Technologies) was used for amplification. The PCR product, chromatographed in 2% agarose gel stained with Ethidium Bromide, as shown in Figure 10 (304 bp in Lane 1; the leftmost Lane corresponds to 1 kb plus ladder from Life Technologies), was consistent with an intact MS2 coat gene. In conclusion, the product obtained contained MS2 capsids with purity higher than 99%.

### Example J: Simple precipitation with ethanol for purification of MS2 Virus-Like Particles (VLPs).

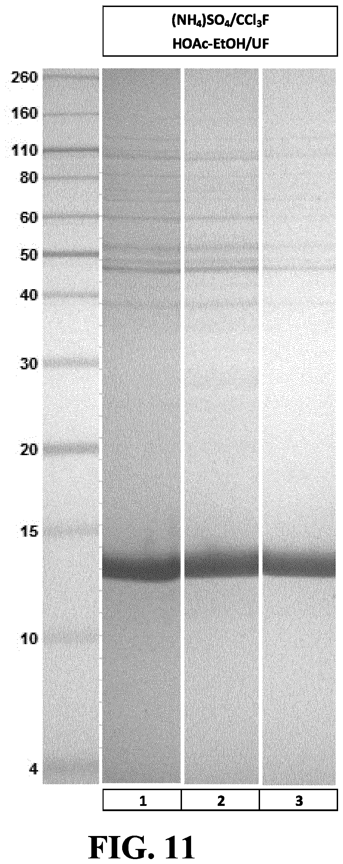

Purification of MS2 VLPs was conducted as follows. Samples were taken during purification and SDS PAGE analysis was run on the samples. Results obtained are shown in Figure 11. One sixth of the pellet obtained from an experiment identical to Example H was resuspended in 20 mM Tris-HCl, pH 7.5, containing 10 mMMgCl₂ and sonicated to lyse cells. Cell debris was removed by centrifugation at 16,000 g. The cell lysate obtained was partially purified by precipitation using ammonium sulfate and extraction using trichlorofluoromethane (Freon 11) as described by Strauss & Sinsheimer (1963) J. Mol. Biol 7:43-54. A sample was taken and analyzed: sample in Lane 1, Figure 11. A strong band at about 14 kDa was found, consistent with the coat protein of MS2 phage. Other bands - impurities - mostly of higher molecular weight, represent about 27% of the sample weight. To the partially purified MS2 VLP solution (1.35 mL) 1.36 mL of 20 mM CaCl2 aqueous solution was added and placed in an ice-water bath. Fifteen minutes afterwards, 50 µL of 10% acetic acid aqueous solution was added to bring the pH of the liquid to 4.1. Then, at the same temperature and with vigorous agitation, 1.44 mL of ethanol was slowly added. The liquid was kept at 0°C for 30 minutes and centrifuged at 16,000 g at 4°C for 20 min. The pellet was suspended in 2 mL of an aqueous buffer consisting of 20 mM Tris-HCl and 10 mMMgCl₂ adjusted to pH 7.5. A sample was taken and analyzed by SDS PAGE: sample in Lane 2, Figure 11. Impurities in this sample represented about 24% of the sample weight. The diluted sample was filtered through a Vivaspin 2 (Sartorius) 100 kDa membrane from which 200µL of retentate was obtained. The retentate was then diluted to 2 mL with the same buffer and filtered through the same 100 kDa membrane. Dilution and ultrafiltration of the retentate (200µL) was repeated four more times. A sample of the retentate was then taken and analyzed by SDS PAGE: sample in Lane 3, Figure 11. Impurities in this sample represented about 9.7% of the sample weight. In conclusion, the product obtained contained MS2 VLPs with purity higher than 90%.

### Example K: Use of Proteinase K (PK) and simple precipitation with ethanol for purification of MS2 VLPs.

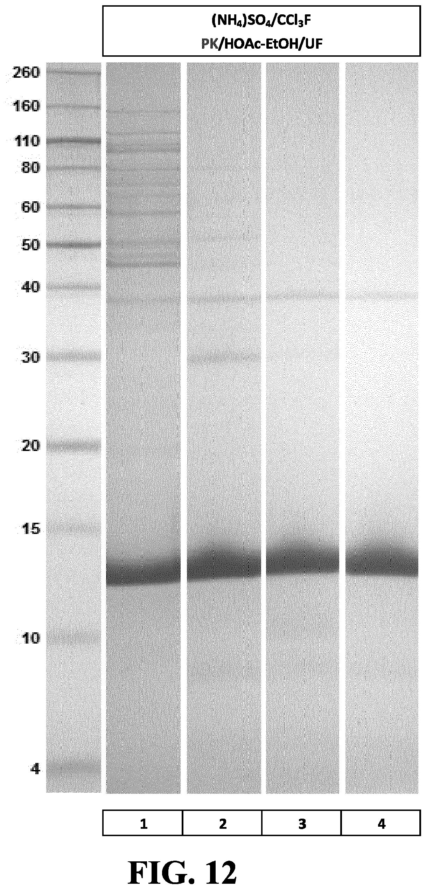

Purification of MS2 VLPs was conducted as follows. Samples were taken during purification and SDS PAGE analysis was run on the samples. Results obtained are shown in Figure 12. One sixth of the pellet obtained from an experiment identical to Example H was resuspended in 20 mM Tris-HCl, pH 7.5, containing 10 mM MgCl₂ and sonicated to lyse cells. Cell debris was removed by centrifugation at 16,000 g. The cell lysate obtained was partially purified by precipitation using ammonium sulfate and extraction using trichlorofluoromethane (Freon 11) as described by Strauss & Sinsheimer (1963) J. Mol. Biol 7:43-54. A sample was taken and analyzed: sample in Lane 1, Figure 12. A strong band at about 14 kDa was found, consistent with the coat protein of MS2 phage. Other bands - impurities - mostly of higher molecular weight represent about 26% of the sample weight. To the partially purified MS2 VLP solution (1.35 mL) 0.6 mg of Proteinase K dissolved in 1.36 mL of 20 mM CaCl₂ aqueous solution was added. The mixture was incubated at 37°C and after 2.5 hours placed in an ice-water bath. A sample was taken and analyzed by SDS PAGE: sample in Lane 2, Figure 12. Impurities in this sample represented about 14% of the sample weight. Fifteen minutes afterwards, about 50 µL of 10% acetic acid aqueous solution was added in an ice/water bath to bring the pH of the liquid to 4.1. Then, at the same temperature and with vigorous agitation, 1.54 mL of ethanol was slowly added. The liquid was kept at 0°C for 30 minutes and centrifuged at 16,000 g at 4°C for 20 min. The pellet was suspended in 2 mL of an aqueous buffer consisting of 20 mM Tris-HCl and 10 mM MgCl₂ adjusted to pH 7.5. A sample was taken and analyzed by SDS PAGE: sample in Lane 3, Figure 12. Impurities in this sample represented about 10% of the sample weight. The diluted sample was filtered through a Vivaspin 2 (Sartorius) 100 kDa membrane from which 200µL of retentate was obtained. The retentate was then diluted to 2 mL with the same buffer and filtered through the same 100 kDa membrane. Dilution and ultrafiltration of the retentate (200µL) was repeated four more times. A sample of the retentate was then taken and analyzed by SDS PAGE: sample in Lane 4, Figure 12. Impurities in this sample represented about 5.1% of the sample weight. In conclusion, the product obtained contained MS2 VLPs with purity of about 95%.

### Example L: Use of constitutive hydrolases (CH), fractional precipitation with ethanol, and ultrafiltration for purification of MS2 VLPs.

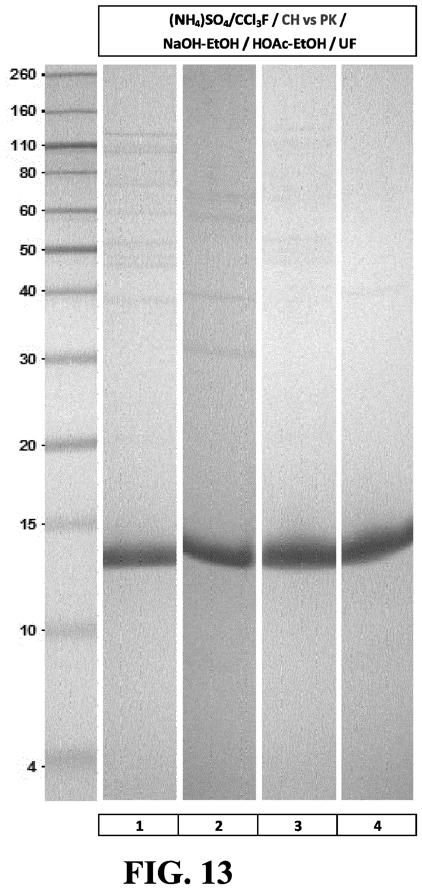

Purification of MS2 VLPs was conducted as follows. Samples were taken during purification and SDS PAGE analysis was run on the samples. Results obtained are shown in Figure 13. One sixth of the pellet obtained from an experiment identical to Example H was resuspended in 20 mM Tris-HCl, pH 7.5, containing 10 mM MgCl₂ and sonicated to lyse cells. Cell debris was removed by centrifugation at 16,000 g. The cell lysate obtained was partially purified by precipitation using ammonium sulfate and extraction using trichlorofluoromethane (Freon 11) as described by Strauss & Sinsheimer (1963) J. Mol. Biol 7:43-54. To the partially purified MS2 VLP solution (1.35 mL) 1.36 mL of 20 mM CaCl₂ aqueous solution was added. The mixture was incubated at 37°C during 2.5 hours (to allow constitutive hydrolases to act) and afterwards was placed in an ice-water bath. A sample was taken and analyzed by SDS PAGE: sample in Lane 1, Figure 13. Impurities in this sample represented about 12% of the sample weight. Fifteen minutes afterwards, about 120 µL of 1% sodium hydroxide aqueous solution was added in an ice/water bath to bring the pH of the liquid to 7.86. Then, at the same temperature and with vigorous agitation, 0.81 mL of ethanol was slowly added. The liquid was kept at 0°C for 30 minutes and centrifuged at 16,000 g at 4°C for 20 min. About 100 µL of 10% acetic acid aqueous solution was slowly added to the supernatant with vigorous agitation in an ice/water bath to bring the pH of the liquid to 4.01. Then, at the same temperature and with vigorous agitation, 1.3 mL of ethanol was slowly added. The liquid was kept at 0°C for 30 minutes and centrifuged at 16,000 g at 4°C for 20 min. The pellet was suspended in 2 mL of an aqueous buffer consisting of 20 mM Tris-HCl and 10 mM MgCl₂ adjusted to pH 7.5. The diluted sample was filtered through a Vivaspin 2 (Sartorius) 100 kDa membrane from which 200µL of retentate was obtained. The retentate was then diluted to 2 mL with the same buffer and filtered through the same 100 kDa membrane. Dilution and ultrafiltration of the retentate (200µL) was repeated four more times. A sample of the retentate was then taken and analyzed by SDS PAGE: sample in Lane 3, Figure 13. Impurities in this sample represented about 4.7% of the sample weight. In conclusion, the product obtained contained MS2 VLPs with purity higher than about 95%.

### Example M: Use of Proteinase K (PK), fractional precipitation with ethanol, and ultrafiltration for purification of MS2 VLPs.

Purification of MS2 VLPs was conducted as follows. Samples were taken during purification and SDS PAGE analysis was run on the samples. Results obtained are shown in Figure 13. One sixth of the pellet obtained from an experiment identical to Example H was resuspended in 20 mM Tris-HCl, pH 7.5, containing 10 mM MgCl₂ and sonicated to lyse cells. Cell debris was removed by centrifugation at 16,000 g. The cell lysate obtained was partially purified by precipitation using ammonium sulfate and extraction using trichlorofluoromethane (Freon 11) as described by Strauss & Sinsheimer (1963) J. Mol. Biol 7:43-54. To the partially purified MS2 VLP solution (1.35 mL) 0.3mg of Proteinase K dissolved in 1.36 mL of 20 mM CaCl₂ aqueous solution was added. The mixture was incubated at 37°C during 2.5 hours and afterwards was placed in an ice-water bath. A sample was taken and analyzed by SDS PAGE: sample in Lane 2, Figure 13. Impurities in this sample represented about 8.1% of the sample weight. Fifteen minutes afterwards, about 120 µL of 1% sodium hydroxide aqueous solution was added in an ice/water bath to bring the pH of the liquid to 7.86. Then, at the same temperature and with vigorous agitation, 0.81 mL of ethanol was slowly added. The liquid was kept at 0°C for 30 minutes and centrifuged at 16,000 g at 4°C for 20 min. About 100 µL of 10% acetic acid aqueous solution was added to the supernatant in an ice/water bath to bring the pH of the liquid to 4.01. Then, at the same temperature and with vigorous agitation, 1.3 mL of ethanol was slowly added. The liquid was kept at 0°C for 30 minutes and centrifuged at 16,000 g at 4°C for 20 min. The pellet was suspended in 2 mL of an aqueous buffer consisting of 20 mM Tris-HCl and 10 mM MgCl₂ adjusted to pH 7.5. The diluted sample was filtered through a Vivaspin 2 (Sartorius) 100 kDa membrane from which 200µL of retentate was obtained. The retentate was then diluted to 2 mL with the same buffer and filtered through the same 100 kDa membrane. Dilution and ultrafiltration of the retentate (200µL) was repeated four more times. A sample of the retentate was then taken and analyzed by SDS PAGE: sample in Lane 4, Figure 13. Impurities in this sample represented about 0.9% of the sample weight. In conclusion, the product obtained contained MS2 VLPs with purity higher than about 99%.

### Example N: Use of various hydrolases, and factional precipitation with ammonium sulfate for purification of MS2 VLPs.

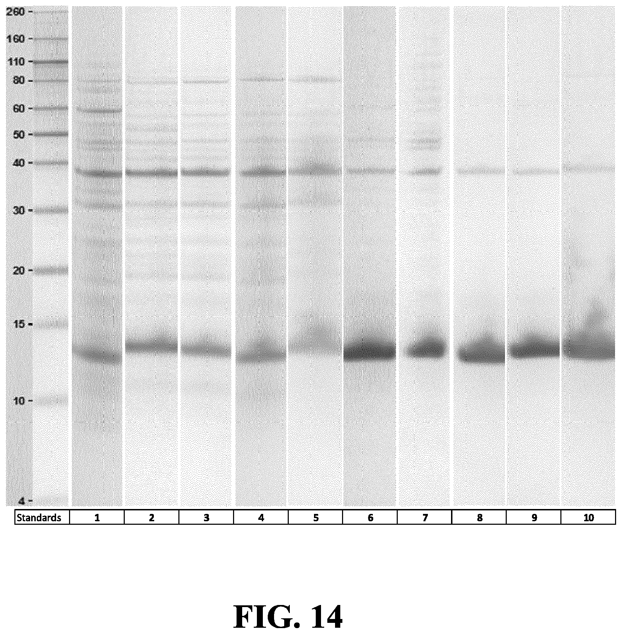

Purification of MS2 VLPs was conducted as follows. Samples were taken during purification and SDS PAGE analysis was run on the samples. Results obtained are shown in Figure 14. One sixth of the pellet obtained from an experiment identical to Example H was resuspended in 20 mM Tris-HCl, pH 7.5, containing 10 mM MgCl2 and sonicated to lyse cells. Cell debris was removed by centrifugation at 16,000 g. A sample of the supernatant was taken and analyzed by SDS PAGE: sample in Lane 1, Figure 14. Impurities in this sample represented about 70% of the sample weight. Four other identical fractions of the pellet obtained from such experiment identical to Example H were processed in the same manner.

The five centrifuged cell lysates obtained, each 3.7 mL in volume, were further processed in five different manners, as follows. The first centrifuged cell lysate was placed in an ice-water bath for 15 minutes and 0.1 grams of ammonium sulfate was added. The mixture was vortexed until complete dissolution of ammonium sulfate was achieved. The liquid was kept at 0°C for 2 hours and centrifuged at 16,000 g at 4°C for 30 min. 0.4 grams of ammonium sulfate was added to the supernatant and vortexed until complete dissolution of ammonium sulfate was achieved. The liquid was kept at 0°C for 2 hours and centrifuged at 16,000 g at 4°C for 30 min. The purified MS2 VLPs pellet was suspended in 0.2 mL of an aqueous buffer consisting of 20 mM Tris-HCl and 10 mM MgCl2 adjusted to pH 7.5. The second centrifuged cell lysate was incubated at 37°C for five hours, placed in an ice-water bath for the same amount of time as the first centrifuged cell lysate and subsequently processed in identical manner as the first centrifuged cell lysate. 0.15 mg of Proteinase K (Sigma Aldrich, St. Louis, MO) was added to the third centrifuged cell lysate. It was incubated then at 37°C for five hours, placed in an ice-water bath for the same amount of time as the first centrifuged cell lysate and subsequently processed in identical manner as the first centrifuged cell lysate. The fourth centrifuged cell lysate was incubated at 37°C for two hours. 0.15 mg of PK was then added. It was incubated at 37°C for an additional three hours, placed in an ice-water bath for the same amount of time as the first centrifuged cell lysate and subsequently processed in identical manner as the first centrifuged cell lysate.

500 units of Benzonase^{®} Nuclease(Sigma Aldrich, St. Louis, MO) and 35 units of Lipase from *Candida rugosa* (Sigma Aldrich, St. Louis, MO) was added to the fifth centrifuged cell lysate and incubated at 37°C for one hour. 15 units of α-Amylase from *Bacillus* sp*.* (Sigma Aldrich, St. Louis, MO) was then added and incubated at 37°C for one additional hour. 0.15 mg of PK was then added. The mixture was incubated at 37°C for an additional three hours, placed in an ice-water bath for the same amount of time as the first centrifuged cell lysate and subsequently processed in identical manner as the first centrifuged cell lysate.

A sample was taken of the second centrifuged cell lysate after its 5 hours incubation and analyzed by SDS PAGE: sample in Lane 2, Figure 14.A sample was taken of the third centrifuged cell lysate after its 5 hours incubation and analyzed by SDS PAGE: sample in Lane 3, Figure 14. A sample was taken of the fourth centrifuged cell lysate after its 5 hours incubation and analyzed by SDS PAGE: sample in Lane 4, Figure 14.A sample was taken of the fifth centrifuged cell lysate after its 5 hours incubation and analyzed by SDS PAGE: sample in Lane 5, Figure 14.

A sample was taken of the purified MS2 VLPs suspension for the first centrifuged cell lysate and analyzed by SDS PAGE: sample in Lane 6, Figure 14. The product obtained contained MS2 VLPs with purity of about 88%.Protein concentration (Pierce^{®} BCA Protein Assay Kit, Thermo Fisher Scientific, Rockford, IL) of this sample was 18.5 mg/mL. Optical density measured in a 1 cm cell at 260 nm (OD-260nm) of a 200:1 dilution of this sample was 0.553 and OD-280nm was 0.303. These measurements are consistent with RNA yield of about 9 mg per liter of culture.

A sample was taken of the purified MS2 VLPs suspension for the second centrifuged cell lysate and analyzed by SDS PAGE: sample in Lane 7, Figure 14. The product obtained contained MS2 VLPs with purity of about 75%. Protein concentration of this sample was 25.4 mg/mL. Optical density measured in a 1 cm cell at 260 nm (OD-260nm) of a 200:1 dilution of this sample was 0.784 and OD-280nm was 0.453. These measurements are consistent with RNA yield of about 11 mg per liter of culture.

A sample was taken of the purified MS2 VLPs suspension for the third centrifuged cell lysate and analyzed by SDS PAGE: sample in Lane 8, Figure 14. The product obtained contained MS2 VLPs with purity of about 94.3%. Protein concentration of this sample was 21.0 mg/mL. Optical density measured in a 1 cm cell at 260 nm (OD-260nm) of a 200:1 dilution of this sample was 0.632 and OD-280nm was 0.321. These measurements are consistent with RNA yield of about 10 mg per liter of culture.

A sample was taken of the purified MS2 VLPs suspension for the fourth centrifuged cell lysate and analyzed by SDS PAGE: sample in Lane 9, Figure 14. The product obtained contained MS2 VLPs with purity of about 95.6%. Protein concentration of this sample was 19.4 mg/mL. Optical density measured in a 1 cm cell at 260 nm (OD-260nm) of a 200:1 dilution of this sample was 0.666 and OD-280nm was 0.353. These measurements are consistent with RNA yield of about 11 mg per liter of culture.

A sample was taken of the purified MS2 VLPs suspension for the fifth centrifuged cell lysate and analyzed by SDS PAGE: sample in Lane 10, Figure 14. The product obtained contained MS2 VLPs with purity of about 96%.Protein concentration of this sample was 19.8 mg/mL. Optical density measured in a 1 cm cell at 260 nm (OD-260nm) of a 200:1 dilution of this sample was 0.661 and OD-280nm was 0.354. These measurements are consistent with RNA yield of about 11 mg per liter of culture.

### Example O: Production of MS2 Capsids encapsidating shRNA targeting Green Fluorescent Protein (GFP) and HDV ribozyme attached to MS2 19-mer RNA hairpin.

Production of MS2 capsids is conducted as follows. The following DNA sequence Sequence A (SEQ ID NO: 7), encoding MS2's coat protein is cloned into pDEST14 (Life Technologies) plasmid:

ACAAGTTTGTACAAAAAAGCAGGCTAAGAAGGAGATATACATATGGCTTCTAACTT
TACTCAGTTCGTTCTCGTCGACAATGGCGGAACTGGCGACGTGACTGTCGCCCCAAG
CAACTTCGCTAACGGGGTCGCTGAATGGATCAGCTCTAACTCGCGTTCACAGGCTTA
CAAAGTAACCTGTAGCGTTCGTCAGAGCTCTGCGCAGAATCGCAAATACACCATCA
AAGTCGAGGTGCCTAAAGTGGCAACCCAGACTGTTGGTGGTGTAGAGCTTCCTGTA
GCCGCATGGCGTTCGTACTTAAATATGGAACTAACCATTCCAATTTTCGCTACGAAT
TCCGACTGCGAGCTTATTGTTAAGGCAATGCAAGGTCTCCTAAAAGATGGAAACCC
GATTCCCTCAGCAATCGCAGCAAACTCCGGCATCTACTAATAG Sequence A (SEQ ID
NO: 7)

The following DNA sequence, Sequence B (SEQ ID NO: 8) was cloned into plasmid pACYC184. A transcription terminator was also cloned at the 3' end of Sequence B (SEQ ID NO: 8)(not shown). Sequence B (SEQ ID NO: 8) encodes, shRNA hairpin, Hepatitis Delta Virus (HDV) ribozyme designed to cleave the 3' end of the siRNA hairpin, and MS2's specific RNA 19-mer, was cloned into plasmid pACYC184:

### Sequence T7-Rz6

GGATCCTAATACGACTCACTATAGGCAAGCTGACCCTGAAGTTCTCAAGAGGAACTT
CAGGGTCAGCTTGCCAAGGCCGGCATGGTCCCAGCCTCCTCGCTGGCGCCGGCTGG
GCAACATTCGTGGCGAATGGGACCACGCTTCAAACATGAGGATTACCCATGTCGAA
GCGACCATGG (SEQ ID NO: 8).

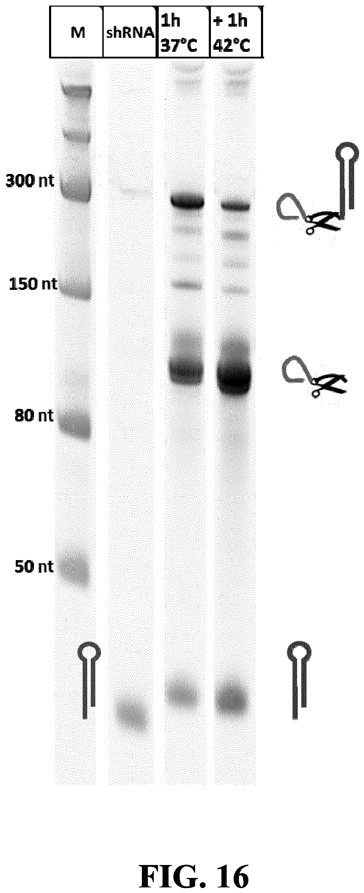

One Shot BL21(DE3) Chemically Competent E. coli (Life Technologies) cells were transformed with the 2 plasmids one containing Sequence A (SEQ ID NO: 104) and Sequence B (SEQ ID NO: 105) selecting for chloramphenicol and ampicillin resistant transformants. For capsid production these transformants were grown at 37o in 32 mL LB medium containing both ampicillin and chloramphenicol. When the culture density reached OD (600 nm)=0.8, isopropyl β-D-1-thiogalactopyranoside (Sigma-Aldrich) was then added to a final concentration of 1 mM. Cells were harvested 4 hours post-induction by centrifugation at 3,000 g and 4°C for 40 min. RNA was extracted from purified VLPs as described in example Z, and analyzed as described in Example AA. A band of the same molecular weight as expected for the encoded shRNA, as observed in lane shRNA in Figure 16, was observed.

### Example P: Production of MS2 Capsids using a transcript coding for siGFP flanked on its 5' end by a long Hammerhead ribozyme, and its 3' end by an HDV ribozyme attached toMS2 19-mer RNA hairpin and a second HDV ribozyme.

Production of MS2 capsids is conducted as follows. The following DNA sequence Sequence A (SEQ ID NO: 7), encoding MS2's coat protein is cloned into pDEST14 (Life Technologies) plasmid:

ACAAGTTTGTACAAAAAAGCAGGCTAAGAAGGAGATATACATATGGCTTCTAACTT
TACTCAGTTCGTTCTCGTCGACAATGGCGGAACTGGCGACGTGACTGTCGCCCCAAG
CAACTTCGCTAACGGGGTCGCTGAATGGATCAGCTCTAACTCGCGTTCACAGGCTTA
CAAAGTAACCTGTAGCGTTCGTCAGAGCTCTGCGCAGAATCGCAAATACACCATCA
AAGTCGAGGTGCCTAAAGTGGCAACCCAGACTGTTGGTGGTGTAGAGCTTCCTGTA
GCCGCATGGCGTTCGTACTTAAATATGGAACTAACCATTCCAATTTTCGCTACGAAT
TCCGACTGCGAGCTTATTGTTAAGGCAATGCAAGGTCTCCTAAAAGATGGAAACCC

GATTCCCTCAGCAATCGCAGCAAACTCCGGCATCTACTAATAG Sequence A (SEQ ID
NO: 7)

The following DNA sequence, Sequence C (SEQ ID NO: 9) is cloned into plasmid pACYC184. A transcription terminator is also cloned at the 3' end of Sequence C (SEQ ID NO: 9)(not shown). Sequence C (SEQ ID NO: 9) encodes T7 promoter, Hammerhead ribozyme designed to cleave the 5' end of a siRNA hairpin, siRNA hairpin, Hepatitis Delta Virus (HDV) ribozyme designed to cleave the 3' end of the siRNA hairpin, and MS2's specific RNA 19-mer, and another HDV ribozyme is cloned into plasmid pACYC184:

### Sequence T7-Rz12

GGATCCTAATACGACTCACTATAGGGAGATAAATAAATAAATTTGAATGAACTTCA
GGGTCAGCTTGCTGATGAGGCGCTTCGGCGCCGAAACACCCAGTGGTGTCCAAGCT
GACCCTGAAGTTCATTCAAGAGATGAACTTCAGGGTCAGCTTGTCGGCCGGCATGGT
CCCAGCCTCCTCGCTGGCGCCGGCTGGGCAACATTCGTGGCGAATGGGACCACGCTT
CAAACATGAGGATTACCCATGTCGAAGCGAATTTATTTATTTAATTATTATTATTATT
ATTGGCCGGCATGGTCCCAGCCTCCTCGCTGGCGCCGGCTGGGCAACACCTTCGGGT
GGCGAATGGGACCAAAAAAAAATAATAATAATAATCCATGG (SEQ ID NO: 9)

One Shot BL21 (DE3) Chemically Competent E. coli (Life Technologies) cells are transformed with the 2 plasmids one containing Sequence A (SEQ ID NO: 104) and Sequence C (SEQ ID NO: 106) selecting for chloramphenicol and ampicillin resistant transformants. For capsid production these transformants are grown at 37o in 32 mL LB medium containing both ampicillin and chloramphenicol. When the culture density reaches OD (600 nm)=0.8, isopropyl β-D-1-thiogalactopyranoside (Sigma-Aldrich) is then added to a final concentration of 1 mM. Cells are harvested 4 hours post-induction by centrifugation at 3,000 g and 4°C for 40 min..

### Example Q: Production of MS2 Capsids using transcripts coding for shRNA and slow HDV ribozymes attached to MS2 19-mer RNA hairpin.

Several experiments producing MS2 capsids, each encapsidating different cargoes are conducted as described in Example O. However, instead of the HDV ribozyme sequence included in Sequence B (SEQ ID NO: 8), a mutant cleaving the 3' end of the siRNA with a slower reaction rate constant is used in each experiment. The following sequences, containing slower HDV ribozymes, are used instead of Sequence B, for each experiment:
Sequence G76U: 
TAATACGACTCACTATAGCAAGCTGACCCTGAAGTTCATCAAGAGTGAACTTCAGG
GTCAGCTTGTCGGCCGGCATGGTCCCAGCCTCCTCGCTGGCGCCGGCTGGGCAACAT
TCGTGGCTAATGGGACCATATATATATACATGAGGATTACCCATGTCCATGG (SEQ
ID NO: 10)
Sequence G40U: 
TAATACGACTCACTATAGCAAGCTGACCCTGAAGTTCATCAAGAGTGAACTTCAGG
GTCAGCTTGTCGGCCGGCATGGTCCCAGCCTCCTCGCTGGCGCCGGCTGGTCAACAT
TCGTGGCGAATGGGACCATATATATATACATGAGGATTACCCATGTCCATGG (SEQ
ID NO: 11)
Sequence A16G: 
TAATACGACTCACTATAGCAAGCTGACCCTGAAGTTCATCAAGAGTGAACTTCAGG
GTCAGCTTGTCGGCCGGCATGGTCCCGGCCTCCTCGCTGGCGCCGGCTGGGCAACAT
TCGTGGCGAATGGGACCATATATATATACATGAGGATTACCCATGTCCATGG (SEQ
ID NO: 12)
Sequence G39U: 
TAATACGACTCACTATAGCAAGCTGACCCTGAAGTTCATCAAGAGTGAACTTCAGG
GTCAGCTTGTCGGCCGGCATGGTCCCAGCCTCCTCGCTGGCGCCGGCTGTGCAACAT
 
TCGTGGCGAATGGGACCATATATATATACATGAGGATTACCCATGTCCATGG (SEQ
ID NO: 13)
Sequence A78G: 
TAATACGACTCACTATAGCAAGCTGACCCTGAAGTTCATCAAGAGTGAACTTCAGG
GTCAGCTTGTCGGCCGGCATGGTCCCAGCCTCCTCGCTGGCGCCGGCTGGGCAACAT
TCGTGGCGAGTGGGACCATATATATATACATGAGGATTACCCATGTCCATGG (SEQ
ID NO: 14)
Sequence C21U: 
TAATACGACTCACTATAGCAAGCTGACCCTGAAGTTCATCAAGAGTGAACTTCAGG
GTCAGCTTGTCGGCCGGCATGGTCCCAGCCTTCTCGCTGGCGCCGGCTGGGCAACAT
TCGTGGCGAATGGGACCATATATATATACATGAGGATTACCCATGTCCATGG (SEQ
ID NO: 15)
Sequence G25A: 
TAATACGACTCACTATAGCAAGCTGACCCTGAAGTTCATCAAGAGTGAACTTCAGG
GTCAGCTTGTCGGCCGGCATGGTCCCAGCCTCCTCACTGGCGCCGGCTGGGCAACAT
TCGTGGCGAATGGGACCATATATATATACATGAGGATTACCCATGTCCATGG (SEQ
ID NO: 16)
Sequence A78U: 
TAATACGACTCACTATAGCAAGCTGACCCTGAAGTTCATCAAGAGTGAACTTCAGG
GTCAGCTTGTCGGCCGGCATGGTCCCAGCCTCCTCGCTGGCGCCGGCTGGGCAACAT
TCGTGGCGATTGGGACCATATATATATACATGAGGATTACCCATGTCCATGG (SEQ
ID NO: 17)
Sequence G74C:

TAATACGACTCACTATAGCAAGCTGACCCTGAAGTTCATCAAGAGTGAACTTCAGG
GTCAGCTTGTCGGCCGGCATGGTCCCAGCCTCCTCGCTGGCGCCGGCTGGGCAACAT

TCGTGCCGAATGGGACCATATATATATACATGAGGATTACCCATGTCCATGG (SEQ
ID NO: 18)

### Example R: Production of MS2 Capsids using a transcript coding for shGFP flanked on its 5' end by one long Hammerhead ribozyme, and its 3' end by another long Hammerhead ribozyme attached to MS2 19-mer RNA hairpin and an HDV ribozyme.

Production of MS2 capsids is conducted as follows. The following DNA sequence, Sequence A (SEQ ID NO: 7), encoding MS2's coat protein is cloned into pDEST14 (Life Technologies) plasmid:

ACAAGTTTGTACAAAAAAGCAGGCTAAGAAGGAGATATACATATGGCTTCTAACTT
TACTCAGTTCGTTCTCGTCGACAATGGCGGAACTGGCGACGTGACTGTCGCCCCAAG
CAACTTCGCTAACGGGGTCGCTGAATGGATCAGCTCTAACTCGCGTTCACAGGCTTA
CAAAGTAACCTGTAGCGTTCGTCAGAGCTCTGCGCAGAATCGCAAATACACCATCA
AAGTCGAGGTGCCTAAAGTGGCAACCCAGACTGTTGGTGGTGTAGAGCTTCCTGTA
GCCGCATGGCGTTCGTACTTAAATATGGAACTAACCATTCCAATTTTCGCTACGAAT
TCCGACTGCGAGCTTATTGTTAAGGCAATGCAAGGTCTCCTAAAAGATGGAAACCC
GATTCCCTCAGCAATCGCAGCAAACTCCGGCATCTACTAATAG

The following DNA sequence, Sequence D (SEQ ID NO: 19) is cloned into plasmid pACYC184. A transcription terminator is also cloned at the 3' end of Sequence D (SEQ ID NO: 19)(not shown). Sequence D (SEQ ID NO: 19) encodes T7 promoter, Hammerhead ribozyme designed to cleave the 5' end of a siRNA hairpin, siRNA hairpin, Hammerhead ribozyme designed to cleave the 3' end of the siRNA hairpin, MS2's specific RNA 19-mer and an HDV ribozyme:

### Sequence T7-Rz15:

GGATCCTAATACGACTCACTATAGGGAGACGTTCACGTTGAATGAACTTCAGGGTCA
GCTTGCTGATGAGGCGCTTCGGCGCCGAAACACCCAGTGGTGTCCAAGCTGACCCTG
AAGTTCATTCAAGAGATGAACTTCAGGGTCAGCTTGTCACCGGATGTGCTCTCCGGT
CTGATGAGTCCGTGAGGACGAAACAAGCTGACCCTGAAGTTCATCCGTGAACGACG
CTTCAAACATGAGGATTACCCATGTCGAAGCGAATATATATATATAGGCCGGCATG

GTCCCAGCCTCCTCGCTGGCGCCGGCTGGGCAACACCTTCGGGTGGCGAATGGGAC
CAAAAAAATATATATATATACCATGG (SEQ ID NO: 19)

One Shot BL21(DE3) Chemically Competent E. coli (Life Technologies) cells are transformed with the 2 plasmids one containing Sequence A (SEQ ID NO: 7) and the Sequence D (SEQ ID NO: 19) selecting for chloramphenicol and ampicillin resistant transformants. For capsid production these transformants are grown at 37° in 750 mL LB medium containing both ampicillin and chloramphenicol. When the culture density reaches OD (600 nm)=0.8, isopropyl β-D-1-thiogalactopyranoside (Sigma-Aldrich) is then added to a final concentration of 1 mM. Cells are harvested 4 hours post-induction by centrifugation at 3,000 g and 4°C for 40 min. A sample is taken prior to induction and at the time of harvest for analysis.

### Example S: Production of MS2 Capsids using transcripts coding for shRNA and long Hammerhead ribozymes attached to MS2 19-mer RNA hairpin

Several experiments producing MS2 capsids, each encapsidating different cargoes, are conducted as described in Example O. However, instead of the HDV ribozyme sequence included in Sequence B (SEQ ID NO: 8), a long Hammerhead ribozyme sequence hybridizing the 3' end of the siRNA, and designed to cleave it is used in each experiment. The following sequences, containing long Hammerhead ribozymes are used instead of Sequence B (SEQ ID NO: 8), for each experiment:
Sequence 10MNT (doesn't cut well): 
TAATACGACTCACTATAGCAAGCTGACCCTGAAGTTCATCAAGAGTGAACTTCAGG
GTCAGCTTGTCACCGGATGTGCTCTCCGGTCTGATGAGTCCGTGAGGACGAAACAAG
CTGACCATATATATATACATGAGGATTACCCATGTCCATGG (SEQ ID NO: 20)
Sequence 15MNT (doesn't cut well): 
TAATACGACTCACTATAGCAAGCTGACCCTGAAGTTCATCAAGAGTGAACTTCAGG
GTCAGCTTGTCACCGGATGTGCTCTCCGGTCTGATGAGTCCGTGAGGACGAAACAAG
CTGACCCTGAAATATATATATACATGAGGATTACCCATGTCCATGG (SEQ ID NO: 21)
Sequence 20MNT (cuts well): 
TAATACGACTCACTATAGCAAGCTGACCCTGAAGTTCATCAAGAGTGAACTTCAGG
GTCAGCTTGTCACCGGATGTGCTCTCCGGTCTGATGAGTCCGTGAGGACGAAACAAG
CTGACCCTGAAGTTCAATATATATATACATGAGGATTACCCATGTCCATGG (SEQ ID
NO: 22)
Sequence 22MNT (cuts well): 
TAATACGACTCACTATAGCAAGCTGACCCTGAAGTTCATCAAGAGTGAACTTCAGG
GTCAGCTTGTCACCGGATGTGCTCTCCGGTCTGATGAGTCCGTGAGGACGAAACAAG
CTGACCCTGAAGTTCACTATATATATATACATGAGGATTACCCATGTCCATGG (SEQ
ID NO: 23)
Sequence 25MNT (cuts well): 
TAATACGACTCACTATAGCAAGCTGACCCTGAAGTTCATCAAGAGTGAACTTCAGG
GTCAGCTTGTCACCGGATGTGCTCTCCGGTCTGATGAGTCCGTGAGGACGAAACAAG
CTGACCCTGAAGTTCACTCTTATATATATATACATGAGGATTACCCATGTCCATGG
(SEQ ID NO: 24)
Sequence 27MNT (cuts well):

TAATACGACTCACTATAGCAAGCTGACCCTGAAGTTCATCAAGAGTGAACTTCAGG
GTCAGCTTGTCACCGGATGTGCTCTCCGGTCTGATGAGTCCGTGAGGACGAAACAAG
CTGACCCTGAAGTTCACTCTTGAATATATATATACATGAGGATTACCCATGTCCATG
G (SEQ ID NO: 25)

Experimental data set forth in the Examples supports the conclusion that RNA strands which cut well according to the present disclosure are those which include a Hammerhead ribozyme sequence for which proper folding of the ribozyme sequence is thermodynamically favored over the folding of the shRNA ("hairpin") section of the whole strand. Thermodynamic parameters for each of the above sequences were calculated to determine which sequences cut well and which do not.

### Example T: Production of MS2 Capsids using a transcript coding for siRNA flanked by a long Hammerhead ribozyme at its 5' end, and trans-acting HDV ribozyme at its 3' end attached to MS2 19-mer RNA hairpin.

Production of MS2 capsids is conducted as follows. The following DNA sequence (Sequence A; SEQ ID NO: 7), encoding MS2's coat protein is cloned into pDEST14 (Life Technologies) plasmid:

ACAAGTTTGTACAAAAAAGCAGGCTAAGAAGGAGATATACATATGGCTTCTAACTT
TACTCAGTTCGTTCTCGTCGACAATGGCGGAACTGGCGACGTGACTGTCGCCCCAAG
CAACTTCGCTAACGGGGTCGCTGAATGGATCAGCTCTAACTCGCGTTCACAGGCTTA
CAAAGTAACCTGTAGCGTTCGTCAGAGCTCTGCGCAGAATCGCAAATACACCATCA
AAGTCGAGGTGCCTAAAGTGGCAACCCAGACTGTTGGTGGTGTAGAGCTTCCTGTA
GCCGCATGGCGTTCGTACTTAAATATGGAACTAACCATTCCAATTTTCGCTACGAAT
TCCGACTGCGAGCTTATTGTTAAGGCAATGCAAGGTCTCCTAAAAGATGGAAACCC
GATTCCCTCAGCAATCGCAGCAAACTCCGGCATCTACTAATAG (SEQ ID NO: 7)

The following DNA sequence, Sequence E (SEQ ID NO: 26) is cloned into plasmid pACYC184. A transcription terminator was also cloned at the 3' end of Sequence E (SEQ ID NO: 26)(not shown). Sequence E (SEQ ID NO: 26) encodes BamHI restriction site, T7 promoter and start sequence, HDV ribozyme designed to cleave in trans mode the 3' end of the siRNA against Enhanced Green Fluorescent Protein (siEGPF), ATAT spacer, long Hammerhead ribozyme designed to cleave the 5' end of the siEGFP hairpin, siEGFP hairpin, complementary sequence to the HDV ribozyme cleaving in trans mode the 3' end of the siEGFP hairpin, MS2's specific RNA 19-mer, and PacI restriction site.:

GGATCCTAATACGACTCACTATAGGGATCTCCCAGCCTCCTCGCTGGCGCCGGCTGG
GCAACATTCGTGGCGAATGGGGATCATATCTTGATGAACTTCAGGGTCAGCTTGCT
GATGAGGCGCTTCGGCGCCGAAACACCGTGTCCAAGCTGACCCTGAAGTTCATCAA
GAATGAACTTCAGGGTCAGCTTGTCGGCCGGCATGCATTCAAACATGAGGATTACCC
ATGTCGAAGTTAATTAA (SEQ ID NO: 26)

BamHI and HindIII site are added at the 5' and 3' end respectively to facilitate cloning into pACYC184 (not shown).

One Shot BL21(DE3) Chemically Competent E. coli (Life Technologies) cells are transformed with the 2 plasmids one containing Sequence A (SEQ ID NO: 104) and Sequence E (SEQ ID NO: 26) selecting for chloramphenicol and ampicillin resistant transformants. For capsid production these transformants are grown at 37° in 750 mL LB medium containing both ampicillin and chloramphenicol. When the culture density reaches OD (600 nm)=0.8, isopropyl β-D-1-thiogalactopyranoside (Sigma-Aldrich) is then added to a final concentration of 1 mM. Cells are harvested 4 hours post-induction by centrifugation at 3,000 g and 4°C for 40 min. A sample is taken prior to induction and at the time of harvest for analysis.

### Example U: Production of MS2 Capsids using a transcript coding for 3 different siRNAs targeting GFP flanked by long Hammerhead ribozymes attached to MS2 19-mer RNA hairpin.

Production of MS2 capsids is conducted as follows. The following DNA sequence (Sequence A; SEQ ID NO: 7), encoding MS2's coat protein is cloned into pDEST14 (Life Technologies) plasmid:

ACAAGTTTGTACAAAAAAGCAGGCTAAGAAGGAGATATACATATGGCTTCTAACTT
TACTCAGTTCGTTCTCGTCGACAATGGCGGAACTGGCGACGTGACTGTCGCCCCAAG
CAACTTCGCTAACGGGGTCGCTGAATGGATCAGCTCTAACTCGCGTTCACAGGCTTA
CAAAGTAACCTGTAGCGTTCGTCAGAGCTCTGCGCAGAATCGCAAATACACCATCA
AAGTCGAGGTGCCTAAAGTGGCAACCCAGACTGTTGGTGGTGTAGAGCTTCCTGTA
GCCGCATGGCGTTCGTACTTAAATATGGAACTAACCATTCCAATTTTCGCTACGAAT
TCCGACTGCGAGCTTATTGTTAAGGCAATGCAAGGTCTCCTAAAAGATGGAAACCC
GATTCCCTCAGCAATCGCAGCAAACTCCGGCATCTACTAATAG (SEQ ID NO: 7)

The following DNA sequence, Sequence F (SEQ ID NO: 27) is cloned into plasmid pACYC184. A transcription terminator was also cloned at the 3' end of Sequence F (SEQ ID NO: 27)(not shown). Sequence F (SEQ ID NO: 27) encodes T7 promoter and start sequence, 3 Hammerhead ribozymes designed to cleave the 5' ends of the siRNAs against Enhanced Green Fluorescent Protein (siEGPF), 3 different siEGFP hairpins, 3 long Hammerhead ribozymes designed to cleave the 3' ends of the siEGFP hairpins, MS2's specific RNA 19-mer:

GGATCCTAATACGACTCACTATAGGGAGACTTGATGAACTTCAGGGTCAGCTTGCTG
ATGAGGCGCTTCGGCGCCGAAACACCCAGTGGTGTCCAAGCTGACCCTGAAGTTCA
TCAAGAATGAACTTCAGGGTCAGCTTGTCACCGGATGTGCTCTCCGGTCTGATGAGT
CCGTGAGGACGAAACAAGCTGACCCTGAAGTTCATTATATCTTGGCAGATGAACTTC
AGGGTCAGCTGATGAGACTCTTCGGAGTCGAAACACCCAGTGGTGTCCTGACCCTG
AAGTTCATCTGCCAAGAGCAGATGAACTTCAGGGTCAGTCACCGGATGTGCTCTCCG
GTCTGATGAGTCCGTGAGGACGAAACTGACCCTGAAGTTCATCTGCTATATCTTGTG
GTGCAGATGAACTTCAGGGCTGATGAGGCTCTTCGGAGCCGAAACACCCAGTGGTG
TCCCCTGAAGTTCATCTGCACCACAAGATGGTGCAGATGAACTTCAGGGTCACCGGA
TGTGCTCTCCGGTCTGATGAGTCCGTGAGGACGAAACCCTGAAGTTCATCTGCACCA
TACGCCGGCCATTCAAACATGAGGATTACCCATGTCGAAGTTAATTAA (SEQ ID NO:
27)

One Shot BL21 (DE3) Chemically Competent E. coli (Life Technologies) cells are transformed with the 2 plasmids one containing Sequence A (SEQ ID NO: 7) and the other Sequence F (SEQ ID NO: 27) selecting for chloramphenicol and ampicillin resistant transformants. For capsid production these transformants are grown at 37° in 750 mL LB medium containing both ampicillin and chloramphenicol. When the culture density reaches OD (600 nm)=0.8, isopropyl β-D-1-thiogalactopyranoside (Sigma-Aldrich) is then added to a final concentration of 1 mM. Cells are harvested 4 hours post-induction by centrifugation at 3,000 g and 4°C for 40 min. A sample is taken prior to induction and at the time of harvest for analysis.

### Example V: Production of MS2 Capsids using a transcript coding for 3 different siRNAs targeting GFP flanked by spacers located at their 5' ends and HDV ribozymes at their 3' ends, attached to MS2 19-mer RNA hairpin.

Production of MS2 capsids is conducted as follows. The following DNA sequence (Sequence A; SEQ ID NO: 7), encoding MS2's coat protein is cloned into pDEST14 (Life Technologies) plasmid:

ACAAGTTTGTACAAAAAAGCAGGCTAAGAAGGAGATATACATATGGCTTCTAACTT
TACTCAGTTCGTTCTCGTCGACAATGGCGGAACTGGCGACGTGACTGTCGCCCCAAG
CAACTTCGCTAACGGGGTCGCTGAATGGATCAGCTCTAACTCGCGTTCACAGGCTTA
CAAAGTAACCTGTAGCGTTCGTCAGAGCTCTGCGCAGAATCGCAAATACACCATCA
AAGTCGAGGTGCCTAAAGTGGCAACCCAGACTGTTGGTGGTGTAGAGCTTCCTGTA
GCCGCATGGCGTTCGTACTTAAATATGGAACTAACCATTCCAATTTTCGCTACGAAT
TCCGACTGCGAGCTTATTGTTAAGGCAATGCAAGGTCTCCTAAAAGATGGAAACCC
GATTCCCTCAGCAATCGCAGCAAACTCCGGCATCTACTAATAG (SEQ ID NO 7)

The following DNA sequence, Sequence G (SEQ ID NO: 28) is cloned into plasmid pACYC184. A transcription terminator was also cloned at the 3' end of Sequence G (SEQ ID NO: 28) (not shown). Sequence G (SEQ ID NO: 28) encodes BamHI restriction site, T7 promoter and start sequence, 3 spacers at the 5' ends of the siRNAs against Enhanced Green Fluorescent Protein (siEGPF), 3 different siEGFP hairpins, 3 HDV ribozymes designed to cleave the 3' ends of the siEGFP hairpins, MS2's specific RNA 19-mer, and PacI restriction site.:

GGATCCTAATACGACTCACTATAGGGAGAATATATATACAAGCTGACCCTGAAGTTC
ATCAAGAATGAACTTCAGGGTCAGCTTGTCGGCCGGCATGGTCCCAGCCTCCTCGCT
GGCGCCGGCTGGGCAACATTCGTGGCGAATGGGACCAATTAATTACTGACCCTGAA
GTTCATCTGCCAAGAGCAGATGAACTTCAGGGTCAGTCGGCCGGCATGGTCCCAGC
CTCCTCGCTGGCGCCGGCTGGGCAACATTCGTGGCGAATGGGACCAATAATAATCCC
TGAAGTTCATCTGCACCACAAGATGGTGCAGATGAACTTCAGGGTCGGCCGGCATG
GTCCCAGCCTCCTCGCTGGCGCCGGCTGGGCAACATTCGTGGCGAATGGGACCCATT
CAAACATGAGGATTACCCATGTCGAAGTTAATTAA (SEQ ID NO: 28)

One Shot BL21 (DE3) Chemically Competent E. coli (Life Technologies) cells are transformed with the 2 plasmids one containing Sequence A (SEQ ID NO: 7)and Sequence G (SEQ ID NO: 28) selecting for chloramphenicol and ampicillin resistant transformants. For capsid production these transformants are grown at 37° in 750 mL LB medium containing both ampicillin and chloramphenicol. When the culture density reaches OD (600 nm)=0.8, isopropyl β-D-1-thiogalactopyranoside (Sigma-Aldrich) is then added to a final concentration of 1 mM. Cells are harvested 4 hours post-induction by centrifugation at 3,000 g and 4°C for 40 min. A sample is taken prior to induction and at the time of harvest for analysis.

### Example W: Production of MS2 Capsids using a transcript coding for the two strands of an siRNA targeting GFP each flanked by a long Hammerhead ribozyme located at their 3' ends and HDV ribozymes at their 5' ends, attached to MS2 19-mer RNA hairpin.

Production of MS2 capsids is conducted as follows. The following DNA sequence (Sequence A; SEQ ID NO: 7), encoding MS2's coat protein is cloned into pDEST14 (Life Technologies) plasmid:

ACAAGTTTGTACAAAAAAGCAGGCTAAGAAGGAGATATACATATGGCTTCTAACTT
TACTCAGTTCGTTCTCGTCGACAATGGCGGAACTGGCGACGTGACTGTCGCCCCAAG
CAACTTCGCTAACGGGGTCGCTGAATGGATCAGCTCTAACTCGCGTTCACAGGCTTA
CAAAGTAACCTGTAGCGTTCGTCAGAGCTCTGCGCAGAATCGCAAATACACCATCA
AAGTCGAGGTGCCTAAAGTGGCAACCCAGACTGTTGGTGGTGTAGAGCTTCCTGTA
GCCGCATGGCGTTCGTACTTAAATATGGAACTAACCATTCCAATTTTCGCTACGAAT
TCCGACTGCGAGCTTATTGTTAAGGCAATGCAAGGTCTCCTAAAAGATGGAAACCC
GATTCCCTCAGCAATCGCAGCAAACTCCGGCATCTACTAATAG (SEQ ID NO: 7)

The following DNA sequence, Sequence H (SEQ ID NO: 29) was cloned into plasmid pACYC184. A transcription terminator was also cloned at the 3' end of sequence H (not shown).

### Sequence T7-Rz8

GGATCCTAATACGACTCACTATAGGGAGAATGAACTTCAGGGTCAGCTTGCTGATG
AGGCGCTTCGGCGCCGAAACACCGTGTCCAAGCTGACCCTGAAGTTCATGGCCGGC
ATGGTCCCAGCCTCCTCGCTGGCGCCGGCTGGGCAACATTCGTGGCGAATGGGACC
ATTAGCCAAGCTGACCCTGAAGTTCATCTGATGAGACTCCGAATTCGGAGTCGAAAC
ACGGTAACCGTGTCATGAACTTCAGGGTCAGCTTGGCGGCCGGCATGGTCCCAGCCT
CCTCGCTGGCGCCGGCTGGGCAACATTCGTGGCGAATGGGACCCATTCAAACATGA
GGATTACCCATGTCGAAGCCATGG (SEQ ID NO: 29)

One Shot BL21 (DE3) Chemically Competent E. coli (Life Technologies) cells were transformed with the 2 plasmids one containing Sequence A (SEQ ID NO: 104) and Sequence H (SEQ ID NO: 126) selecting for chloramphenicol and ampicillin resistant transformants. For capsid production these transformants were grown at 37o in 750 mL LB medium containing both ampicillin and chloramphenicol. When the culture density reaches OD (600 nm)=0.8, isopropyl β-D-1-thiogalactopyranoside (Sigma-Aldrich) was then added to a final concentration of 1 mM. Cells were harvested 4 hours post-induction by centrifugation at 3,000 g and 4°C for 40 min..

### Example X: Production of MS2 Capsids using a transcript coding for 2 different siRNAs targeting Green Fluorescent Protein (GFP) flanked by spacers located at their 3' ends and 5' ends, attached to MS2 19-mer RNA hairpin.

Production of MS2 capsids is conducted as follows. The following DNA sequence (Sequence A; SEQ ID NO: 7), encoding MS2's coat protein was cloned into pDEST14 (Life Technologies) plasmid:

ACAAGTTTGTACAAAAAAGCAGGCTAAGAAGGAGATATACATATGGCTTCTAACTT
TACTCAGTTCGTTCTCGTCGACAATGGCGGAACTGGCGACGTGACTGTCGCCCCAAG
CAACTTCGCTAACGGGGTCGCTGAATGGATCAGCTCTAACTCGCGTTCACAGGCTTA
CAAAGTAACCTGTAGCGTTCGTCAGAGCTCTGCGCAGAATCGCAAATACACCATCA
AAGTCGAGGTGCCTAAAGTGGCAACCCAGACTGTTGGTGGTGTAGAGCTTCCTGTA
GCCGCATGGCGTTCGTACTTAAATATGGAACTAACCATTCCAATTTTCGCTACGAAT
TCCGACTGCGAGCTTATTGTTAAGGCAATGCAAGGTCTCCTAAAAGATGGAAACCC
GATTCCCTCAGCAATCGCAGCAAACTCCGGCATCTACTAATAG (SEQ ID NO: 7)

The following DNA sequence, Sequence I (SEQ ID NO: 30) was cloned into plasmid pACYC184. A transcription terminator was also cloned at the 3' end of Sequence I (SEQ ID NO: 30)(not shown). Sequence I (SEQ ID NO: 30) coded for T7 promoter and start sequence, 3 spacers separating the ends of the siRNAs against Green Fluorescent Protein (siGPF), 2 different siGFP hairpins, MS2's specific RNA 19-mer:

GGATCCTAATACGACTCACTATAGGGAGAAATAATAATCAAGCTGACCCTGAA
GTTCATCAAGAATGAACTTCAGGGTCAGCTTGTCAATAATAATCCGCTACCCCG
ACCACATGAACAAGATTCATGTGGTCGGGGTAGCGGTCAATAATAATACGCTT
CAAACATGAGGATTACCCATGTCGAAGCGACCATGG (SEQ ID NO: 30)

One Shot BL21 (DE3) Chemically Competent E. coli (Life Technologies) cells are transformed with the 2 plasmids one containing Sequence A (SEQ ID NO: 7) and Sequence I (SEQ ID NO: 30) selecting for chloramphenicol and ampicillin resistant transformants. For capsid production these transformants are grown at 37° in 750 mL LB medium containing both ampicillin and chloramphenicol. When the culture density reaches OD (600 nm)=0.8, isopropyl β-D-1-thiogalactopyranoside (Sigma-Aldrich) is then added to a final concentration of 1 mM. Cells are harvested 4 hours post-induction by centrifugation at 3,000 g and 4°C for 40 min. A sample is taken prior to induction and at the time of harvest for analysis.

### Example Y: Purification of MS2 VLPs obtained in Examples O through X.

MS2 capsids obtained in Examples O through X are purified using procedures outlined in Example N.

### Example Z: Isolation of RNA encapsidated in MS2 capsids obtained in Example N

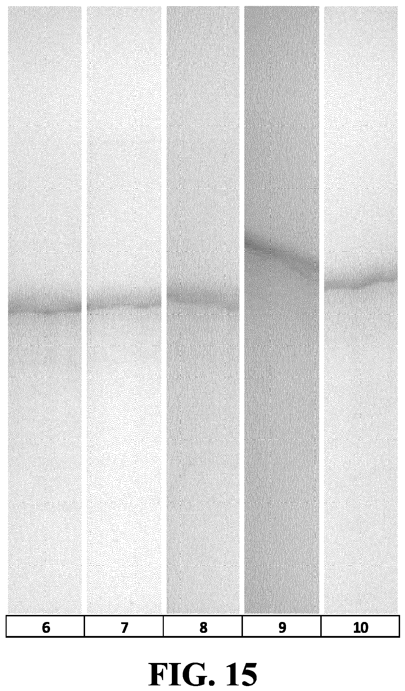

RNA encapsidated in MS2 capsids purified as described in Example N was extracted from each experiment using TRIzol^{®} reagent according to the protocol supplied by the manufacturer (Life Technologies, Grand Island, NY). RNA obtained was denatured by heating for 5 min at 95°C in formamide and analyzed by electrophoresis in 17.6cmx38cmx0.04cm (W,L,T) gels composed of 8% polyacrylamide, 8 molar urea, 1.08% Tris base, 0.55% Boric acid, and 0.093% EDTA. The running buffer had the same concentrations of Tris base, Boric acid and EDTA as the gel. Power was delivered at about 40W. Gels were stained using a 0.025% solution of Stains-All dye (Sigma-Aldrich, St. Louis, MO) in an aqueous mixture containing 25% formamide, 19% isopropanol and 15 mM Tris at pH 8. Results obtained are shown in Figure 15. Lane numbers for RNA electrophoresis in Figure 15 refer to the same lane numbers for protein electrophoresis in Figure 14. A single RNA band can be observed in each lane, consistent with high purity RNA recovered in each case.

### Example AA: HDV Ribozyme produced shRNA during in-vitro transcriptions.

Construct T7-Rz2 was used for *in-vitro* transcriptions. This construct was cloned into pACYC184 plasmid (New England Biolabs). One Shot BL21 (DE3) Chemically Competent E. coli (Life Technologies) cells were transformed using this plasmid. BL21(DE3) containing the plasmid were grown in LB medium containing ampicillin at 37°C, to OD(600nm) equal to 0.8. Plasmids were isolated using QIAprep^{®} Spin Miniprep Kit (Qiagen) following manufacturer's instructions. NcoI (New England Biolabs) was used to cut isolated plasmids at the restrictions sites introduced into this construct. After digestion, templates were purified by electrophoresis on 1.5% agarose gels and isolated using PureLink^{™} Quick Gel Extraction Kit (Life Technologies) following manufacturer's instructions. Reverse transcriptions were done using MAXIscript^{®} T7 Kit following manufacturer's instructions. RNA products were electrophoresed in Novex^{®} denaturing 15% polyacrylamide TBE-Urea gels (Life Technologies) run at 70°C. RNA bands were visualized using ethidium bromide (Sigma-Aldrich). Gel imaging was done using Image Lab 4.0.1 software (Bio-Rad).

Template T7-Rz2 encodes T7 promoter, shRNA hairpin, HDV ribozyme designed to cleave the 3' end of the shRNA hairpin, ATATATATAT spacer, and NcoI restriction site as follows:

TAATACGACTCACTATAGGCTTGTGATGCTTCAGCCAAATCAAGAGTTTGGCT
GAAGCATCACAAGCGGCCGGCATGGTCCCAGCCTCCTCGCTGGCGCCGGCTGGGCA
ACATTCGTGGCGAATGGGACCATATATATATACATGAGGATTACCCATGTCCATGG
(SEQ ID NO: 31)

The results show that the HDV ribozyme cut. The top band was the uncut transcript and the two lower bands were the expected cut pieces of RNA.

The gel in Figure 16 shows a set of RNA markers in the leftmost lane, a 49 nucleotide shRNA marker in the following lane, in-vitro transcription and cutting of T7-Rz2 construct run for 1 hour at 37°C in the third lane and in-vitro transcription and cutting of T7-Rz2 construct run for 1 hour at 37°C and incubated one additional hour at 42°C in the fourth, rightmost lane.

The slowest of the three most intense bands had a higher intensity at 37 than at 37/42. In addition, the two faster bands had higher intensity at 37/42 than at 37. The lowest molecular weight band in the last lane run slightly slower than the 49 nt shRNA standard, as expected for the resulting 51 nucleotide shRNA in T7Rz2. In summary, the results as shown in Figures 16 are consistent with the hypothesis that HDV ribozyme cut in T7-Rz2.

### Example BB: Long flanking Hammerhead Ribozymes cut to a significantly higher extent during in-vitro transcriptions than short flanking Hammerhead Ribozymes

Constructs T7-Rz1 and T7-Rz4 were used for *in-vitro* transcriptions. T7-Rz1 comprises common ribozymes, *i.e.*, with stems hybridizing the siRNA being cut of less than 6 pairs of hybridizing nucleotides. T7-Rz4 comprises flanking rybozymes with long-length stems that hybridize the siRNA being cut, i.e. stems with more than 6 pairs of hybridizing nucleotides.

Construct T7-Rz1 encoded T7 promoter, Hammerhead (HH) ribozyme designed to cleave the 5' end of a siRNA hairpin with 5 nucleotides complementary to siRNA, siRNA hairpin, HH ribozyme designed to cleave the 3' end of the siRNA hairpin with 5 nucleotides complementary to siRNA, and NcoI restriction site:

TAATACGACTCACTATAGGCTCGAGCAAGCCTGATGAGGCGCTTCGGCGCCGAAAC
ACCGTGTCGCTTGTGATGCTTCAGCCAAATCAAGAGTTTGGCTGAAGCATCACAAGC
TCACCGGATGTGCTCTCCGGTCTGATGAGTCCGTGAGGACGAAAGCTTGCCATGG
(SEQ ID NO: 32)

Construct T7-Rz4 encoded ansiRNA flanked by a HH ribozyme designed to cut its 5' end having 12 nucleotides hybridizing to the siRNA and a HH ribozyme designed to cut its 3' end having 23 nucleotides hybridizing to the siRNA:

TAATACGACTCACTATAGGGAGAACGCCGGCCATTCAAATAGTAAATAATAGAGGG
TCAGCTTGCTGATGAGGCGCTTCGGCGCCGAAACACCGTGTCCAAGCTGACCCTGAA
GTTCATCAAGAGTGAACTTCAGGGTCAGCTTGTCACCGGATGTGCTCTCCGGTCTGA
TGAGTCCGTGAGGACGAAACAAGCTGACCCTGAAGTTCACTACGCCGGCCATTCAA
ACATGAGGATTACCCATGTCCATGG (SEQ ID NO: 33)

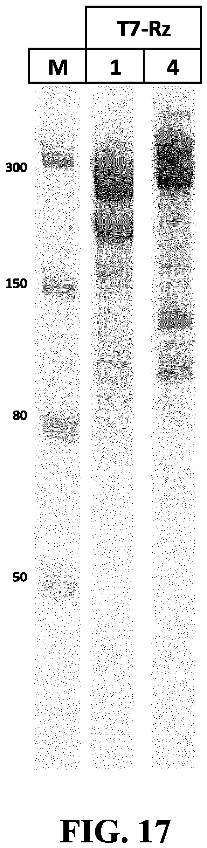

*In-vitro* transcription experiments and analyses were run with these constructs in a similar manner to those in Example Y. As shown in Figure 17, results obtained with construct T7-Rz1 were consistent with a hypothesis that ribozymes cut to a very low extent. Results obtained with construct T7-Rz4 shown in Figure 17 were consistent with a hypothesis that ribozymes cut to a significant extent.

### Example CC: Production of MS2 Capsids using a transcript coding for shRNA against EGFP flanked by a long Hammerhead ribozyme at its 5' end and another long Hammerhead ribozyme attached to MS2 19-mer RNA hairpin at its 3 end..

Production of MS2 capsids was conducted as follows. The following DNA sequence (Sequence A; SEQ ID NO: 7), encoding MS2's coat protein was cloned into pDEST14 (Life Technologies) plasmid:

ACAAGTTTGTACAAAAAAGCAGGCTAAGAAGGAGATATACATATGGCTTCTAACTT
TACTCAGTTCGTTCTCGTCGACAATGGCGGAACTGGCGACGTGACTGTCGCCCCAAG
CAACTTCGCTAACGGGGTCGCTGAATGGATCAGCTCTAACTCGCGTTCACAGGCTTA
CAAAGTAACCTGTAGCGTTCGTCAGAGCTCTGCGCAGAATCGCAAATACACCATCA
AAGTCGAGGTGCCTAAAGTGGCAACCCAGACTGTTGGTGGTGTAGAGCTTCCTGTA
GCCGCATGGCGTTCGTACTTAAATATGGAACTAACCATTCCAATTTTCGCTACGAAT
TCCGACTGCGAGCTTATTGTTAAGGCAATGCAAGGTCTCCTAAAAGATGGAAACCC
GATTCCCTCAGCAATCGCAGCAAACTCCGGCATCTACTAATAG (SEQ ID NO 7)

The following DNA sequence (construct T7-Rz4) was cloned into plasmid pACYC184. A transcription terminator was also cloned at the 3' end of construct T7-Rz4 (not shown).

TAATACGACTCACTATAGGGAGAACGCCGGCCATTCAAATAGTAAATAATAGAGGG
TCAGCTTGCTGATGAGGCGCTTCGGCGCCGAAACACCGTGTCCAAGCTGACCCTGAA
GTTCATCAAGAGTGAACTTCAGGGTCAGCTTGTCACCGGATGTGCTCTCCGGTCTGA
TGAGTCCGTGAGGACGAAACAAGCTGACCCTGAAGTTCACTACGCCGGCCATTCAA
ACATGAGGATTACCCATGTCCATGG (SEQ ID NO: 33)

One Shot BL21(DE3) Chemically Competent E. coli (Life Technologies) cells were transformed with the 2 plasmids one containing Sequence A (SEQ ID NO: 7) and construct T7-Rz4 (SEQ ID NO: 33) selecting for chloramphenicol and ampicillin resistant transformants. For capsid production these transformants were grown at 37° in 750 mL LB medium containing both ampicillin and chloramphenicol. When the culture density reached OD (600 nm)=0.8, isopropyl β-D-1-thiogalactopyranoside (Sigma-Aldrich) was then added to a final concentration of 1 mM. Cells were harvested 4 hours post-induction by centrifugation at 3,000 g and 4°C for 40 min. A sample was taken prior to induction and at the time of harvest for analysis.

### Example DD: Purification of Virus Like Particles obtained in Example CC.

Purification of Virus Like Particles produced in Example CC. was conducted as in Example N.

### Example EE: Isolation of RNA in Virus Like Particles obtained in Example DD

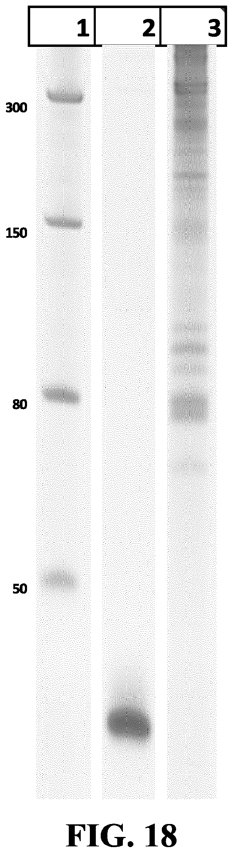

RNA encapsidated in Virus Like Particles purified as described in Example DD were extracted using TRIzol^{®} reagent according to the protocol supplied by the manufacturer (Life Technologies, Grand Island, NY). RNA obtained was denatured by heating for 5 min at 95°C in formamide and analyzed by electrophoresis in Novex^{®} denaturing15% polyacrylamide TBE-Urea gels (Life Technologies) run at 70°C. RNA bands were visualized using 0.5 µg of Ethidium Bromide (Sigma-Aldrich, St. Louis, MO) per mL of aqueous solution. Results obtained are shown in lane 3, Figure 18. Lane 1 shows a set of molecular standards. Lane 2 shows a chemically synthesized shRNA 49 nucleotides long.

### Example FF: Virus Like Particles comprising MS2 Capsids obtained in Example DD are resistant to Proteinase K from Engyodontium album, licheniformis, Pepsin from porcine gastric mucosa, and Papain from papaya latex.

Virus Like Particles comprising MS2 Capsids obtained from 250 mL of culture and purified as described in Example DD were suspended in 400 µL 20mM CaCl₂ aqueous solution at pH = 7.5.

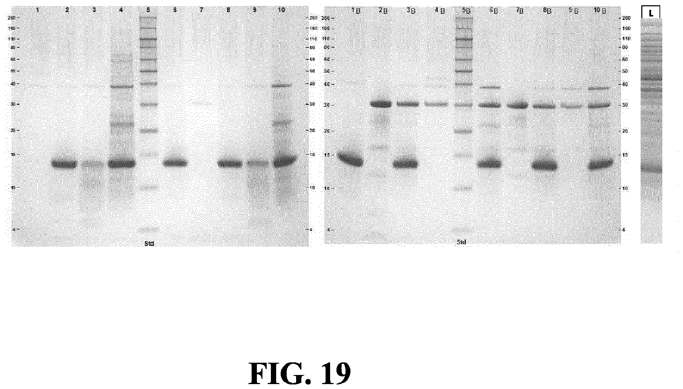

A 66 µL aliquote of this suspension was diluted to 0.25 mL with 20mM CaCl₂ aqueous solution at pH = 7.5 and incubated at 37°C. Samples were taken for protein concentration (Pierce^{®} BCA Protein Assay Kit, Thermo Fisher Scientific, Rockford, IL) and SDS PAGE analyses after 1 hour, and 4 hours of incubation. Protein concentration in these 2 samples was 3086, and 4656 mg/L respectively. SDS PAGE analyses are shown in Figure 19, Lanes 1B, and 6 respectively. The same amount of protein was loaded in each lane (4 µg). This set of experiments was used as a negative control.

2 µg Protease from *Streptomyces griseus* (Sigma Aldrich, St. Louis, MO) was diluted to 0.25 mL with 20mM CaCl₂ aqueous solution at pH = 7.5 and incubated at 37°C. Samples were taken for protein concentration and SDS PAGE analyses after 1 hour, and 4 hours of incubation. Protein concentration in these 2 samples was 361, and 324 mg/L respectively. SDS PAGE analyses are shown in Figure 19, Lanes 1, and 7 respectively. The same amount of protein was loaded in each lane (4 µg). This set of experiments was used as another negative control.

2 µg of Protease from *Streptomyces griseus* was added to another 66 µL aliquote of the Virus Like Particles comprising MS2 Capsids suspension, diluted to 0.25 mL with 20mM CaCl₂ aqueous solution at pH = 7.5 and incubated at 37°C. Samples were taken for protein concentration and SDS PAGE analyses after 1 hour, and 4 hours of incubation. Protein concentration in these 2 samples was 2940, and 3012 mg/L respectively. SDS PAGE analyses are shown in Figure 19, Lanes 2, and 8 respectively. The same amount of protein was loaded in each lane (4 µg). This set of experiments was used to test the proteolytic stability towards Protease from *Streptomyces griseus* of MS2 capsids forming the Virus Like Particles. Less than 10% degradation was observed.

Another 66 µL aliquote of the Virus Like Particles comprising MS2 Capsids suspension, diluted to 0.25 mL with 20mM CaCl₂ aqueous solution at pH = 7.5 was subjected to three cycles of heating to 95°C for 10 minutes and cooling on wet ice for 10 min to achieve the disassembly of the Virus Like Particles. 2 µg of Protease from *Streptomyces griseus* was then added to this suspension and was incubated at 37°C. Samples were taken for protein concentration and SDS PAGE analyses after 1 hour, and 4 hours of incubation. Protein concentration in these 2 samples was 2601, and 3033 mg/L respectively. SDS PAGE analyses are shown in Figure 19, Lanes 3, and 9 respectively. The same amount of protein was loaded in each lane (4 µg). Disassembled particles were degraded to a significant extent by Protease from *Streptomyces griseus.* This set of experiments was used as a positive control.

2 µg of Protease from *Streptomyces griseus* dissolved in 0.002 mL of 20mM CaCl₂ aqueous solution at pH = 7.5 was added to 0.248 mL of bacterial cell lysate obtained from 41 mL of cell culture from example CC and incubated at 37°C. Samples were taken for protein concentration and SDS PAGE analyses after 1 hour, and 4 hours of incubation. Protein concentration in these 2 samples was 3192, and 4837 mg/L respectively. SDS PAGE analyses are shown in Figure 19, Lanes 4, and 10 respectively. The last lane of Figure 19, labeled L, shows untreated bacterial cell lysate. The same amount of protein was loaded in each lane (4 µg). More than 90% of proteins other than MS2 capsid protein were degraded by Protease from *Streptomyces griseus.* This set of experiments was used as another positive control.

This set of five experiments demonstrate that MS2 capsids forming the Virus Like Particles of this disclosure are resistant to proteolysis by Protease from *Streptomyces griseus.*

2 µg Protease from Bacillus *licheniformis* (Sigma Aldrich, St. Louis, MO) was diluted to 0.25 mL with 10 mM Na acetate and 5 mM Ca acetate aqueous solution at pH = 7.5 and incubated at 37°C. Samples were taken for protein concentration and SDS PAGE analyses after 1 hour, and 4 hours of incubation. Protein concentration in these 2 samples was 976, and 1003 mg/L respectively. SDS PAGE analyses are shown in Figure 19, Lanes 2B, and 7B respectively. The same amount of protein was loaded in each lane (4 µg). This set of experiments was used as another negative control.

2 µg of Protease from *Bacillus licheniformis* was added to another 66 µL aliquote of the Virus Like Particles comprising MS2 Capsids suspension, diluted to 0.25 mL with 10 mM Na acetate and 5 mM Ca acetate aqueous solution at pH = 7.5 and incubated at 37°C. Samples were taken for protein concentration and SDS PAGE analyses after 1 hour, and 4 hours of incubation. Protein concentration in these 2 samples was 3144, and 3727 mg/L respectively. SDS PAGE analyses are shown in Figure 19, Lanes 3B, and 8B respectively. The same amount of protein was loaded in each lane (4 µg). This set of experiments was used to test the proteolytic stability towards Protease from *Bacillus licheniformis* of MS2 capsids forming the Virus Like Particles. Less than 10% degradation was observed.

Another 66 µL aliquote of the Virus Like Particles comprising MS2 Capsids suspension, diluted to 0.25 mL with 10 mM Na acetate and 5 mM Ca acetate aqueous solution at pH = 7.5 was subjected to three cycles of heating to 95°C for 10 minutes and cooling on wet ice for 10 min to achieve the disassembly of the Virus Like Particles. 2 µg of Protease from *Bacillus licheniformis* was then added to this suspension and was incubated at 37°C. Samples were taken for protein concentration and SDS PAGE analyses after 1 hour, and 4 hours of incubation. Protein concentration in these 2 samples was 1769, and 1785 mg/L respectively. SDS PAGE analyses are shown in Figure 19, Lanes 4B, and 9B respectively. The same amount of protein was loaded in each lane (4 µg). Disassembled particles were degraded by Protease from *Bacillus licheniformis.* This set of experiments was used as a positive control.

2 µg of Protease from *Bacillus licheniformis* dissolved in 0.002 mL of 10 mM Na acetate and 5 mM Ca acetate aqueous solution at pH = 7.5 was added to 0.248 mL of bacterial cell lysate obtained from 41 mL of cell culture from example CC and incubated at 37°C. Samples were taken for protein concentration and SDS PAGE analyses after 1 hour, and 4 hours of incubation. Protein concentration in these 2 samples was 3696, and 4078 mg/L respectively. SDS PAGE analyses are shown in Figure 19, Lanes 6B, and 10B respectively. The last lane of Figure 19, labeled L, shows untreated bacterial cell lysate. The same amount of protein was loaded in each lane (4 µg). More than 90% of proteins other than MS2 capsid protein were degraded by Protease from *Bacillus licheniformis.* This set of experiments was used as another positive control.

This set of four experiments demonstrated that MS2 capsids forming the Virus Like Particles of this disclosure are resistant to proteolysis by Protease from *Bacillus licheniformis.*

Three additional sets of equivalent experiments demonstrated that MS2 capsids forming the Virus Like Particles of this disclosure are resistant to proteolysis by any of the following three proteases: Proteinase K from *Engyodontium album,* Pepsin from porcine gastric mucosa (CAS Number 9001-75-6), and Papain from papaya latex (CAS Number 9001-73-4) (Sigma-Aldrich, St. Louis, MO). Each protease was used according the manufacturer's instructions. Proteinase K was used at pH = 7.5, Pepsin was used at pH = 1.6, and Papain was used at pH = 6.6.

### Example GG: Capsid coat protein variants

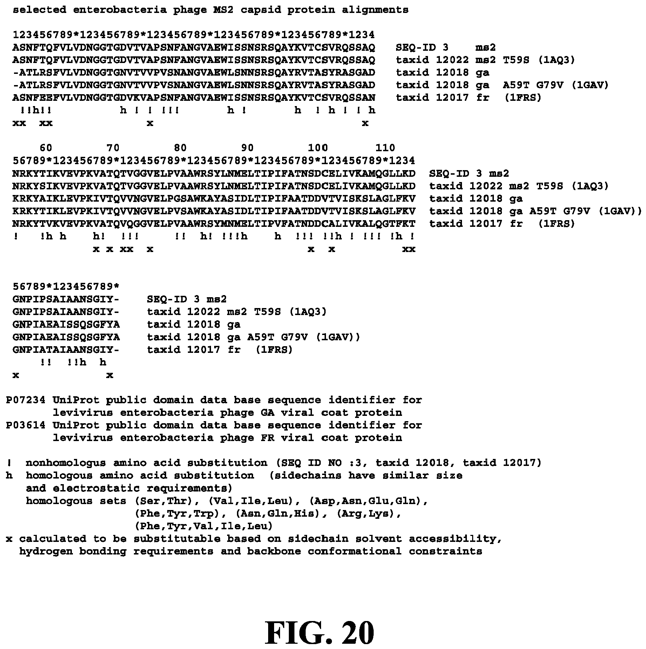

The MS2 viral capsid protein (SEQ.ID No. 3) has a single folding domain and belongs to fold family d.85.1 (RNA bacteriophage capsid protein) of superfamily d.85, which includes leviviridae and alloleviviridae capsid proteins. Each capsid monomer in this family is made up of a 6-stranded beta sheet followed by the two helices (sometimes described as a long helix with a kink). 180 monomers assemble noncovalently to form an icosahedral (roughly spherical) viral capsid with a continuous beta-sheet layer facing the capsid interior and the alpha-helices on the capsid exterior. X-ray crystal structures have been solved and placed in the public domain for the enterobacteriophage MS2, GA (UniProt sequence identifier P07234) and FR (UniProt sequence identifier P03614) viral capsids and the capsid of MS2 formed from an MS2 dimer in which one C-terminus of one MS2 has been fused to the N-terminus of another, all d. 85.1 family leviviridae coat proteins. The Protein Data Bank identifiers for these structures are 1AQ3 (SEQ ID NO: 34), 1GAV (SEQ ID NO: 35), 1FRS (SEQ ID NO: 36) and 2VTU (SEQ ID NO: 37), respectively, and alignment of these is shown Figure 20. In this an all alignments described herein, the residue numbering is sequential residue numbering, for example SEQ ID 3 starting with 0 for the lead Met (M) residue which is removed by the cell, as used for most PDB structures.

The sequences of MS2 viral capsid protein vs the GA and FR viral capsid proteins are 59% and 87% identical respectively. Only 56% of the sequence positions have identical sequence and topologically equivalent positions with respect to the backbone overlays when all three sequences are considered together. The rms deviation of the backbone conformations of MS2 viral capsid protein vs the GA and FR viral capsid monomers are under 1A. The backbone rms deviation of 1AQ3 monomer A vs 1GAV monomer 0 is 0.89 Angstroms. The backbone rms deviation of 1AQ3 monomer A vs 1FRS monomer A is 0.37 Angstroms. Comparisons were made using the freeware utility jFATCAT rigid (Prlic, et al, Bioinformatics 26,2983-2985 (2010); www.rcsb.org/pdb/workbench/workbench.do; www.rcsb.org/pdb/workbench/workbench.do), a tool familiar to practitioners of structure study protein available at the RCSB Protein Data Bank site in their standard workbench of protein structure tools. The overall fold of these proteins is identical. There are no insertions or deletions. Each protein in the crystallographic asymmetric unit is independently refined. Different, compositionally identical proteins within an asymmetric unit generally backbone rms deviations of 1 Angstrom or greater although topologically equivalent Calpha atoms of the core tend to differ by less, about 0.45 Anstroms (Cyrus Chothia & Arthur M Lesk (1986) EMBO J 5, 823-826). For example, 1AQ3 monomer A and 1AQ3 monomer B have rms deviation of 1.72A (jFATCAT rigid) primarily because of conformational differences in the Lys66-Trp82 region.

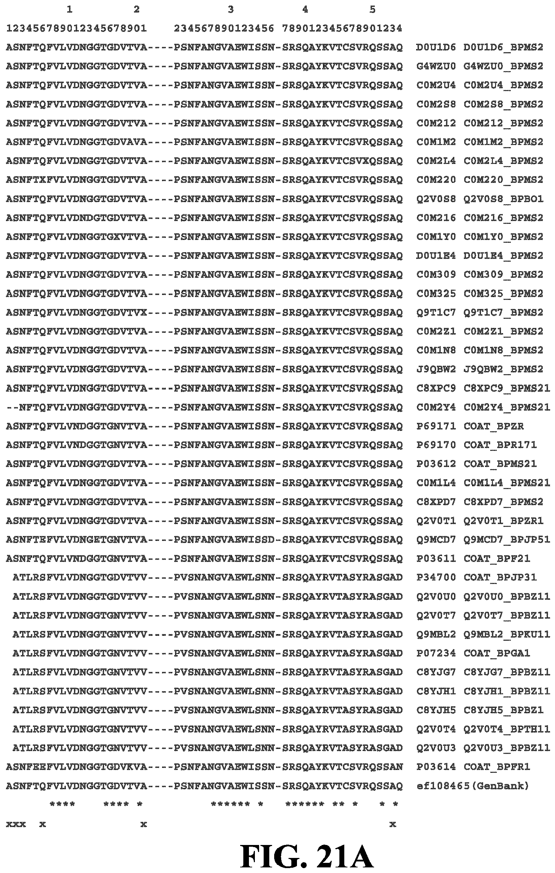

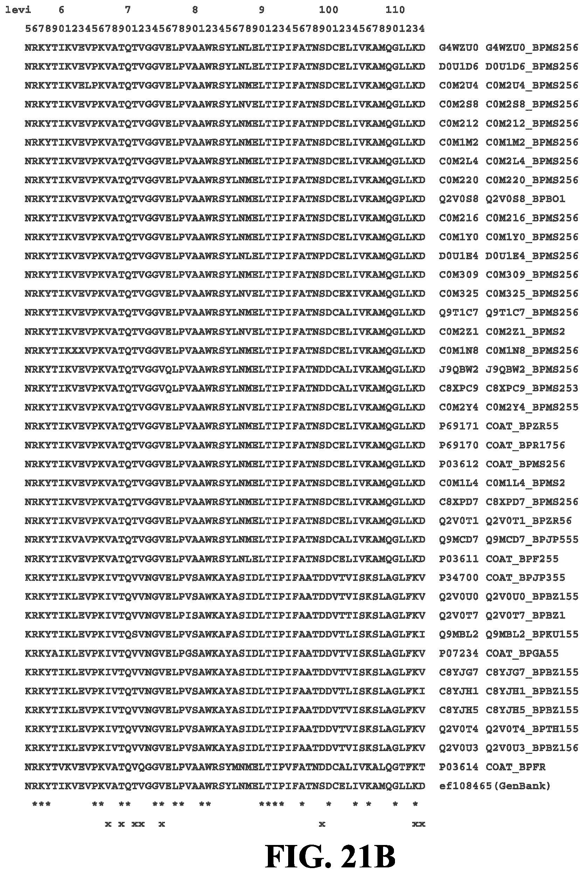

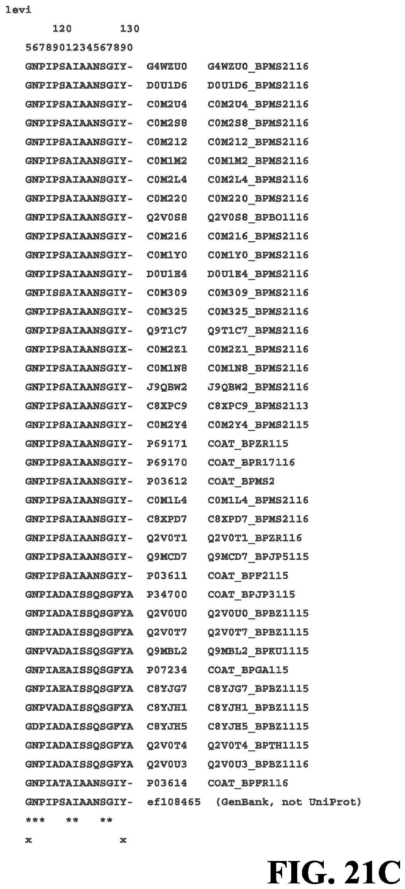

If sufficient members of a fold family have been identified, a clear picture of conserved residues, topologically equivalent residue positions within the sequences which seldom or never mutate within the family, emerges. Nonconserved positions can be expected to mutate from one sequence to another without disturbing the family fold, perhaps in conjunction with the concerted mutation of spatial neighbor(s) in the fold particularly if the sidechain packs against the sidechain(s) of the spatial neighbors. Conserved residues can be critical for fold stability, function or processing of the protein, for example proteolytic digestion. Some can be coincidentally conserved. GenBank (Dennis A. Benson, Ilene Karsch-Mizrachi, David J. Lipman, James Ostell, and David L. Wheeler (2005) Nucleic Acids Res 33, D34-D38) currently holds 353 leviviridae coat protein sequences. The alignment table shown in Figure 21 shows the multiple alignment of 40 complete leviviridae coat protein sequences retrieved from the global protein sequence database UniProt (Universal Protein Resource, (The UniProt Consortium, Reorganizing the protein space at the Universal Protein Resource (UniProt) Nucleic Acids Res. 40: D71-D75 (2012)), http://www.uniprot.org) (See Table 1 below) and aligned with BLAST (threshold=10, Auto weighting array selection, no filtering, gaps allowed). All sequences except ef108465 were taken from UniProt. ef108465 came from GenBank (www.ncbi.nlm.nih.gov/genbank). In the alignment table, asterisk (*) indicates conserved residues, x is calculated to be substitutable based on sidechain solvent accessibility, hydrogen bonding requirements and backbone conformational constraints. Fifty-seven (57) residues in the sequences of these family members are conserved, or 45% of the sequences are identical to on another. Some of these sequences have an additional residue following the C-terminal Tyr129 residue of SEQ ID NO: 3, others have 1-2 residues removed from the N-terminus with respect to SEQ ID NO: 3. There are no insertions or deletions within the fold.

**Table 1: List of 40 complete leviviridae coat protein sequences retrieved from the global protein sequence database UniProt**

| **Accession** | **Entry Name** | **Organism** | **Identifier** |
|---|---|---|---|
| G4WZU0 | G4WZU0_BPMS2116 | enterobacteria phage ms2 | 329852 |
| D0U1D6 | D0U1D6_BPMS2116 | enterobacteria phage ms2 | 12022 |
| C0M2U4 | C0M2U4_BPMS2116 | enterobacteria phage ms2 | 12022 gene ms2g2 |
| C0M2S8 | C0M2S8_BPMS2116 | enterobacteria phage ms2 | 12022 gene ms2g2 |
| C0M212 | C0M212_BPMS2116 | enterobacteria phage ms2 | 12022 gene ms2g2 |
| COM1M2 | C0M1M2_BPMS2116 | enterobacteria phage ms2 | 12022 gene ms2g2 |
| C0M2L4 | C0M2L4_BPMS2116 | enterobacteria phage ms2 | 12022 gene ms2g2 |
| C0M220 | C0M220_BPMS2116 | enterobacteria phage ms2 | 12022 gene ms2g2 |
| Q2V0S8 | Q2V0S8_BPBO1116 | enterobacteria phage bo1 | 12014 |
| C0M216 | C0M216_BPMS2116 | enterobacteria phage ms2 | 12022 gene ms2g2 |
| C0M1Y0 | C0M1Y0_BPMS2116 | enterobacteria phage ms2 | 12022 gene ms2g2 |
| D0U1E4 | D0U1E4_BPMS2116 | enterobacteria phage ms2 | 12022 gene ms2g2 |
| C0M309 | C0M309_BPMS2116 | enterobacteria phage ms2 | 12022 gene ms2g2 |
| C0M325 | C0M325_BPMS2116 | enterobacteria phage ms2 | 12022 gene ms2g2 |
| Q9T1C7 | Q9T1C7_BPMS2116 | enterobacteria phage ms12 | 110679 |
| C0M2Z1 | C0M2Z1_BPMS2116 | enterobacteria phage ms2 | 12022 gene ms2g2 |
| C0M1N8 | C0M1N8_BPMS2116 | enterobacteria phage ms2 | 12022 gene ms2g2 |
| J9QBW2 | J9QBW2_BPMS2116 | enterobacteria phage ms2 | 12022 gene ms2g2 |
| C8XPC9 | C8XPC9_BPMS2113 | enterobacteria phage ms2 | 329852 |
| C0M2Y4 | C0M2Y4_BPMS2115 | enterobacteria phage ms2 | 12022 gene ms2g2 |
| P69171 | COAT_BPZR115 | enterobacteria phage zr | 332942 |
| P69170 | COAT_BPR17116 | enterobacteria phage r17 | 12026 |
| P03612 | COAT_BPMS2 | enterobacteria phage ms2 | 329852 |
| C0M1L4 | C0M1L4_BPMS2116 | enterobacteria phage ms2 | 12022 gene ms2g2 |
| C8XPD7 | C8XPD7_BPMS2116 | enterobacteria phage ms2 | 329852 gene cp |
| Q2V0T1 | Q2V0T1_BPZR116 | enterobacteria phage zr | 332942 |
| Q9MCD7 | Q9MCD7_BPJP5115 | enterobacteria phage jp501 | 12020 |
| P03611 | COAT_BPF2115 | enterobacteria phage f2 12016 | |
| P34700 | COAT_BPJP3115 | enterobacteria phage jp34 | 12019 |
| Q2V0U0 | Q2V0U0_BPBZ1115 | enterobacteria phage jp500 | 332939 |
| Q2V0T7 | Q2V0T7 BPBZ1115 | enterobacteria phage sd | 332940 |
| Q9MBL2 | Q9MBL2_BPKU1115 | enterobacteria phage ku1 | 12021 |
| P07234 | COAT_BPGA115 | enterobacteria phage ga | 12018 |
| C8YJG7 | C8YJG7 BPBZ1115 | enterobacteria phage bz13 | 329853 |
| C8YJH1 | C8YJH1_BPBZ1115 | enterobacteria phage bz13 | 329853 |
| C8YJH5 | C8YJH5_BPBZ1115 | enterobacteria phage bz13 | 329853 |
| Q2V0T4 | Q2V0T4_BPTH1115 | enterobacteria phage th1 | 12029 |
| Q2V0U3 | Q2V0U3_BPBZ1116 | enterobacteria phage t12 | 332938 |
| P03614 | COAT_BPFR116 | enterobacteria phage fr | 12017 |
| ef108465 | | enterobacteria phage r17 | 329852 |

SEQ ID NO: 34 1AQ3 Enterobacteria phage MS2 coat protein T59S 
ASNFTQFVLVDNGGTGDVTVAPSNFANGVAEWISSNSRSQAYKVTCSVRQSSAQNRKYSI
KVEVPKVATQTVGGVELPVAAWRSYLNMELTIPIFATNSDCELIVKAMQGLLKDGNPIPS
AIAANSGIY
SEQ ID NO: 35 1GAV Enterobacteria phage GA coat protein A59T G79V 
ATLRSFVLVDNGGTGNVTVVPVSNANGVAEWLSNNSRSQAYRVTASYRASGADKRKYTIK
LEVPKIVTQVVNGVELPGSAWKAYASIDLTIPIFAATDDVTVISKSLAGLFKVGNPIAEA
ISSQSGFYA
SEQ ID NO: 36 1FRS Enterobacteria phage FR coat protei >sp|P03614|COAT_BPFR Coat protein OS=Enterobacteria phage fr PE=1 SV=4 
ASNFEEFVLVDNGGTGDVKVAPSNFANGVAEWISSNSRSQAYKVTCSVRQSSANNRKYTV
KVEVPKVATQVQGGVELPVAAWRSYMNMELTIPVFATNDDCALIVKALQGTFKTGNPIAT
AIAANSGIY
SEQ ID NO: 37 2VTU Enterobacteria phage MS2 coat protein covalent dimer
   sp|P03612|COAT_BPMS2 Coat protein OS=Enterobacteria phage MS2 PE=1 SV=2 
ASNFTQFVLVDNGGTGDVTVAPSNFANGVAEWISSNSRSQAYKVTCSVRQSSAQNRKYTI
KVEVPKVATQTVGGVELPVAAWRSYLNMELTIPIFATNSDCELIVKAMQGLLKDGNPIPS
 
AIAANSGIYANFTQFVLVDNGGTGDVTVAPSNFANGVAEWISSNSRSQAYKVTCSVRQSS
AQNRKYTIKVEVPKVATQTVGGVELPVAAWRSYLNMELTIPIFATNSDCELIVKAMQGLL
KDGNPIPSAIAANSGIY
SEQ ID NO: 38 (SEQ ID NO: 38) G4WZU0 G4WZU0_BPMS2116 enterobacteria phage ms2 329852
   >sp|P03612|COAT_BPMS2 Coat protein OS=Enterobacteria phage MS2 PE=1 SV=2 
MASNFTQFVLVDNGGTGDVTVAPSNFANGVAEWISSNSRSQAYKVTCSVRQSSAQNRKYT
IKVEVPKVATQTVGGVELPVAAWRSYLNLELTIPIFATNSDCELIVKAMQGLLKDGNPIP
SAIAANSGIY
SEQ ID NO: 39
   D0U1D6 D0U1D6_BPMS2116 enterobacteria phage ms2 12022
   >tr|D0U1D6|D0U1D6_BPMS2 Coat protein OS=Enterobacterio phage MS2 PE=4 SV=1 
MASNFTQFVLVDNGGTGDVTVAPSNFANGVAEWISSNSRSQAYKVTCSVRQSSAQNRKYTIKVEVPKVAT
QTVGGVELPVAAWRSYLNLELTIPIFATNSDCELIVKAMQGLLKDGNPIPSAIAANSGIY
SEQ ID NO: 40
   C0M2U4 C0M2U4_BPMS2116 enterobacteria phage ms2 12022 gene ms2g2
   >tr|C0M2U4|C0M2U4_BPMS2 Coat protein OS=Enterobacterio phage MS2 GN=MS2g2 PE=4 SV=1 
MASNFTQFVLVDNGGTGDVTVAPSNFANGVAEWISSNSRSQAYKVTCSVRQSSAQNRKYTIKVELPKVAT
QTVGGVELPVAAWRSYLNMELTIPIFATNSDCELIVKAMQGLLKDGNPIPSAIAANSGIY
SEQ ID NO: 41
   C0M2S8 C0M2S8_BPMS2116 enterobacteria phage ms2 12022 gene ms2g2
   >tr|C0M2S8|C0M2S8_BPMS2 Coat protein OS=Enterobacterio phage MS2 GN=MS2g2 PE=4 SV=1 
MASNFTQFVLVDNGGTGDVTVAPSNFANGVAEWISSNSRSQAYKVTCSVRQSSAQNRKYTIKVEVPKVAT
QTVGGVELPVAAWRSYLNVELTIPIFATNSDCELIVKAMQGLLKDGNPIPSAIAANSGIY
SEQ ID NO: 42
   C0M212 C0M212_BPMS2116 enterobacteria phage ms2 12022 gene ms2g2
   tr|C0M212|C0M212_BPMS2 Coat protein OS=Enterobacterio phage MS2 GN=MS2g2 PE=4 SV=1 
MASNFTQFVLVDNGGTGDVTVAPSNFANGVAEWISSNSRSQAYKVTCSVRQSSAQNRKYTIKVEVPKVAT
QTVGGVELPVAAWRSYLNMELTIPIFATNPDCELIVKAMQGLLKDGNPIPSAIAANSGIY
SEQ ID NO: 43
   C0M1M2 C0M1M2_BPMS2116 enterobacteria phage ms2 12022 gene ms2g2
   >tr|C0M1M2|C0M1M2_BPMS2 Coat protein OS=Enterobacterio phage MS2 GN=MS2g2 PE=4 SV=1 
MASNFTQFVLVDNGGTGDVAVAPSNFANGVAEWISSNSRSQAYKVTCSVRQSSAQNRKYTIKVEVPKVAT
QTVGGVELPVAAWRSYLNMELTIPIFATNSDCELIVKAMQGLLKDGNPIPSAIAANSGIY
SEQ ID NO: 44
   C0M2L4 C0M2L4_BPMS2116 enterobacteria phage ms2 12022 gene ms2g2
   >tr|C0M2L4|C0M2L4_BPMS2 Coat protein OS=Enterobacterio phage MS2 GN=MS2g2 PE=4 SV=1 
MASNFTQFVLVDNGGTGDVTVAPSNFANGVAEWISSNSRSQAYKVTCSVXQSSAQNRKYTIKVEVPKVAT
QTVGGVELPVAAWRSYLNMELTIPIFATNSDCELIVKAMQGLLKDGNPIPSAIAANSGIY
SEQ ID NO: 45
   C0M220 C0M220_BPMS2116 enterobacteria phage ms2 12022 gene ms2g2
   >tr|C0M220|C0M220_BPMS2 Coat protein OS=Enterobacterio phage MS2 GN=MS2g2 PE=4 SV=1 
MASNFTXFVLVDNGGTGDVTVAPSNFANGVAEWISSNSRSQAYKVTCSVRQSSAQNRKYTIKVEVPKVAT
QTVGGVELPVAAWRSYLNMELTIPIFATNSDCELIVKAMQGLLKDGNPIPSAIAANSGIY
SEQ ID NO: 46
   Q2V0S8 Q2V0S8_BPB01116 enterobacteria phage bo1 12014
   >tr|Q2V0S8|Q2V0S8_BPBO1 Coat protein OS=Enterobacteria phage BO1 PE=4 SV=1 
MASNFTQFVLVDNGGTGDVTVAPSNFANGVAEWISSNSRSQAYKVTCSVRQSSAQNRKYTIKVEVPKVAT
QTVGGVELPVAAWRSYLNMELTIPIFATNSDCELIVKAMQGPLKDGNPIPSAIAANSGIY
SEQ ID NO: 47
   C0M216 C0M216_BPMS2116 enterobacteria phage ms2 12022 gene ms2g2
   >tr|C0M216|C0M216_BPMS2 Coat protein OS=Enterobacterio phage MS2 GN=MS2g2 PE=4 SV=1 
MASNFTQFVLVDNDGTGDVTVAPSNFANGVAEWISSNSRSQAYKVTCSVRQSSAQNRKYTIKVEVPKVAT
QTVGGVELPVAAWRSYLNMELTIPIFATNSDCELIVKAMQGLLKDGNPIPSAIAANSGIY
SEQ ID NO: 48
   C0M1Y0 C0M1Y0_BPMS2116 enterobacteria phage ms2 12022 gene ms2g2
   >tr|C0M1Y0|C0M1Y0_BPMS2 Coat protein OS=Enterobacterio phage MS2 GN=MS2g2 PE=4 SV=1 
MASNFTQFVLVDNGGTGXVTVAPSNFANGVAEWISSNSRSQAYKVTCSVRQSSAQNRKYTIKVEVPKVAT
QTVGGVELPVAAWRSYLNMELTIPIFATNSDCELIVKAMQGLLKDGNPIPSAIAANSGIY
SEQ ID NO: 49
   D0U1E4 D0U1E4_BPMS2116 enterobacteria phage ms2 12022 gene ms2g2
   >tr|D0U1E4|D0U1E4_BPMS2 Coat protein OS=Enterobacterio phage MS2 PE=4 SV=1 
MASNFTQFVLVDNGGTGDVTVAPSNFANGVAEWISSNSRSQAYKVTCSVRQSSAQNRKYTIKVEVPKVAT
QTVGGVELPVAAWRSYLNLELTIPIFATNPDCELIVKAMQGLLKDGNPIPSAIAANSGIY
SEQ ID NO: 50
   C0M309 C0M309_BPMS2116 enterobacteria phage ms2 12022 gene ms2g2
   >tr|C0M309|C0M309_BPMS2 Coat protein OS=Enterobacterio phage MS2 GN=MS2g2 PE=4 SV=1 
MASNFTQFVLVDNGGTGDVTVAPSNFANGVAEWISSNSRSQAYKVTCSVRQSSAQNRKYTIKVEVPKVAT
QTVGGVELPVAAWRSYLNMELTIPIFATNSDCELIVKAMQGLLKDGNPISSAIAANSGIY
SEQ ID NO: 51
   C0M325 C0M325_BPMS2116 enterobacteria phage ms2 12022 gene ms2g2
   >tr|C0M325|C0M325_BPMS2 Coat protein OS=Enterobacterio phage MS2 GN=MS2g2 PE=4 SV=1 
MASNFTQFVLVDNGGTGDVTVAPSNFANGVAEWISSNSRSQAYKVTCSVRQSSAQNRKYTIKVEVPKVAT
QTVGGVELPVAAWRSYLNVELTIPIFATNSDCEXIVKAMQGLLKDGNPIPSAIAANSGIY
SEQ ID NO: 52
   Q9T1C7 Q9T1C7_BPMS2116 enterobacteria phage ms12 110679
   >tr|Q9T1C7|Q9T1C7_BPMS2 Coat protein OS=Enterobacteria phage M12 PE=4 SV=1 
MASNFTQFVLVDNGGTGDVTVXPSNFANGVAEWISSNSRSQAYKVTCSVRQSSAQNRKYTIKVEVPKVAT
QTVGGVELPVAAWRSYLNMELTIPIFATNSDCALIVKAMQGLLKDGNPIPSAIAANSGIY
SEQ ID NO: 53
   C0M2Z1 C0M2Z1_BPMS2116 enterobacteria phage ms2 12022 gene ms2g2
   >tr|C0M2Z1|C0M2Z1_BPMS2 Coat protein OS=Enterobacterio phage MS2 GN=MS2g2 PE=4 SV=1 
MASNFTQFVLVDNGGTGDVTVAPSNFANGVAEWISSNSRSQAYKVTCSVRQSSAQNRKYTIKVEVPKVAT
QTVGGVELPVAAWRSYLNVELTIPIFATNSDCELIVKAMQGLLKDGNPIPSAIAANSGIX
SEQ ID NO: 54
   C0M1N8 C0M1N8_BPMS2116 enterobacteria phage ms2 12022 gene ms2g2
   >tr|C0M2Z1|C0M2Z1_BPMS2 Coat protein OS=Enterobacterio phage MS2 GN=MS2g2 PE=4 SV=1 
MASNFTQFVLVDNGGTGDVTVAPSNFANGVAEWISSNSRSQAYKVTCSVRQSSAQNRKYTIKVEVPKVAT
QTVGGVELPVAAWRSYLNVELTIPIFATNSDCELIVKAMQGLLKDGNPIPSAIAANSGIX
SEQ ID NO: 55
   J9QBW2 J9QBW2_BPMS2116 enterobacteria phage ms2 12022 gene ms2g2
   >tr|J9QBW2|J9QBW2_BPMS2 Capsid protein OS=Enterobacterio phage MS2 PE=4 SV=1 
MASNFTQFVLVDNGGTGDVTVAPSNFANGVAEWISSNSRSQAYKVTCSVRQSSAQNRKYTIKVEVPKVAT
QTVGGVQLPVAAWRSYLNMELTIPIFATNDDCALIVKAMQGLLKDGNPIPSAIAANSGIY
SEQ ID NO: 56
   C8XPC9 C8XPC9_BPMS2113 enterobacteria phage ms2 329852
   >tr|C8XPC9|C8XPC9_BPMS2 Coat protein OS=Enterobacteria phage MS2 PE=4 SV=1 
MASNFTQFVLVDNGGTGDVTVAPSNFANGVAEWISSNSRSQAYKVTCSVRQSSAQNRKYTIKVEVPKVAT
QTVGGVQLPVAAWRSYLNMELTIPIFATNDDCALIVKAMQGLLKDGNPIPSAIAANSGIY
SEQ ID NO: 57
   C0M2Y4 C0M2Y4_BPMS2115 enterobacteria phage ms2 12022 gene ms2g2
   >tr|C0M2Y4|C0M2Y4_BPMS2 Coat protein (Fragment) OS=Enterobacterio phage MS2 GN=MS2g2 PE=4 SV=1 
NFTQFVLVDNGGTGDVTVAPSNFANGVAEWISSNSRSQAYKVTCSVRQSSAQNRKYTIKVEVPKVATQTV
GGVELPVAAWRSYLNVELTIPIFATNSDCELIVKAMQGLLKDGNPIPSAIAANSGIY
SEQ ID NO: 58
   P69171 COAT_BPZR115 enterobacteria phage zr 332942
   >sp|P69171|COAT_BPZR Coat protein OS=Enterobacteria phage ZR PE=1 SV=1 
ASNFTQFVLVNDGGTGNVTVAPSNFANGVAEWISSNSRSQAYKVTCSVRQSSAQNRKYTIKVEVPKVATQ
TVGGVELPVAAWRSYLNMELTIPIFATNSDCELIVKAMQGLLKDGNPIPSAIAANSGIY
SEQ ID NO: 59
   P69170 COAT_BPR17116 enterobacteria phage r17 12026
   >sp|P69170|COAT_BPR17 Coat protein OS=Enterobacteria phage R17 PE=1 SV=1 
ASNFTQFVLVNDGGTGNVTVAPSNFANGVAEWISSNSRSQAYKVTCSVRQSSAQNRKYTIKVEVPKVATQ
TVGGVELPVAAWRSYLNMELTIPIFATNSDCELIVKAMQGLLKDGNPIPSAIAANSGIY
SEQ ID NO: 60
   P03612 COAT_BPMS2 enterobacteria phage ms2 329852
   >sp|P03612|COAT_BPMS2 Coat protein OS=Enterobacteria phage MS2 PE=1 SV=2 
MASNFTQFVLVDNGGTGDVTVAPSNFANGVAEWISSNSRSQAYKVTCSVRQSSAQNRKYTIKVEVPKVAT
QTVGGVELPVAAWRSYLNMELTIPIFATNSDCELIVKAMQGLLKDGNPIPSAIAANSGIY
SEQ ID NO: 61
   C0M1L4 C0M1L4_BPMS2116 enterobacteria phage ms2 12022 gene ms2g2 >tr|C0M1L4|C0M1L4_BPMS2 Coat protein OS=Enterobacterio phage MS2 GN=MS2g2 PE=2 SV=1 
MASNFTQFVLVDNGGTGDVTVAPSNFANGVAEWISSNSRSQAYKVTCSVRQSSAQNRKYTIKVEVPKVAT
QTVGGVELPVAAWRSYLNMELTIPIFATNSDCELIVKAMQGLLKDGNPIPSAIAANSGIY
SEQ ID NO: 62
   C8XPD7 C8XPD7_BPMS2116 enterobacteria phage ms2 329852 gene cp
   >tr|C8XPD7|C8XPD7_BPMS2 Coat protein OS=Enterobacteria phage MS2 GN=cp PE=4 SV=1 
MASNFTQFVLVDNGGTGDVTVAPSNFANGVAEWISSNSRSQAYKVTCSVRQSSAQNRKYTIKVEVPKVAT
QTVGGVELPVAAWRSYLNMELTIPIFATNSDCELIVKAMQGLLKDGNPIPSAIAANSGIY
SEQ ID NO: 63
   Q2V0T1 Q2V0T1_BPZR116 enterobacteria phage zr 332942
   >tr|Q2V0T1|Q2V0T1_BPZR Coat protein OS=Enterobacteria phage ZR PE=4 SV=1 
MASNFTQFVLVDNGGTGDVTVAPSNFANGVAEWISSNSRSQAYKVTCSVRQSSAQNRKYTIKVEVPKVAT
QTVGGVELPVAAWRSYLNMELTIPIFATNSDCELIVKAMQGLLKDGNPIPSAIAANSGIY
SEQ ID NO: 64
   Q9MCD7 Q9MCD7_BPJP5115 enterobacteria phage jp501 12020
   >tr|Q9MCD7|Q9MCD7_BPJP5 Coat protein OS=Enterobacteria phage JP501 PE=4 SV=1 
MASNFTEFVLVDNGETGNVTVAPSNFANGVAEWISSDSRSQAYKVTCSVRQSSAQNRKYTIKVAVPKVAT
QTVGGVELPVAAWRSYLNMELTIPIFATNSDCALIVKAMQGLLKDGNPIPSAIAANSGIY
SEQ ID NO: 65
   P03611 COAT_BPF2115 enterobacteria phage f2 12016
   >sp|P03611|COAT_BPF2 Coat protein OS=Enterobacteria phage f2 PE=1 SV=1 
ASNFTQFVLVNDGGTGNVTVAPSNFANGVAEWISSNSRSQAYKVTCSVRQSSAQNRKYTIKVEVPKVATQ
TVGGVELPVAAWRSYLNLELTIPIFATNSDCELIVKAMQGLLKDGNPIPSAIAANSGIY
SEQ ID NO: 66
   P34700 COAT_BPJP3115 enterobacteria phage jp34 12019
   >sp|P34700|COAT_BPJP3 Coat protein OS=Enterobacteria phage JP34 PE=3 SV=2 
MATLRSFVLVDNGGTGDVTVVPVSNANGVAEWLSNNSRSQAYRVTASYRASGADKRKYTIKLEVPKIVTQ
VVNGVELPVSAWKAYASIDLTIPIFAATDDVTVISKSLAGLFKVGNPIADAISSQSGFYA
SEQ ID NO: 67
   Q2V0U0 Q2V0U0_BPBZ1115 enterobacteria phage jp500 332939
   >tr|Q2V0U0|Q2V0U0_BPBZ1 Coat protein OS=Enterobacteria phage JP500 PE=4 SV=1 
MATLRSFVLVDNGGTGDVTVVPVSNANGVAEWLSNNSRSQAYRVTASYRASGADKRKYTIKLEVPKIVTQ
VVNGVELPVSAWKAYASIDLTIPIFAATDDVTVISKSLAGLFKVGNPIADAISSQSGFYA
SEQ ID NO: 68
   Q2V0T7 Q2V0T7_BPBZ1115 enterobacteria phage sd 332940
   >tr|Q2V0T7|Q2V0T7_BPBZ1 Coat protein OS=Enterobacteria phage SD PE=4 SV=1 
MATLRSFVLVDNGGTGNVTVVPVSNANGVAEWLSNNSRSQAYRVTASYRASGADKRKYTIKLEVPKIVTQ
VVNGVELPISAWKAYASIDLTIPIFAATDDVTTISKSLAGLFKVGNPIADAISSQSGFYA
SEQ ID NO: 69
   Q9MBL2 Q9MBL2_BPKU1115 enterobacteria phage ku1 12021
   >tr|Q9MBL2|Q9MBL2_BPFU1 Coat protein OS=Enterobacteria phage KU1 PE=4 SV=1 
MATLRSFVLVDNGGTGNVTVVPVSNANGVAEWLSNNSRSQAYRVTASYRASGADKRKYTIKLEVPKIVTQ
SVNGVELPVSAWKAFASIDLTIPIFAATDDVTLISKSLAGLFKIGNPVADAISSQSGFYA
SEQ ID NO: 70
   P07234 COAT_BPGA115 enterobacteria phage ga 12018
   >sp|P07234|COAT_BPGA Coat protein OS=Enterobacteria phage GA PE=1 SV=3 
MATLRSFVLVDNGGTGNVTVVPVSNANGVAEWLSNNSRSQAYRVTASYRASGADKRKYAIKLEVPKIVTQ
VVNGVELPGSAWKAYASIDLTIPIFAATDDVTVISKSLAGLFKVGNPIAEAISSQSGFYA
SEQ ID NO: 71
   C8YJG7 C8YJG7_BPBZ1115 enterobacteria phage bz13 329853
   >tr|C8YJG7|C8YJG7_BPBZ1 Capsid protein OS=Enterobacteria phage BZ13 PE=4 SV=1 
MATLRSFVLVDNGGTGNVTVVPVSNANGVAEWLSNNSRSQAYRVTASYRASGADKRKYTIKLEVPKIVTQ
VVNGVELPVSAWKAYASIDLTIPIFAATDDVTVISKSLAGLFKVGNPIAEAISSQSGFYA
SEQ ID NO: 72
   C8YJH1 C8YJH1_BPBZ1115 enterobacteria phage bz13 329853
   >tr|C8YJH1|C8YJH1_BPBZ1 Capsid protein OS=Enterobacteria phage BZ13 PE=4 SV=1 
MATLRSFVLVDNGGTGNVTVVPVSNANGVAEWLSNNSRSQAYRVTASYRASGADKRKYTIKLEVPKIVTQ
TVNGVELPVSAWKAYASIDLTIPIFAATDDVTLISKSLAGLFKIGNPVADAISSQSGFYA
SEQ ID NO: 73
   C8YJH5 C8YJH5_BPBZ1115 enterobacteria phage bz13 329853
   >tr|C8YJH5|C8YJH5_BPBZ1 Capsid protein OS=Enterobacteria phage BZ13 PE=4 SV=1 
MATLRSFVLVDNGGTGNVTVVPVSNANGVAEWLSNNSRSQAYRVTASYRASGADKRKYTIKLEVPKIVTQ
VVNGVELPVSAWKAYASIDLTIPIFAATDDVTVISKSLAGLFKVGDPIADAISSQSGFYA
SEQ ID NO: 74
   Q2V0T4 Q2V0T4_BPTH1115 enterobacteria phage th1 12029
   >tr|Q2V0T4|Q2V0T4_BPTH1 Coat protein OS=Enterobacteria phage TH1 PE=4 SV=1 
MATLRSFVLVDNGGTGNVTVVPVSNANGVAEWLSNNSRSQAYRVTASYRASGADKRKYTIKLEVPKIVTQ
VVNGVELPVSAWKAYASIDLTIPIFAATDDVTVISKSLAGLFKVGNPIADAISSQSGFYA
SEQ ID NO: 75
   Q2V0U3 Q2V0U3_BPBZ1116 enterobacteria phage t12 332938
   >tr|Q2V0U3|Q2V0U3_BPBZ1 Coat protein OS=Enterobacteria phage TL2 PE=4 SV=1 
MATLRSFVLVDNGGTGNVTVVPVSNANGVAEWLSNNSRSQAYRVTASYRASGADKRKYTIKLEVPKIVTQ
VVNGVELPVSAWKAYASIDLTIPIFAATDDVTVISKSLAGLFKVGNPIADAISSQSGFYA
SEQ ID NO: 76
   P03614 COAT_BPFR116 enterobacteria phage fr 12017
   >sp|P03614|COAT_BPFR Coat protein OS=Enterobacteria phage fr PE=1 SV=4 
MASNFEEFVLVDNGGTGDVKVAPSNFANGVAEWISSNSRSQAYKVTCSVRQSSANNRKYTVKVEVPKVAT
QVQGGVELPVAAWRSYMNMELTIPVFATNDDCALIVKALQGTFKTGNPIATAIAANSGIY
SEQ ID NO: 77
   ef108465 enterobacteria phage r17 329852 gi|132424616|gb1AB033465.1| coat protein [Enterobacteria phage MS2] 
MASNFTQFVLVDNGGTGDVTVAPSNFANGVAEWISSNSRSQAYKVTCSVRQSSAQNRKYTIKVEVPKVAT
QTVGGVELPVAAWRSYLNMELTIPIFATNSDCELIVKAMQGLLKDGNPIPSAIAANSGIY
SEQ ID NO: 78 1QBE
   >sp|P03615|COAT_BPQBE Coat protein OS=Enterobacteria phage Qbeta PE=1 SV=2

AKLETVTLGNIGKDGKQTLVLNPRGVNPTNGVASLSQAGAVPALEKRVTVSVSQPSRNRKN
YKVQVKIQNPTACTANGSCDPSVTRQAYADVTFSFTQYSTDEERAFVRTELAALLASPLLI

### DAIDQLNPAY

Further, amino acid residues are distinguished by the identity of their sidechains. They share a common backbone and a common set of allowed backbone conformations (Kleywegt & Jones, Structure 4 1395-1400 (1996)), with two exceptions. Glycines can stably fold into backbone conformations disallowed to other amino acids because its sidechain consists of a single hydrogen atom. The proline sidechain is cyclized into a stiff ring which is covalently bound to its backbone nitrogen through elimination of its amide hydrogen, constraining proline to a small subset of backbone conformations with respect to the other amino acids and eliminating its ability to be a hydrogen bond donor.

The domain fold and domain association for assembly into capsids (for example of the amino sequence of SEQ ID NO: 3 is stabilized by the backbone hydrogen bonding patterns that define its secondary structural units, hydrogen bonds between sidechain and backbone atoms that stabilize local structure or bind neighboring secondary structure units (*e.g.* helices, strands, coil, loops, turns and flexible termini) together, hydrogen bonds between the atoms of different sidechains that stabilize local structure or bind neighboring secondary structure units (*e.g*. helices, strands, coil, loops, turns and flexible termini) together and the close packing of hydrophobic sidechain atoms that serves to both energetically stabilize the fold through van der Waals interactions and to prevent solvent penetration into the fold which might lead to destabilization and local unfolding. The sidechains of the remaining residues do not participate in domain fold maintenance or in domain-domain interactions. So long as their backbone conformations do not have special requirements satisfied only by Gly or cis-Pro in order to participate in the domain fold, these residues can be mutated, singly or as a group, without substantially affecting the final domain fold or the overall topology of its surface, and can be identified as a class unequivocally by surface accessibility calculations performed on known structures (*See, e.g.,* Fraczkiewicz & Braun, JMB; Meth Enzym; J Comp Chem 19, 319 (1998)), followed by hydrogen bond analysis of known structures, all conventional techniques in the study of protein structure and function.

Using two MS2 capsid structures from the Protein Data Bank for examination, 1AQ3 of an isosahedral capsid containing RNA & 2VTU of a stable octahedral capsid formed by 2 MS2 capsid protein monomers fused C-terminus to N-terminus to form a single chain protein 2 domain protein, 17 residues (Ala1, Ser2, Thr5, Gln6, Ala21, Ala53, Val67, Thr69, Thr71, Val72, Val75, Ser99, Glu102, Lys113, Asp114, Gly115, Tyr129) were identified which have highly solvated sidechain positions (Fraczkiewicz & Braun server http://curie.utmb.edu/getarea with 1.4A solvent probe, no gradient, 2 area/energy per residue); do not participate in hydrogen bonds with other parts of the capsid (hydrogen bonds calculated in the widely used freeware software visualization package Chimera (ERIC F. PETTERSEN, THOMAS D. GODDARD, CONRAD C. HUANG, GREGORY S. COUCH, DANIEL M. GREENBLATT, ELAINE C. MENG, THOMAS E. FERRIN (2004 J Comp Chem 25, 1605-1612) with hydrogen bond criteria relaxed by 0.5A and 30 deg); and which backbone conformations allowed by all amino acid residues except proline. When the subset of these 17 residues is compared to the structural alignment of the enterobacteria phage MS2, wherein GA and FR capsid sequences and residues which have mutated in the enterobacteria phage GA or FR capsid sequences are disregarded, leaving 6 positions remaining which are putatively susceptible to mutation without effecting the structure or function of the monomers or their ability to assemble into stable capsids. This represents 52% sequence identity to wild-type enterobacteria phage MS2 capsid protein (SEQ ID NO:3).

The insertion and/or deletion of residues within secondary structure elements (helices, strands, turns with defining hydrogen bonding patterns and structured loops, e.g. omega loops) cause those elements to lose their defining hydrogen bonding or hydrophobic packing patterns or force a change in their hydrogen bonding or hydrophobic packing patterns which can alter stability, shape and/or function from the original protein sequence. This can disrupt packing and effect the global stability of a fold. On the other hand, unstructured loops, random coils and N- & C-termini which have surface exposure but do not provide critical stabilization to the rest of the protein fold (frequently via the packing of sidechains against structured elements or the shielding of interacting faces of adjacent structured elements from solvent or in the case of capsids, cargo) are excellent candidates for (1) residue deletion if significant repositioning of the joined structured elements is not required,(2) insertion of amino acid residues if the addition of residues will not significantly alter the relative disposition of structured elements in the fold or screen surface exposed residues from satisfying their hydrogen bonding capacity with hydrogen bond donors or acceptors in the protein's environment or (3) the incorporation of naturally-occurring amino acid mutation(s) or mutation(s) to nonnative residues which can be covalently linked to useful moieties, *e.g*. fluorophores, phosphorescent groups, polyethylene glycols, affinity tags, reporter groups, etc. Of course, such insertions, deletions and mutations can occur within a single suitable element concurrently or in any combination and their incorporation may give rise to a protein with improved characteristics. A straightforward way to distinguish optimal spots for insertion and/or deletions is to scan the multiple alignments of closely related sequences for insertions and/or deletions. Aside from N- and C-terminal additions and deletions, the known leviviridae coat protein sequences do not have insertion or deletions with respect to each other. This does not mean insertion and/or deletions cannot occur. One simply must examine more distant members of the structure/function or fold family.

The simplest multiple alignment algorithms are usually available to the general public at the public domain sequence and structure data bases. These algorithms can correctly align sequences that share a very low %identity if the sequence space is populated by a continuous spectrum of sequences from a high % identity, for example 90%, to a low %identity, for example 20%. These algorithms tend to fail to correctly align clusters of sequences with the same fold when those cluster share a low %identity; however, such clusters can be successfully and unequivocally aligned if the x-ray crystal structure of one or more members of each cluster has been solved and well refined. By optimally superimposing backbone atoms of the secondary structure elements of the structures of proteins closely related by fold but distantly related by sequence, a one-to-one correspondence between their sequences is clearly defined and the high %identity clusters successfully generated by straightforward sequence alignment protocols can be anchored to the pairwise alignment resulting from the backbone superposition and a correct global sequence alignment for the fold family generated resulting in a topologically meaningful alignment of the fold family members (Arthur M Lesk, Michael Levitt, Cyrus Chothia (1986), Prot Eng 1, 77-78). By examining the global sequence alignment, a comprehensive picture of where the fold will tolerate insertion and/or deletion without compromising its form or function can be viewed.

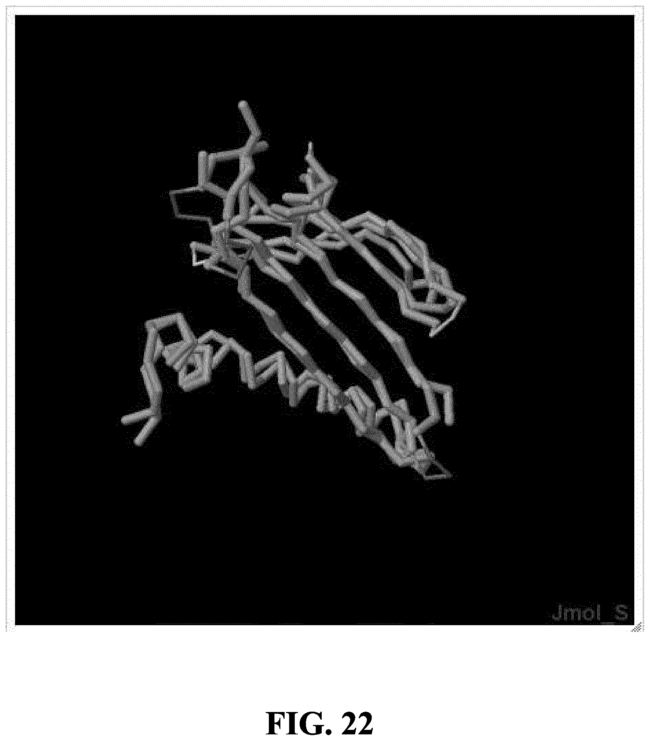

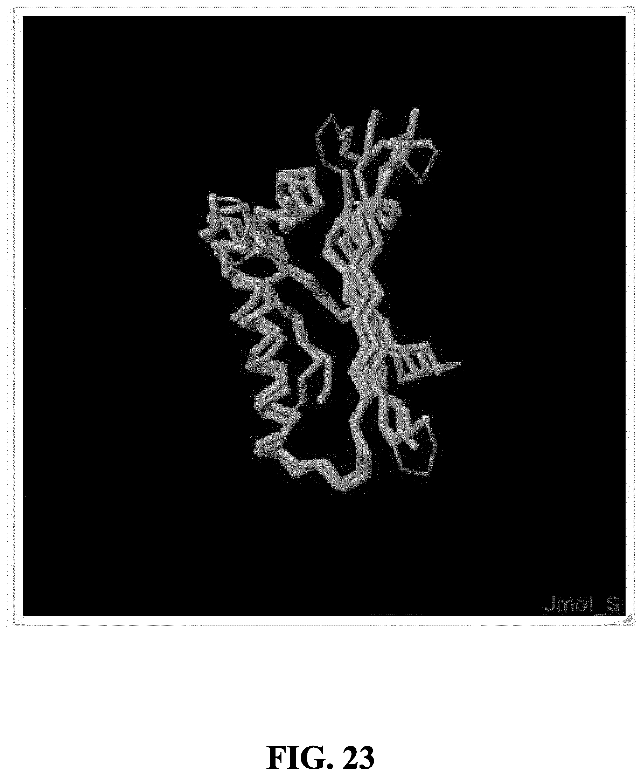

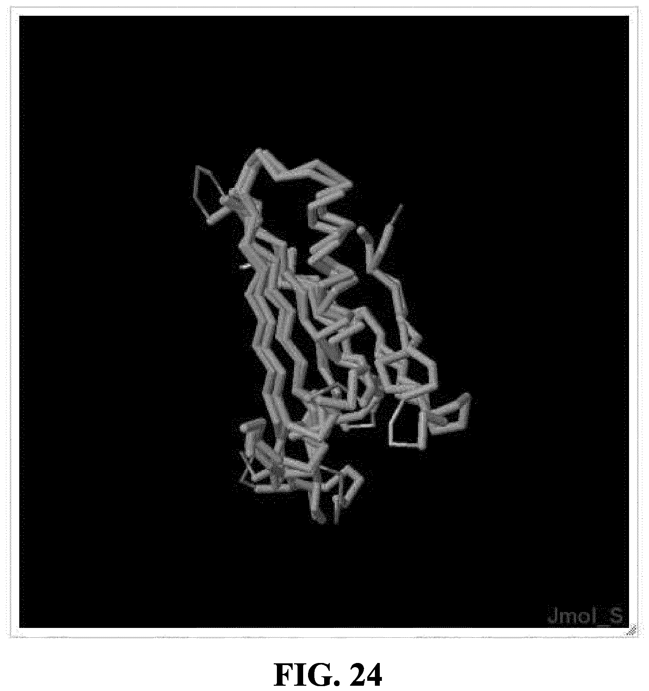

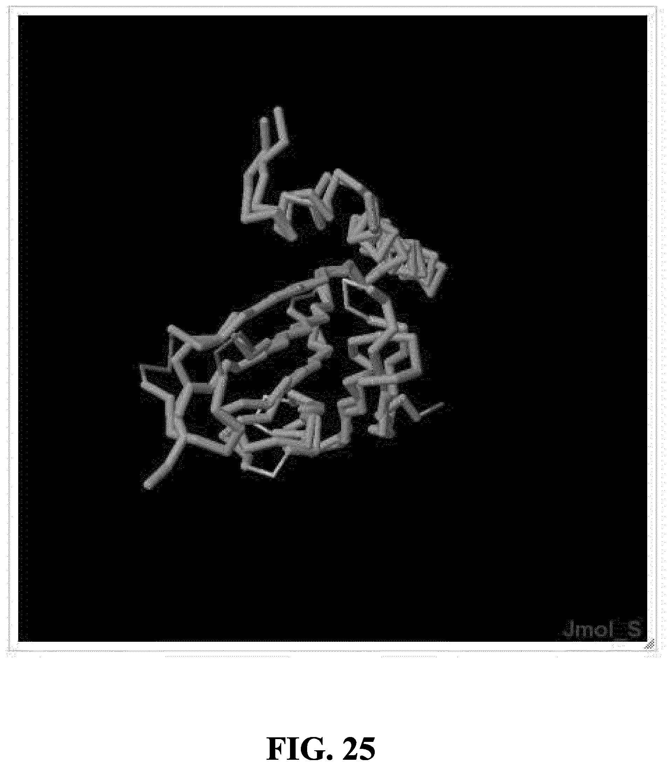

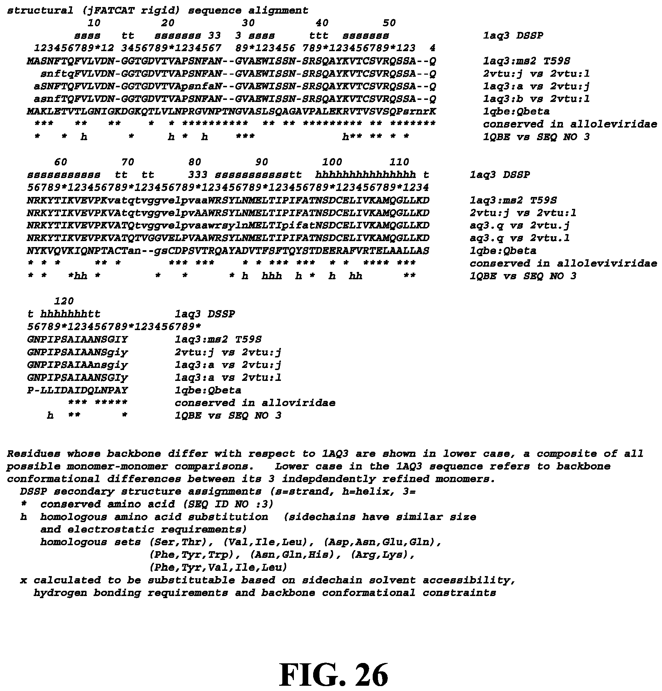

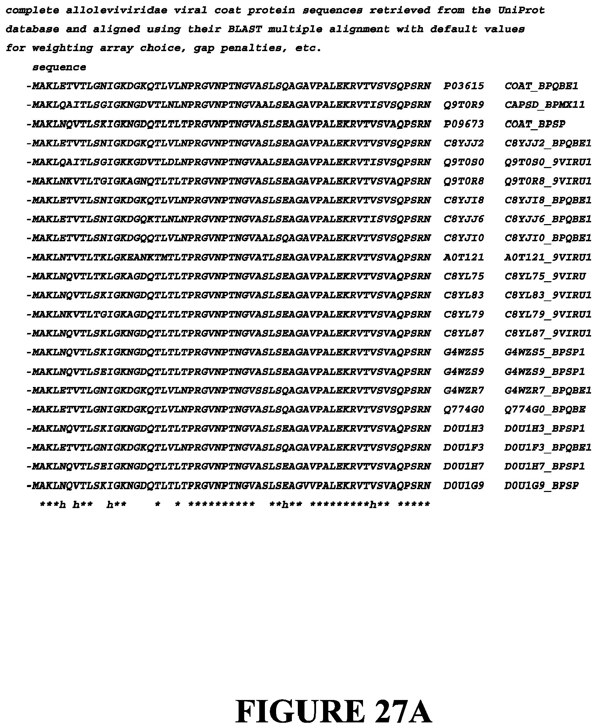

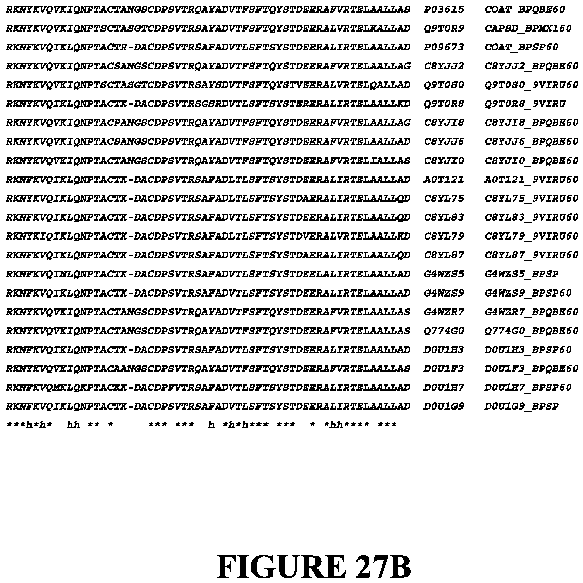

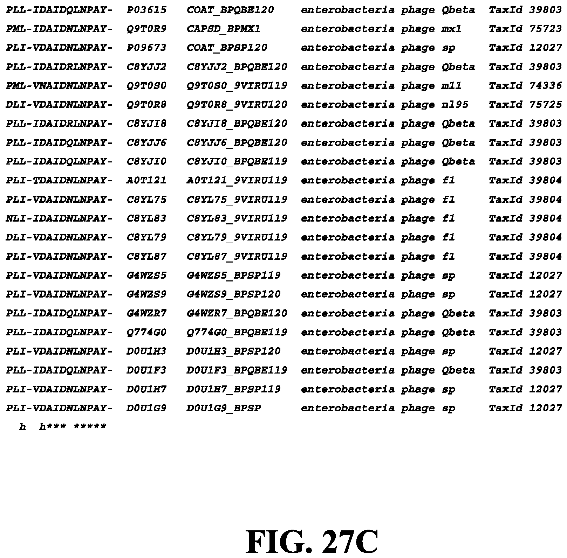

The alloleviviridae coat proteins belong to the same fold family as the leviviridae coat proteins (fold family d.85.1) and also assemble into isosahedral capsids comprised of 180 monomers. The multiple alignments of the sequences of alloleviviridae coat proteins deposited in UniProt are shown in the alignment table in Figure 27. Sixty percent (60 %) of the alloleviviridae coat protein sequence is conserved. The coat proteins of levi- and alloleviviridae are both about 130 amino acid residues long but because the percent of identical residues is low, about 20%, multiple sequence alignment algorithms typically fail to correctly alignment the allolevi- against the leviviridae sequences. A simple way to recognize this is to reverse the sequences and then use the same protocol to align the reversed sequences. The multiple alignments of the sequences and reversed sequences will not agree. This difficulty can be circumvented by examining representative structures. An x-ray crystal structure of a capsid of allolevividae Qbeta (PDB-ID:1QBE) (SEQ ID NO: 78. see below) has been deposited in the public domain database, RCSB Protein Data Bank (http://www.rcsb.org). The independently refined monomers of 1QBE were fit to the independently refined monomers of 1AQ3 by minimizing the rms deviation between {Calpha} atoms using the jFATCAT comparison tool at the RCSB Protein Data Bank. The rms deviation is in the range 2.33-2.76 Angstroms depending upon which of the independently refined monomers is compared, primarily due to differences in the backbone disposition of N-terminal residues 1-3 and segments 8-18, 26-28, 50-55 and 67-76 (numbering references the topologically equivalent residues in the MS2 structure 1AQ3) which connect secondary structure elements, as shown in Figures 22-25 and described in the accompanying figure descriptions. The backbone rms deviation measured by jFATCAT for independently refined monomers in 1AQ3 is 1.72A due to conformational differences in the same regions. The topological alignment is shown in the table, secondary structure assignment by hydrogen bonding pattern (DSSP, W Wolfgang Kabsch & Christian Sander (1983), Biopolymers 22, 2577-2636) is indicated for 1AQ3 and segments that show the greatest deviation either because the refined backbone conformations are substantially different are because the segments were too mobile to be localized in electron density during refinement are provided in lower case. Regions which show backbone flexibility in the crystal environment are also excellent candidates for insertion/and or deletion as if the interactions between these residues and the rest of the fold was important for fold stabilization, their electron density would be localized. Appending the same information for 2VTU provides further insight into segments best adapted to accommodate change. These comparisons are captured symbolically in Figure 26 which shows alignment of 1AQ3 vs 2VTU vs 1QBE.

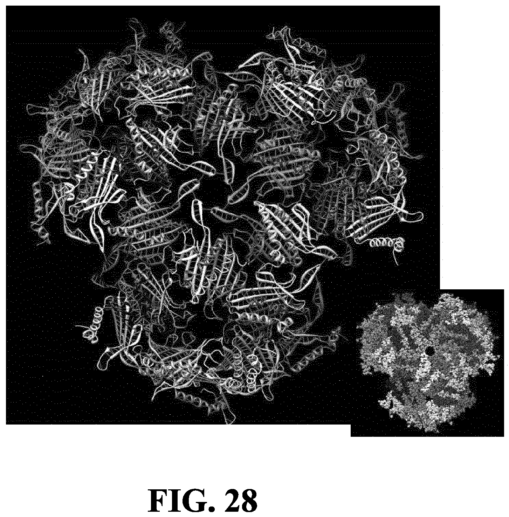

Examination of the 1AQ3 and 1QBE monomers provides the following insights, as further illustrated by reference to Figures 28-31 and their respective descriptions. All residue numbers are given with respect to the monomers in 1AQ3.

This also means that the fold of SEQ ID #3 Enterobacteria phage MS2 coat protein is preserved down to 21% identity vs the sequence of 1QBE Enterobacteria phage coat protein Qbeta and 16% identity with respect to the conserved residues for all of the alloleviviridae coat protein sequences referenced here. Only one of the highly solvated sidechain positions calculated earlier, sidechains which do not participate in hydrogen bonds with other parts of the capsid and whose backbone conformations are allowed by all amino acid residues except proline, remains conserved, Y129 (in SEQ NO 3 numbering). Its backbone position and sidechain packing is substantially changed in the octahedral Enterobacteria phage MS2 capsid structure formed by the fused MS2 dimer (2VTU). Taking this last change into account brings the threshold amino acid sequence percent identity to 15%. See the alignment tables in Figure 26 and Figure 27 (1AQ3 vs 2VTU vs 1QBE, & allolevi multiple sequence alignment tables for clarification). All percent similarities in this paragraph are valid only in the context of structure anchored alignments.

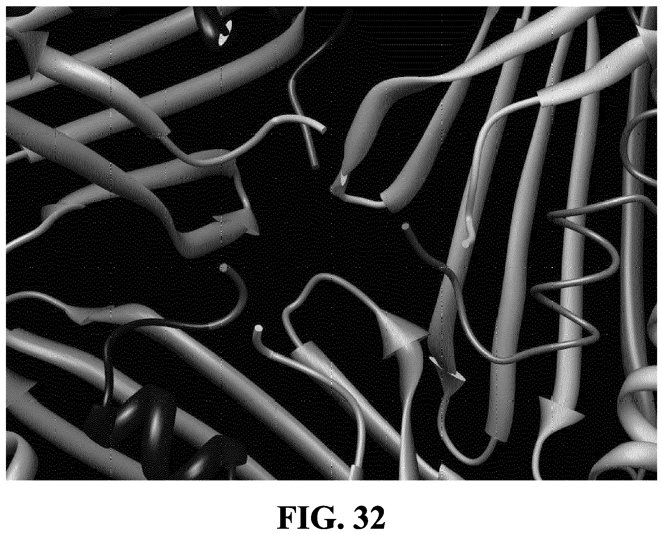

N-terminal residues 1-3 can satisfy their hydrogen bonding potential with the C-terminal residue 129 and water and vice versa; therefore, it should be possible to delete some or all of these residues and form stable VLPs with the truncated proteins. Figure 32 shows backbone ribbon diagrams of 3 noncovalent Enterobacteria phage MS2 noncovalent dimers packed around a symmetry point in the assembled capsid. All of the N-termini are colored green, the C-termini red. The proximity of the termini mean that that the sequences of the monomers can be fused into a single chain to form a covalent dimer, either as done for 2VTU by appending one monomer after the other, *i.e.,* creating a single protein chain that consists of (monomer residues 1-129 - monomer residues 1-129) or by adding additional linking residues between the monomer sequences (monomer 1-129 - linker residues - monomer 1-129) as long as the relative chain directions (from N- to C-terminus) allow a continuous peptide chain to be formed from the concatenated monomers. A monomer-monomer concatenation without the addition of linker residues was solved (PDB-ID:2VTU). In 2VTU each noncovalent dimer has been engineered into a single protein; however, since the Calpha's of residues 2 & 129 are around 6 Angstroms apart, barely close enough to join with a linking segment without disturbing the fold (The Calpha-Calpha distance is constrained to about 3.8 Angstroms because of the resonance forms of the peptide unit.) and in some monomers their backbones hydrogen bond with each other. The beta-sheet side of each dimer (covalent or noncovalent) forms the interior wall of the capsid. The geometry of a beta sheet can be defined by the curvature of the sheet (Cyrus Chothia, Jiri Novotny, Robert Bruccoleri, Martin Karplus (1985) J Mol Biol 186, 651-663). The tight coupling in 2VTU constrains the beta sheet to a lower curvature giving rise to an octahedral rather than an isosahedral capsid. The incorporation of a linker between monomers of 0-6 residues would provide enough flexibility to allow the covalent dimer to relax into the same required for an isosahedral capsid, with physical properties likely to be more closely related to the isosahedral noncovalent capsid structure. Generally, the linker will be 1-6 residues, However, for example, the covalent dimer of 2VTU actually has Ser2 deleted in the second copy. In such cases, the linker length can be 0.

Residues chosen for the linker should have small sidechains to avoid steric strain which can be caused by a large number of atoms packing into a relatively small volume. Strain can also be minimized by avoiding the choice of amino acid residues with smaller backbone conformational space, for example Pro. Avoiding strain can translate into a protein which folds more quickly or more efficiently. Bulkier and charged sidechains, particularly in the middle section of longer loops tend to be binding targets for proteases. Gly-containing linkers are preferred.

From Figure 32 it is also clear that the C-terminus of one monomer can be linked to the N-terminus of a monomer participating in the neighboring noncovalent dimer and a stable isosahedral capsid could still form as long as the linker was of appropriate length and flexibility and did not contain a potential cleavage site accessible by proteases in the capsid environment. In fact, three monomers could be linked with appropriate linkers and still form this section of capsid. Because the tan, gray and medium blue monomers of Figure 32, are also the asymmetric unit of the capsid. Three monomers concatenated end to end with appropriate linking segments should also be able to form a stable isosahedral capsid.

N-terminal residues 1-3 can satisfy their hydrogen bonding potential with the C-terminal residue 129 and water and vice versa; therefore, it should be possible to delete some or all of these residues and form stable VLPs with the truncated proteins or alternatively with the corresponding potential linker lengths extended by the number of deletions in concatenated proteins.

Accordingly, the present disclosure encompasses VLPs comprising a capsid comprising a capsid protein which is a variant of wild type Enterobacteria phage MS2 capsid (SEQ ID NO :3) and is resistant to hydrolysis catalyzed by a peptide bond hydrolase category EC 3.4. For example, a VLP may comprise a capsid protein with the amino acid sequence of wild type Enterobacteria phage MS2 capsid (SEQ ID NO :3) except that the A residue at position 1 is deleted. A VLP may comprise a capsid protein with the amino acid sequence of wild type Enterobacteria phage MS2 capsid (SEQ ID NO :3) except that the A residue at position 1 is deleted and the S residue at position 2 is deleted. A VLP may comprise a capsid protein with the amino acid sequence of wild type Enterobacteria phage MS2 capsid (SEQ ID NO :3) except that that the A residue at position 1 is deleted, the S residue at position 2 is deleted and the N residue at position 3 is deleted. A VLP may comprise a capsid protein with the amino acid sequence of wild type Enterobacteria phage MS2 capsid (SEQ ID NO :3) except that the Y reside at position 129 is deleted. A VLP may comprise a capsid protein with the amino acid sequence of wild type Enterobacteria phage MS2 capsid (SEQ ID NO :3) but having a single (1) amino acid deletion in the 112-117 segment. A VLP may comprise a capsid protein with the amino acid sequence of wild type Enterobacteria phage MS2 capsid (SEQ ID NO :3) but having a single (1) amino acid deletion in the 112-117 segment. A VLP may comprise a capsid protein with the amino acid sequence of wild type Enterobacteria phage MS2 capsid (SEQ ID NO :3) but having a 1-2 residue insertion in the 65-83 segment and is resistant to hydrolysis catalyzed by a peptide bond hydrolase category EC 3.4. A VLP may comprise a capsid protein with the amino acid sequence of wild type Enterobacteria phage MS2 capsid (SEQ ID NO :3) but NO:3) having a 1-2 residue insertion in the 44-55 segment. A VLP may comprise a capsid protein with the amino acid sequence of wild type Enterobacteria phage MS2 capsid (SEQ ID NO :3) but having a single (1) residue insertion in the 33-43 segment and is resistant to hydrolysis catalyzed by a peptide bond hydrolase category EC 3.4. A VLP may comprise a capsid protein with the amino acid sequence of wild type Enterobacteria phage MS2 capsid (SEQ ID NO :3) but having a 1-2 residue insertion in the 24-30 segment. A VLP may comprise a capsid protein with the amino acid sequence of wild type Enterobacteria phage MS2 capsid (SEQ ID NO :3) but having a single (1) residue insertion in the 10-18 segment. A VLP may comprise a capsid protein monomer sequence concatenated with a second capsid monomer sequence which assembles into a capsid which resistant to hydrolysis catalyzed by a peptide bond hydrolase category EC 3.4. A VLP may comprise a capsid protein monomer sequence whose C-terminus is extended with a 0-6 residue linker segment whose C-terminus is concatenated with a second capsid monomer sequence, all of which which assembles into a capsid which resistant to hydrolysis catalyzed by a peptide bond hydrolase category EC 3.4. Suitable linker sequences include but are not limited to -(Gly)x-, where x is 0-6, or a Gly-Ser linker such as but not limited to -Gly-Gly-Ser-Gly-Gly-, - Gly-Gly-Ser and -Gly-Ser-Gly-. A VLP may further comprise a capsid protein monomer sequence concatenated with a third capsid monomer sequence which assembles into a capsid which resistant to hydrolysis catalyzed by a peptide bond hydrolase category EC 3.4. Again, in the capsid protein, the C-terminus can be extended with a 0-6 residue linker segment whose C-terminus is concatenated with a third capsid monomer sequence, all of which which assembles into a capsid which resistant to hydrolysis catalyzed by a peptide bond hydrolase category EC 3.4. One or both linker sequences can be selected from -(Gly)x-, where x=0-6, or a Gly-Ser linker selected from -Gly-Gly-Ser-Gly-Gly-, -Gly-Gly-Ser and -Gly-Ser-Gly-. For example, in one or both linker sequences, the linker is -(Gly)x-, and x is 1, 2 or 3. A VLP may comprise one or more coat protein sequences which are N-terminally truncated by 1-3 residues, wherein a linker sequence is lengthened by the number of residues deleted, wherein the linker sequence is-(Gly)x-, wherein x=0-6. For example, a VLP may comprise one or more coat protein sequences which is C-terminally truncated by 1 residue and then a linker sequence is lengthened by the 1 residue, wherein the linker sequence is -(Gly)x-, wherein x=0-6. A VLP may comprise two coat protein sequences, wherein the first coat protein sequence in a concatenated dimer is C-terminally truncated by 1 residue and a linker sequence is lengthened by the one residue or wherein the first and/or second coat protein sequence in the concatenated trimer is C-terminally truncated by 1 residues, wherein the linker sequence is -(Gly)x-, wherein x=0-6.

All patents and publications cited herein are herein incorporated by reference in their entireties, including those listed below.

### REFERENCES

1. Shenton, W. et al. (2001) Synthesis of Nanophase Iron Oxide in Lumazine Synthase Capsids. AngewandteChemie. 113:456-459
2. Wörsdöerfer, B. et al. (2011) Directed Evolution of a Protein Container. Science. 331:589-592
3. Webb, E. C. (1992). Enzyme nomenclature 1992: Recommendations of the Nomenclature Committee of the International Union of Biochemistry and Molecular Biology on the nomenclature and classification of enzymes. Published for the International Union of Biochemistry and Molecular Biology by Academic Press (San Diego). ISBN 0-12-227164-5
4. Li, H. et al. (1991) Fat fractal and multifractals for protein and enzyme surfaces. Int. J. Biol. Macromol. 13:210-216
5. Varadwaj, P. (2005) Surface Roughness Index, a novel approach to compare protein surfaces. Proceedings of International Conference on Intelligent Sensing and Information Processing. 474-478
6. Burster, T. et al. (2007) Design of protease-resistant myelin basic protein-derived peptides by cleavage site directed amino acid substitutions. Biochemical Pharmacology. 74:1514-1523
7. Matlin, K. S. et al. (1982) Pathway of Vesicular Stomatitis Virus Entry Leading to Infection. J. Mol. Biol. 156:609-631
8. Petruzziello, R. et al. (1996) Pathway of rubella virus infectious entry into Vero cells. J. of General Virology 77:303-308
9. Agbottah, E. et al. (2005) Antiviral Activity of CYC202 in HIV-1-infected Cells. J. Biol. Chem. 280:3029-3042
10. Walker, S.C. et al. (2000) Proteolytic inactivation of simian-11 rotavirus: a pilot study. Veterinary microbiology. 74:195-206
11. Clark, S.M. et al. (1981) Trypsin Enhancement of Rotavirus Infectivity: Mechanism of Enhancement. Journal of Virology. 39:816-822
12. Schmid, F.X. et al. (1998) Selecting proteins with improved stability by a phage-based method. Nature Biotechnology. 16:955-960
13. Gailus, V. et al. (1994) The adsorption protein of bacteriophage fd and its neighbour minor coat protein build a structural entity. Eur. J. Biochem. 222:927-931
14. Schwind, P. et al. (1992) Subtilisin removes the surface layer of the phage fd coat. Eur. J. Biochem. 210:431-436
15. Pickett, G. G. et al. (1993) Encapsidation of heterologous RNAs by bacteriophage MS2 coat protein. Nucleic Acids Research. 19:4621-4626
16. Wilson, T. M. A. et al. (1995) RNA Packaging System. U.S. Patent 5,443,969
17. Fiedler, J. et al. (2010) RNA-Directed Packaging of Enzymes within Virus-like Particles. Angew. Chem. Int. Ed. 49: 9648 -9651
18. Fischlechner, M. et al. (2007) Viruses as Building Blocks for Materials and Devices. Angew. Chem. Int. Ed. 46:3184-3193
19. Jegerlehner, A. et al. (2007) TLR9 Signaling in B Cells Determines Class Switch Recombination to IgG2a. J. Immunol. 178:2415-2420
20. Bachmann et al. US 2003/0099668 A1. Packaging of Immunostimulatory Substances into Virus-like Particles: Methods of Preparation and Use.
21. Nelson, A. L. et al. (2010) Development trends for human monoclonal antibody therapeutics. Nature Reviews - Drug Discovery. 9:767-774
22. Walton, S. P. et al. (2010) Designing highly active siRNAs for therapeutic applications. FEBS Journal. 277:4806-4813
23. Haussecker, D. (2008) The Business of RNAi Therapeutics. Human Gene Therapy. 19:451-462
24. Low, D. et al. (2007) Future of antibody purification. Journal of Chromatography B. 848:48-63
25. Gottschalk, U. (2008) Bioseparation in Antibody Manufacturing: The Good, The Bad and The Ugly. Biotechnol. Prog. 24:496-503
26. Mazzola, P. G. et al. (2008) Liquid-liquid extraction of biomolecules: an overview and update of the main techniques. J. Chem. Technol. Biotechnol. 83:143-157
27. Przybycien, T. M. et al. (2004) Alternative bioseparation operations: life beyond packed-bed chromatography. Current Opinion in Biotechnology. 15:469-478
28. Kelley, B. (2007) Very Large Scale Monoclonal Antibody Purification: The Case for Conventional Unit Operations. Biotechnol. Prog. 23:995-1008
29. Micura, R. (2002) Small Interfering RNAs and Their Chemical Synthesis. Angew. Chem. Int. Ed. 41:2265-2269
30. Pattenden, L. K. et al. (2005) Towards the preparative and large-scale precision manufacture of virus-like particles. TRENDS in Biotechnology. 23:523-529
31. Simon, L. D. et al. (1983) Stabilization of proteins by a bacteriophage T4 gene cloned in Escherichia coli. Proc. Natl. Acad. Sci. USA. 80:2059-2062
32. Vogels, G. et al. (1992) Combination of enzymatic and/or thermal pretreatment with mechanical cell disintegration. Chemical Engineering Science. 47:123-131
33. Xie, J. et al. (2006) A chemical toolkit for proteins -an expanded genetic code. Nature Reviews. Molecular Cell Biology. 7:775-782
34. Lesur, A. et al. (2010) Accelerated tryptic digestion for the analysis of biopharmaceutical monoclonal antibodies in plasma by liquid chromatography with tandem mass spectrometric detection. Journal of Chromatography A. 1217:57-64
35. Aysuso-Tejedor, S. et al. (2010) Underexposed polar residues and protein stabilization. Protein Engineering, Design & Selection. 24:1-7
36. Carr, P. A. et al. (2009) Genome Engineering. Nature Biotechnology. 27:1151-1162
37. Draghi, J. A. et al. (2010) Mutational robustness can facilitate adaptation. Nature. 463:353-355
38. Sanjuán, R. et al. (2010) Viral Mutation Rates. Journal of Virology. 84:9733-9748
39. Van Loo, B. et al. (2004) Directed Evolution of Epoxide Hydrolase from A. radiobacter toward Higher Enantioselectivity by Error-Prone PCR and DNA Shuffling. Chemistry & Biology. 11:981-990
40. Song, H. et al. (2006) Reactions in Droplets in Microfluidic Channels. Angew. Chem. Int. Ed. 45:7336-7356
41. Marsh, E. N. et al. (2009) Fluorine-a new element in the design of membrane-active peptides. Molecular BioSystems. 5:1143-1147
42. Cellitti, S. E. et al. (2008) In vivo Incorporation of Unnatural Amino Acids to Probe Structure, Dynamics, and Ligand Binding in a Large Protein by Nuclear Magnetic Resonance Spectroscopy. J. Am. Chem. Soc. 130:9268-9281
43. Brustad, E. et al. (2008) A Genetically Encoded Boronate-Containing Amino Acid. Angew. Chem. 120:8344-8347
44. Toropova, K. et al. (2008) The Three-dimensional Structure of Genomic RNA in Bacteriophage MS2: Implications for Assembly. J. Mol. Biol. 375:824-836
45. Legendre, D. et al. (2005) Production in Saccharomyces cerevisiae of MS2 virus-like particles packaging functional heterologous mRNAs. Journal of Biotechnology. 117:183-194
46. Valegård, K. et al. (1990) The three-dimensional structure of the bacterial virus MS2. Nature. 345:36-41
47. Kamtekar, S. et al. (1993) Protein Design by Binary Patterning of Polar and Nonpolar Amino Acids. Science. 262:1680-1685
48. Hammill, J. T. et al. (2007) Preparation of site-specifically labeled fluorinated proteins for 19F-NMR structural characterization. Nature Protocols. 2:2601-2607
49. Young et al. (2010) An Enhanced System for Unnatural Amino Acid Mutagenesis in E. coli. J. Mol. Biol. 395:361-374
50. Reis, P. et al. (2009) Lipases at interfaces: A review. Advances in Colloid and Interface Science. 147:237-250
51. Jung, S. et al. (2004) Limited Hydrolysis of Soy Proteins with Endo- and Exoproteases. JAOCS 81:953-960
52. Miyaura, N. et al. (1995) Palladium-Catalyzed Cross-Coupling Reactions of Organoboron Compounds. Chem. Rev. 95:2457-2483
53. Peabody, D. S. et al. (2008) Immunogenic Display of Diverse Peptides on Virus-like Particles of RNA Phage MS2. J. Mol. Biol. 380:252-263
54. Cheng, Y. et al. (2006) Preparation of His-Tagged Armored RNA Phage Particles as a Control for Real-Time Reverse Transcription-PCR Detection of Severe Acute Respiratory Syndrome Coronavirus. Journal of Clinical Microbiology. 44:3557-3561
55. Stockley, P. G. et al. (2007) A Simple, RNA-Mediated Allosteric Switch Controls the Pathway to Formation of a T=3 Viral Capsid. J. Mol. Biol. 369:541-552
56. Kastelein, R. A. et al. (1982) Lysis gene expression of RNA phage MS2 depends on a frameshift during translation of the overlapping coat protein gene. Nature. 295:35-41
57. Wimmer, E. et al. (2009) Synthetic viruses: a new opportunity to understand and prevent viral disease. Nature biotechnology. 27:1163-1172
58. Wei, Y. et al. (2008) RNase-Resistant Virus-Like Particles Containing Long Chimeric RNA Sequences Produced by Two-Plasmid Coexpression System. J. Clin. Microbiol. 46:1734-1740
59. Chang, J.R., A. Poliakov, P.E. Prevelige, J.A. Mobley and T. Dokland, Incorporation of scaffolding protein gpO in bacteriophages P2 and P4. Virology, 2008. 370(2): p. 352-61.
60. Ochoa, W.F., A. Chatterji, T. Lin and J.E. Johnson, Generation and structural analysis of reactive empty particles derived from an icosahedral virus. Chemistry & biology, 2006. 13(7): p. 771-8.
61. Maruyama, I.N., H. Maruyama and S. Brenner, λfoo: A λ phage vector for the expression of foreign proteins. Proc. Natl. Acad. Sci. USA, 1994. 91: p. 8273-8277.
62. Chomczynski, P. and N. Sacchi, The single-step method of RNA isolation by acid guanidinium thiocyanate-phenol-chloroform extraction: twenty-something years on. Nature protocols, 2006. 1(2): p. 581-5.
63. Cheng, Y., J. Niu, Y. Zhang, J. Huang and Q. Li, Preparation of His-tagged armored RNA phage particles as a control for real-time reverse transcription-PCR detection of severe acute respiratory syndrome coronavirus. Journal of clinical microbiology, 2006. 44(10): p. 3557-61.
64. Wan, Y., S. Vasan, R. Ghosh, G. Hale and Z. Cui, Separation of monoclonal antibody alemtuzumab monomer and dimers using ultrafiltration. Biotechnology and bioengineering, 2005. 90(4): p. 422-432.
65. Askonas, B.A., The use of organic solvents at low temperature for the separation of enzymes. Application to aqueous rabbit muscle extract. Biochemical Journal, 1951. 48: p. 42-48.
66. Mazzola, P.G., A.M. Lopes, F.A. Hasmann, A.F. Jozala, T.C.V. Penna, P.O. Magalhaes, C.O. Rangel-Yagui and A. Pessoa Jr, Liquid-liquid extraction of biomolecules: an overview and update of the main techniques. Journal of Chemical Technology & Biotechnology, 2008. 83(2): p. 143-157.
67. Przybycien, T.M., N.S. Pujar and L.M. Steele, Alternative bioseparation operations: life beyond packed-bed chromatography. Current opinion in biotechnology, 2004. 15(5): p. 469-78.
68. Low, D., R. O'Leary and N.S. Pujar, Future of antibody purification. Journal of chromatography. B, Analytical technologies in the biomedical and life sciences, 2007. 848(1): p. 48-63.
69. Adrian R. Ferré-D'Amaré and William G. Scott, Small Self-cleaving Ribozymes, Cold Spring Harb Perspect Biol 2010;2:a003574 originally published online September 15, 2010.
70. Adrian R. Ferré-D'Amaré and Jennifer A. Doudna, Use of cis- and trans-ribozymes to remove 5' and 3'heterogeneities from milligrams of in vitro transcribed RNA, Nucleic Acids Research, 1996, Vol. 24, No. 5, 977-978.
71. Jae H. Lee, Jeffrey A. Engler, James F. Collawn and Bryan A. Moore, Receptor mediated uptake of peptides that bind the human transferrin receptor, Eur. J. Biochem. 268, 2004±2012 (2001).
72. Mark E. Davis, Jonathan E. Zuckerman, Chung Hang J. Choil, David Seligson, Anthony Tolcher, Christopher A. Alabi, Yun Yen, Jeremy D. Heidel & Antoni Ribas, Evidence of RNAi in humans from systemically administered siRNA via targeted nanoparticles, Nature 464, 1067-1070 (15 April 2010).
73. Jeremy S. Paige, Karen Y. Wu, Samie R. Jaffrey, RNA Mimics of Green Fluorescent Protein, Science 333, 642 (2011).
74. Sarah Everts, New Sensor For Cell Metabolites, Chemical & Engineering News, 90(11), March 12, 2012.

## Claims

1. A nucleic acid construct comprising a nucleotide sequence that encodes a short RNA (shRNA), a ribozyme, and a packing sequence, wherein the ribozyme is a mutant Hepatitis Delta V (HDV) ribozyme represented by a nucleic acid sequence selected from SEQ ID NOs: 10-17.

2. A nucleic acid construct according to claim 1, wherein the ribozyme is flanked on one side by the shRNA and on the other side by the packing sequence.

3. A vector comprising the nucleic acid construct of claim 1.

4. A host cell comprising the vector according to claim 3.


SEQUENCE LISTING

<110>  APSE, LLC

<120>  PROCESSES USING VLPS WITH CAPSIDS RESISTANT TO HYDROLASES

<130>  P6051511EP1

<150>  US 61/578,706
<151>  2011-12-21

<150>  US 61/607,900
<151>  2012-03-07

<150>  US 61/661,688
<151>  2012-06-19

<160>  78

<170>  PatentIn version 3.5

<210>  1
<211>  3569
<212>  DNA
<213>  Enterobacteria phage MS2

<400>  1
   gggtgggacc cctttcgggg tcctgctcaa cttcctgtcg agctaatgcc atttttaatg      60
   tctttagcga gacgctacca tggctatcgc tgtaggtagc cggaattcca ttcctaggag     120
   gtttgacctg tgcgagcttt tagtaccctt gatagggaga acgagacctt cgtcccctcc     180
   gttcgcgttt acgcggacgg tgagactgaa gataactcat tctctttaaa atatcgttcg     240
   aactggactc ccggtcgttt taactcgact ggggccaaaa cgaaacagtg gcactacccc     300
   tctccgtatt cacggggggc gttaagtgtc acatcgatag atcaaggtgc ctacaagcga     360
   agtgggtcat cgtggggtcg cccgtacgag gagaaagccg gtttcggctt ctccctcgac     420
   gcacgctcct gctacagcct cttccctgta agccaaaact tgacttacat cgaagtgccg     480
   cagaacgttg cgaaccgggc gtcgaccgaa gtcctgcaaa aggtcaccca gggtaatttt     540
   aaccttggtg ttgctttagc agaggccagg tcgacagcct cacaactcgc gacgcaaacc     600
   attgcgctcg tgaaggcgta cactgccgct cgtcgcggta attggcgcca ggcgctccgc     660
   taccttgccc taaacgaaga tcgaaagttt cgatcaaaac acgtggccgg caggtggttg     720
   gagttgcagt tcggttggtt accactaatg agtgatatcc agggtgcata tgagatgctt     780
   acgaaggttc accttcaaga gtttcttcct atgagagccg tacgtcaggt cggtactaac     840
   atcaagttag atggccgtct gtcgtatcca gctgcaaact tccagacaac gtgcaacata     900
   tcgcgacgta tcgtgatatg gttttacata aacgatgcac gtttggcatg gttgtcgtct     960
   ctaggtatct tgaacccact aggtatagtg tgggaaaagg tgcctttctc attcgttgtc    1020
   gactggctcc tacctgtagg taacatgctc gagggcctta cggcccccgt gggatgctcc    1080
   tacatgtcag gaacagttac tgacgtaata acgggtgagt ccatcataag cgttgacgct 1140
   ccctacgggt ggactgtgga gagacagggc actgctaagg cccaaatctc agccatgcat 1200
   cgaggggtac aatccgtatg gccaacaact ggcgcgtacg taaagtctcc tttctcgatg 1260
   gtccatacct tagatgcgtt agcattaatc aggcaacggc tctctagata gagccctcaa 1320
   ccggagtttg aagcatggct tctaacttta ctcagttcgt tctcgtcgac aatggcggaa 1380
   ctggcgacgt gactgtcgcc ccaagcaact tcgctaacgg ggtcgctgaa tggatcagct 1440
   ctaactcgcg ttcacaggct tacaaagtaa cctgtagcgt tcgtcagagc tctgcgcaga 1500
   atcgcaaata caccatcaaa gtcgaggtgc ctaaagtggc aacccagact gttggtggtg 1560
   tagagcttcc tgtagccgca tggcgttcgt acttaaatat ggaactaacc attccaattt 1620
   tcgctacgaa ttccgactgc gagcttattg ttaaggcaat gcaaggtctc ctaaaagatg 1680
   gaaacccgat tccctcagca atcgcagcaa actccggcat ctactaatag acgccggcca 1740
   ttcaaacatg aggattaccc atgtcgaaga caacaaagaa gttcaactct ttatgtattg 1800
   atcttcctcg cgatctttct ctcgaaattt accaatcaat tgcttctgtc gctactggaa 1860
   gcggtgatcc gcacagtgac gactttacag caattgctta cttaagggac gaattgctca 1920
   caaagcatcc gaccttaggt tctggtaatg acgaggcgac ccgtcgtacc ttagctatcg 1980
   ctaagctacg ggaggcgaat ggtgatcgcg gtcagataaa tagagaaggt ttcttacatg 2040
   acaaatcctt gtcatgggat ccggatgttt tacaaaccag catccgtagc cttattggca 2100
   acctcctctc tggctaccga tcgtcgttgt ttgggcaatg cacgttctcc aacggtgctc 2160
   ctatggggca caagttgcag gatgcagcgc cttacaagaa gttcgctgaa caagcaaccg 2220
   ttaccccccg cgctctgaga gcggctctat tggtccgaga ccaatgtgcg ccgtggatca 2280
   gacacgcggt ccgctataac gagtcatatg aatttaggct cgttgtaggg aacggagtgt 2340
   ttacagttcc gaagaataat aaaatagatc gggctgcctg taaggagcct gatatgaata 2400
   tgtacctcca gaaaggggtc ggtgctttca tcagacgccg gctcaaatcc gttggtatag 2460
   acctgaatga tcaatcgatc aaccagcgtc tggctcagca gggcagcgta gatggttcgc 2520
   ttgcgacgat agacttatcg tctgcatccg attccatctc cgatcgcctg gtgtggagtt 2580
   ttctcccacc agagctatat tcatatctcg atcgtatccg ctcacactac ggaatcgtag 2640
   atggcgagac gatacgatgg gaactatttt ccacaatggg aaatgggttc acatttgagc 2700
   tagagtccat gatattctgg gcaatagtca aagcgaccca aatccatttt ggtaacgccg 2760
   gaaccatagg catctacggg gacgatatta tatgtcccag tgagattgca ccccgtgtgc 2820
   tagaggcact tgcctactac ggttttaaac cgaatcttcg taaaacgttc gtgtccgggc 2880
   tctttcgcga gagctgcggc gcgcactttt accgtggtgt cgatgtcaaa ccgttttaca 2940
   tcaagaaacc tgttgacaat ctcttcgccc tgatgctgat attaaatcgg ctacggggtt 3000
   ggggagttgt cggaggtatg tcagatccac gcctctataa ggtgtgggta cggctctcct 3060
   cccaggtgcc ttcgatgttc ttcggtggga cggacctcgc tgccgactac tacgtagtca 3120
   gcccgcctac ggcagtctcg gtatacacca agactccgta cgggcggctg ctcgcggata 3180
   cccgtacctc gggtttccgt cttgctcgta tcgctcgaga acgcaagttc ttcagcgaaa 3240
   agcacgacag tggtcgctac atagcgtggt tccatactgg aggtgaaatc accgacagca 3300
   tgaagtccgc cggcgtgcgc gttatacgca cttcggagtg gctaacgccg gttcccacat 3360
   tccctcagga gtgtgggcca gcgagctctc ctcggtagct gaccgaggga cccccgtaaa 3420
   cggggtgggt gtgctcgaaa gagcacgggt gcgaaagcgg tccggctcca ccgaaaggtg 3480
   ggcgggcttc ggcccaggga cctcccccta aagagaggac ccgggattct cccgatttgg 3540
   taactagctg cttggctagt taccaccca 3569

<210> 2
<211> 396
<212> DNA
<213> Enterobacteria phage MS2

<400> 2
atggcttcta actttactca gttcgttctc gtcgacaatg gcggaactgg cgacgtgact 60

gtcgccccaa gcaacttcgc taacggggtc gctgaatgga tcagctctaa ctcgcgttca 120

caggcttaca aagtaacctg tagcgttcgt cagagctctg cgcagaatcg caaatacacc 180

atcaaagtcg aggtgcctaa agtggcaacc cagactgttg gtggtgtaga gcttcctgta 240

gccgcatggc gttcgtactt aaatatggaa ctaaccattc caattttcgc tacgaattcc 300

gactgcgagc ttattgttaa ggcaatgcaa ggtctcctaa aagatggaaa cccgattccc 360

tcagcaatcg cagcaaactc cggcatctac taatag 396

<210> 3
<211> 130
<212> PRT
<213> Enterobacteria phage MS2

<400> 3

Met Ala Ser Asn Phe Thr Gln Phe Val Leu Val Asp Asn Gly Gly Thr
1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu

50                  55                  60

Val Pro Lys Val Ala Thr Gln Thr Val Gly Gly Val Glu Leu Pro Val
65                  70                  75                  80

Ala Ala Trp Arg Ser Tyr Leu Asn Met Glu Leu Thr Ile Pro Ile Phe
                85                  90                  95

Ala Thr Asn Ser Asp Cys Glu Leu Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Tyr
    130

<210>  4
<211>  36
<212>  DNA
<213>  Bacteriophage T7

<400>  4
ctagcataac cccttggggc ctctaaacgg gtcttg                                36

<210>  5
<211>  47
<212>  DNA
<213>  Bacteriophage T7

<400>  5
   tagcataacc ccttggggcc tctaaacggg tcttgagggg ttttttg                     47

<210>  6
<211>  480
<212>  DNA
<213>  Artificial Sequence

<220>
<223>  SYNETHESIZED

<400>  6
acaagtttgt acaaaaaagc aggctaagaa ggagatatac atatggcttc taactttact      60

cagttcgttc tcgtcgacaa tggcggaact ggcgacgtga ctgtcgcccc aagcaacttc     120

gctaacgggg tcgctgaatg gatcagctct aactcgcgtt cacaggctta caaagtaacc     180

tgtagcgttc gtcagagctc tgcgcagaat cgcaaataca ccatcaaagt cgaggtgcct     240

aaagtggcaa cccagactgt tggtggtgta gagcttcctg tagccgcatg gcgttcgtac     300

ttaaatatgg aactaaccat tccaattttc gctacgaatt ccgactgcga gcttattgtt     360

aaggcaatgc aaggtctcct aaaagatgga aacccgattc cctcagcaat cgcagcaaac     420

tccggcatct actaatagac gccggccatt caaacatgag gattacccat gtacccagct 480

<210> 7
<211> 438
<212> DNA
<213> Artificial Sequence

<220>
<223> SYNTHESIZED

<400> 7
acaagtttgt acaaaaaagc aggctaagaa ggagatatac atatggcttc taactttact 60

cagttcgttc tcgtcgacaa tggcggaact ggcgacgtga ctgtcgcccc aagcaacttc 120

gctaacgggg tcgctgaatg gatcagctct aactcgcgtt cacaggctta caaagtaacc 180

tgtagcgttc gtcagagctc tgcgcagaat cgcaaataca ccatcaaagt cgaggtgcct 240

aaagtggcaa cccagactgt tggtggtgta gagcttcctg tagccgcatg gcgttcgtac 300

ttaaatatgg aactaaccat tccaattttc gctacgaatt ccgactgcga gcttattgtt 360

aaggcaatgc aaggtctcct aaaagatgga aacccgattc cctcagcaat cgcagcaaac 420

tccggcatct actaatag 438

<210> 8
<211> 179
<212> DNA
<213> Artificial Sequence

<220>
<223> SYNTHESIZED

<400> 8
ggatcctaat acgactcact ataggcaagc tgaccctgaa gttctcaaga ggaacttcag 60

ggtcagcttg ccaaggccgg catggtccca gcctcctcgc tggcgccggc tgggcaacat 120

tcgtggcgaa tgggaccacg cttcaaacat gaggattacc catgtcgaag cgaccatgg 179

<210> 9
<211> 385
<212> DNA
<213> Artificial Sequence

<220>
<223> SYNTHESIZED

<400> 9
ggatcctaat acgactcact atagggagat aaataaataa atttgaatga acttcagggt 60

cagcttgctg atgaggcgct tcggcgccga aacacccagt ggtgtccaag ctgaccctga 120

agttcattca agagatgaac ttcagggtca gcttgtcggc cggcatggtc ccagcctcct 180

cgctggcgcc ggctgggcaa cattcgtggc gaatgggacc acgcttcaaa catgaggatt 240

acccatgtcg aagcgaattt atttatttaa ttattattat tattattggc cggcatggtc 300

ccagcctcct cgctggcgcc ggctgggcaa caccttcggg tggcgaatgg gaccaaaaaa 360

aaataataat aataataatc catgg 385

<210> 10
<211> 165
<212> DNA
<213> Artificial Sequence

<220>
<223> SYNTHESIZED

<400> 10
taatacgact cactatagca agctgaccct gaagttcatc aagagtgaac ttcagggtca 60

gcttgtcggc cggcatggtc ccagcctcct cgctggcgcc ggctgggcaa cattcgtggc 120

taatgggacc atatatatat acatgaggat tacccatgtc catgg 165

<210> 11
<211> 165
<212> DNA
<213> Artificial Sequence

<220>
<223> SYNTHESIZED

<400> 11
taatacgact cactatagca agctgaccct gaagttcatc aagagtgaac ttcagggtca 60

gcttgtcggc cggcatggtc ccagcctcct cgctggcgcc ggctggtcaa cattcgtggc 120

gaatgggacc atatatatat acatgaggat tacccatgtc catgg 165

<210> 12
<211> 165
<212> DNA
<213> Artificial Sequence

<220>
<223> SYNTHESIZED

<400> 12
taatacgact cactatagca agctgaccct gaagttcatc aagagtgaac ttcagggtca 60

gcttgtcggc cggcatggtc ccggcctcct cgctggcgcc ggctgggcaa cattcgtggc 120

gaatgggacc atatatatat acatgaggat tacccatgtc catgg 165

<210> 13
<211> 165
<212> DNA
<213> Artificial Sequence

<220>
<223> SYNTHESIZED

<400>  13
taatacgact cactatagca agctgaccct gaagttcatc aagagtgaac ttcagggtca      60

gcttgtcggc cggcatggtc ccagcctcct cgctggcgcc ggctgtgcaa cattcgtggc     120

gaatgggacc atatatatat acatgaggat tacccatgtc catgg                     165


<210>  14
<211>  165
<212>  DNA
<213>  Artificial Sequence

<220>
<223>  SYNTHESIZED

<400>  14
taatacgact cactatagca agctgaccct gaagttcatc aagagtgaac ttcagggtca      60

gcttgtcggc cggcatggtc ccagcctcct cgctggcgcc ggctgggcaa cattcgtggc     120

gagtgggacc atatatatat acatgaggat tacccatgtc catgg                     165


<210>  15
<211>  165
<212>  DNA
<213>  Artificial Sequence

<220>
<223>  SYNTHESIZED

<400>  15
taatacgact cactatagca agctgaccct gaagttcatc aagagtgaac ttcagggtca      60

gcttgtcggc cggcatggtc ccagccttct cgctggcgcc ggctgggcaa cattcgtggc     120

gaatgggacc atatatatat acatgaggat tacccatgtc catgg                     165


<210>  16
<211>  165
<212>  DNA
<213>  Artificial Sequence

<220>
<223>  SYNTHESIZED

<400>  16
taatacgact cactatagca agctgaccct gaagttcatc aagagtgaac ttcagggtca      60

gcttgtcggc cggcatggtc ccagcctcct cactggcgcc ggctgggcaa cattcgtggc     120

gaatgggacc atatatatat acatgaggat tacccatgtc catgg                     165


<210>  17
<211>  165
<212>  DNA
<213>  Artificial Sequence

<220>
<223>  SYNTHESIZED

<400>  17
taatacgact cactatagca agctgaccct gaagttcatc aagagtgaac ttcagggtca      60

gcttgtcggc cggcatggtc ccagcctcct cgctggcgcc ggctgggcaa cattcgtggc     120

gattgggacc atatatatat acatgaggat tacccatgtc catgg                     165


<210>  18
<211>  165
<212>  DNA
<213>  Artificial Sequence

<220>
<223>  SYNTHESIZED

<400>  18
taatacgact cactatagca agctgaccct gaagttcatc aagagtgaac ttcagggtca      60

gcttgtcggc cggcatggtc ccagcctcct cgctggcgcc ggctgggcaa cattcgtgcc     120

gaatgggacc atatatatat acatgaggat tacccatgtc catgg                     165


<210>  19
<211>  365
<212>  DNA
<213>  Artificial Sequence

<220>
<223>  SYNTHESIZED

<400>  19
ggatcctaat acgactcact atagggagac gttcacgttg aatgaacttc agggtcagct      60

tgctgatgag gcgcttcggc gccgaaacac ccagtggtgt ccaagctgac cctgaagttc     120

attcaagaga tgaacttcag ggtcagcttg tcaccggatg tgctctccgg tctgatgagt     180

ccgtgaggac gaaacaagct gaccctgaag ttcatccgtg aacgacgctt caaacatgag     240

gattacccat gtcgaagcga atatatatat ataggccggc atggtcccag cctcctcgct     300

ggcgccggct gggcaacacc ttcgggtggc gaatgggacc aaaaaaatat atatatatac     360

catgg                                                                 365


<210>  20
<211>  154
<212>  DNA
<213>  Artificial Sequence

<220>
<223>  SYNTHESIZED

<400>  20
taatacgact cactatagca agctgaccct gaagttcatc aagagtgaac ttcagggtca      60

gcttgtcacc ggatgtgctc tccggtctga tgagtccgtg aggacgaaac aagctgacca     120

tatatatata catgaggatt acccatgtcc atgg                                 154

<210>  21
<211>  159
<212>  DNA
<213>  Artificial Sequence

<220>
<223>  SYNTHESIZED

<400>  21
taatacgact cactatagca agctgaccct gaagttcatc aagagtgaac ttcagggtca      60

gcttgtcacc ggatgtgctc tccggtctga tgagtccgtg aggacgaaac aagctgaccc     120

tgaaatatat atatacatga ggattaccca tgtccatgg                            159


<210>  22
<211>  164
<212>  DNA
<213>  Artificial Sequence

<220>
<223>  SYNTHESIZED

<400>  22
taatacgact cactatagca agctgaccct gaagttcatc aagagtgaac ttcagggtca      60

gcttgtcacc ggatgtgctc tccggtctga tgagtccgtg aggacgaaac aagctgaccc     120

tgaagttcaa tatatatata catgaggatt acccatgtcc atgg                      164


<210>  23
<211>  166
<212>  DNA
<213>  Artificial Sequence

<220>
<223>  SYNTHESIZED

<400>  23
taatacgact cactatagca agctgaccct gaagttcatc aagagtgaac ttcagggtca      60

gcttgtcacc ggatgtgctc tccggtctga tgagtccgtg aggacgaaac aagctgaccc     120

tgaagttcac tatatatata tacatgagga ttacccatgt ccatgg                    166


<210>  24
<211>  169
<212>  DNA
<213>  Artificial Sequence

<220>
<223>  SYNTHESIZED

<400>  24
taatacgact cactatagca agctgaccct gaagttcatc aagagtgaac ttcagggtca      60

gcttgtcacc ggatgtgctc tccggtctga tgagtccgtg aggacgaaac aagctgaccc     120

tgaagttcac tcttatatat atatacatga ggattaccca tgtccatgg                169

<210>  25
<211>  171
<212>  DNA
<213>  Artificial Sequence

<220>
<223>  SYNTHESIZED

<400>  25
taatacgact cactatagca agctgaccct gaagttcatc aagagtgaac ttcagggtca      60

gcttgtcacc ggatgtgctc tccggtctga tgagtccgtg aggacgaaac aagctgaccc     120

tgaagttcac tcttgaatat atatatacat gaggattacc catgtccatg g              171

<210>  26
<211>  244
<212>  DNA
<213>  Artificial Sequence

<220>
<223>  SYNTHESIZED

<400>  26
ggatcctaat acgactcact atagggatct cccagcctcc tcgctggcgc cggctgggca      60

acattcgtgg cgaatggggg atcatatctt gatgaacttc agggtcagct tgctgatgag     120

gcgcttcggc gccgaaacac cgtgtccaag ctgaccctga agttcatcaa gaatgaactt     180

cagggtcagc ttgtcggccg gcatgcattc aaacatgagg attacccatg tcgaagttaa     240

ttaa                                                                  244

<210>  27
<211>  615
<212>  DNA
<213>  Artificial Sequence

<220>
<223>  SYNTHESIZED

<400>  27
ggatcctaat acgactcact atagggagac ttgatgaact tcagggtcag cttgctgatg      60

aggcgcttcg gcgccgaaac acccagtggt gtccaagctg accctgaagt tcatcaagaa     120

tgaacttcag ggtcagcttg tcaccggatg tgctctccgg tctgatgagt ccgtgaggac     180

gaaacaagct gaccctgaag ttcattatat cttggcagat gaacttcagg gtcagctgat     240

gagactcttc ggagtcgaaa cacccagtgg tgtcctgacc ctgaagttca tctgccaaga     300

gcagatgaac ttcagggtca gtcaccggat gtgctctccg gtctgatgag tccgtgagga     360

cgaaactgac cctgaagttc atctgctata tcttgtggtg cagatgaact tcagggctga     420

tgaggctctt cggagccgaa acacccagtg gtgtcccctg aagttcatct gcaccacaag     480

atggtgcaga tgaacttcag ggtcaccgga tgtgctctcc ggtctgatga gtccgtgagg 540

acgaaaccct gaagttcatc tgcaccatac gccggccatt caaacatgag gattacccat 600

gtcgaagtta attaa 615

<210> 28
<211> 431
<212> DNA
<213> Artificial Sequence

<220>
<223> SYNTHESIZED

<400> 28
ggatcctaat acgactcact atagggagaa tatatataca agctgaccct gaagttcatc 60

aagaatgaac ttcagggtca gcttgtcggc cggcatggtc ccagcctcct cgctggcgcc 120

ggctgggcaa cattcgtggc gaatgggacc aattaattac tgaccctgaa gttcatctgc 180

caagagcaga tgaacttcag ggtcagtcgg ccggcatggt cccagcctcc tcgctggcgc 240

cggctgggca acattcgtgg cgaatgggac caataataat ccctgaagtt catctgcacc 300

acaagatggt gcagatgaac ttcagggtcg gccggcatgg tcccagcctc ctcgctggcg 360

ccggctgggc aacattcgtg gcgaatggga cccattcaaa catgaggatt acccatgtcg 420

aagttaatta a 431

<210> 29
<211> 362
<212> DNA
<213> Artificial Sequence

<220>
<223> SYNTHESIZED

<400> 29
ggatcctaat acgactcact atagggagaa tgaacttcag ggtcagcttg ctgatgaggc 60

gcttcggcgc cgaaacaccg tgtccaagct gaccctgaag ttcatggccg gcatggtccc 120

agcctcctcg ctggcgccgg ctgggcaaca ttcgtggcga atgggaccat tagccaagct 180

gaccctgaag ttcatctgat gagactccga attcggagtc gaaacacggt aaccgtgtca 240

tgaacttcag ggtcagcttg gcggccggca tggtcccagc ctcctcgctg gcgccggctg 300

ggcaacattc gtggcgaatg ggacccattc aaacatgagg attacccatg tcgaagccat 360

gg 362

<210> 30
<211> 196
<212> DNA
<213> Artificial Sequence

<220>
<223>  SYNTHESIZED

<400>  30
ggatcctaat acgactcact atagggagaa ataataatca agctgaccct gaagttcatc      60

aagaatgaac ttcagggtca gcttgtcaat aataatccgc taccccgacc acatgaacaa     120

gattcatgtg gtcggggtag cggtcaataa taatacgctt caaacatgag gattacccat     180

gtcgaagcga ccatgg                                                     196


<210>  31
<211>  165
<212>  DNA
<213>  Artificial Sequence

<220>
<223>  SYNTHESIZED

<400>  31
taatacgact cactataggc ttgtgatgct tcagccaaat caagagtttg gctgaagcat      60

cacaagcggc cggcatggtc ccagcctcct cgctggcgcc ggctgggcaa cattcgtggc     120

gaatgggacc atatatatat acatgaggat tacccatgtc catgg                     165


<210>  32
<211>  168
<212>  DNA
<213>  Artificial Sequence

<220>
<223>  SYNTHESIZED

<400>  32
taatacgact cactataggc tcgagcaagc ctgatgaggc gcttcggcgc cgaaacaccg      60

tgtcgcttgt gatgcttcag ccaaatcaag agtttggctg aagcatcaca agctcaccgg     120

atgtgctctc cggtctgatg agtccgtgag gacgaaagct tgccatgg                  168


<210>  33
<211>  251
<212>  DNA
<213>  Artificial Sequence

<220>
<223>  SYNTHESIZED

<400>  33
taatacgact cactataggg agaacgccgg ccattcaaat agtaaataat agagggtcag      60

cttgctgatg aggcgcttcg gcgccgaaac accgtgtcca agctgaccct gaagttcatc     120

aagagtgaac ttcagggtca gcttgtcacc ggatgtgctc tccggtctga tgagtccgtg     180

aggacgaaac aagctgaccc tgaagttcac tacgccggcc attcaaacat gaggattacc     240

catgtccatg g                                                          251

<210>  34
<211>  129
<212>  PRT
<213>  Enterobacteria phage MS2

<400>  34

Ala Ser Asn Phe Thr Gln Phe Val Leu Val Asp Asn Gly Gly Thr Gly
1               5                   10                  15

Asp Val Thr Val Ala Pro Ser Asn Phe Ala Asn Gly Val Ala Glu Trp
            20                  25                  30

Ile Ser Ser Asn Ser Arg Ser Gln Ala Tyr Lys Val Thr Cys Ser Val
        35                  40                  45

Arg Gln Ser Ser Ala Gln Asn Arg Lys Tyr Ser Ile Lys Val Glu Val
    50                  55                  60

Pro Lys Val Ala Thr Gln Thr Val Gly Gly Val Glu Leu Pro Val Ala
65                  70                  75                  80

Ala Trp Arg Ser Tyr Leu Asn Met Glu Leu Thr Ile Pro Ile Phe Ala
                85                  90                  95

Thr Asn Ser Asp Cys Glu Leu Ile Val Lys Ala Met Gln Gly Leu Leu
            100                 105                 110

Lys Asp Gly Asn Pro Ile Pro Ser Ala Ile Ala Ala Asn Ser Gly Ile
        115                 120                 125

Tyr

<210>  35
<211>  129
<212>  PRT
<213>  Enterobacteria phage GA

<400>  35

Ala Thr Leu Arg Ser Phe Val Leu Val Asp Asn Gly Gly Thr Gly Asn
1               5                   10                  15

Val Thr Val Val Pro Val Ser Asn Ala Asn Gly Val Ala Glu Trp Leu
            20                  25                  30

Ser Asn Asn Ser Arg Ser Gln Ala Tyr Arg Val Thr Ala Ser Tyr Arg
        35                  40                  45

Ala Ser Gly Ala Asp Lys Arg Lys Tyr Thr Ile Lys Leu Glu Val Pro
    50                  55                  60

Lys Ile Val Thr Gln Val Val Asn Gly Val Glu Leu Pro Gly Ser Ala
65                  70                  75                  80

Trp Lys Ala Tyr Ala Ser Ile Asp Leu Thr Ile Pro Ile Phe Ala Ala
                85                  90                  95

Thr Asp Asp Val Thr Val Ile Ser Lys Ser Leu Ala Gly Leu Phe Lys
            100                 105                 110

Val Gly Asn Pro Ile Ala Glu Ala Ile Ser Ser Gln Ser Gly Phe Tyr
        115                 120                 125

Ala

<210>  36
<211>  129
<212>  PRT
<213>  Enterobacteria phage FR

<400>  36

Ala Ser Asn Phe Glu Glu Phe Val Leu Val Asp Asn Gly Gly Thr Gly
1               5                   10                  15

Asp Val Lys Val Ala Pro Ser Asn Phe Ala Asn Gly Val Ala Glu Trp
            20                  25                  30

Ile Ser Ser Asn Ser Arg Ser Gln Ala Tyr Lys Val Thr Cys Ser Val
        35                  40                  45

Arg Gln Ser Ser Ala Asn Asn Arg Lys Tyr Thr Val Lys Val Glu Val
    50                  55                  60

Pro Lys Val Ala Thr Gln Val Gln Gly Gly Val Glu Leu Pro Val Ala
65                  70                  75                  80

Ala Trp Arg Ser Tyr Met Asn Met Glu Leu Thr Ile Pro Val Phe Ala
                85                  90                  95

Thr Asn Asp Asp Cys Ala Leu Ile Val Lys Ala Leu Gln Gly Thr Phe
            100                 105                 110

Lys Thr Gly Asn Pro Ile Ala Thr Ala Ile Ala Ala Asn Ser Gly Ile
        115                 120                 125

Tyr

<210> 37
<211> 257
<212> PRT
<213> Enterobacteria phage MS2

<400> 37

Ala Ser Asn Phe Thr Gln Phe Val Leu Val Asp Asn Gly Gly Thr Gly
1               5                   10                  15

Asp Val Thr Val Ala Pro Ser Asn Phe Ala Asn Gly Val Ala Glu Trp
            20                  25                  30

Ile Ser Ser Asn Ser Arg Ser Gln Ala Tyr Lys Val Thr Cys Ser Val
        35                  40                  45

Arg Gln Ser Ser Ala Gln Asn Arg Lys Tyr Thr Ile Lys Val Glu Val
    50                  55                  60

Pro Lys Val Ala Thr Gln Thr Val Gly Gly Val Glu Leu Pro Val Ala
65                  70                  75                  80

Ala Trp Arg Ser Tyr Leu Asn Met Glu Leu Thr Ile Pro Ile Phe Ala
                85                  90                  95

Thr Asn Ser Asp Cys Glu Leu Ile Val Lys Ala Met Gln Gly Leu Leu
            100                 105                 110

Lys Asp Gly Asn Pro Ile Pro Ser Ala Ile Ala Ala Asn Ser Gly Ile
        115                 120                 125

Tyr Ala Asn Phe Thr Gln Phe Val Leu Val Asp Asn Gly Gly Thr Gly
    130                 135                 140

Asp Val Thr Val Ala Pro Ser Asn Phe Ala Asn Gly Val Ala Glu Trp
145                 150                 155                 160

Ile Ser Ser Asn Ser Arg Ser Gln Ala Tyr Lys Val Thr Cys Ser Val
                165                 170                 175

Arg Gln Ser Ser Ala Gln Asn Arg Lys Tyr Thr Ile Lys Val Glu Val
            180                 185                 190

Pro Lys Val Ala Thr Gln Thr Val Gly Gly Val Glu Leu Pro Val Ala
        195                 200                 205

Ala Trp Arg Ser Tyr Leu Asn Met Glu Leu Thr Ile Pro Ile Phe Ala
    210                 215                 220

Thr Asn Ser Asp Cys Glu Leu Ile Val Lys Ala Met Gln Gly Leu Leu
225                 230                 235                 240

Lys Asp Gly Asn Pro Ile Pro Ser Ala Ile Ala Ala Asn Ser Gly Ile
                245                 250                 255

Tyr

<210>  38
<211>  130
<212>  PRT
<213>  Enterobacteria phage MS2

<400>  38

Met Ala Ser Asn Phe Thr Gln Phe Val Leu Val Asp Asn Gly Gly Thr
1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Val Pro Lys Val Ala Thr Gln Thr Val Gly Gly Val Glu Leu Pro Val
65                  70                  75                  80

Ala Ala Trp Arg Ser Tyr Leu Asn Leu Glu Leu Thr Ile Pro Ile Phe
                85                  90                  95

Ala Thr Asn Ser Asp Cys Glu Leu Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Tyr
    130

<210>  39
<211>  120
<212>  PRT

<213>  Enterobacteria phage MS2

<400>  39

Met Ala Ser Asn Phe Thr Gln Phe Val Leu Val Asp Asn Gly Gly Thr
1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Val Pro Lys Val Ala Thr Gln Thr Val Gly Gly Val Glu Leu Pro Val
65                  70                  75                  80

Ala Ala Trp Arg Ser Tyr Leu Asn Leu Glu Leu Thr Ile Pro Ile Phe
                85                  90                  95

Ala Thr Asn Ser Asp Cys Glu Leu Ile Val Lys Ala Met Gln Gly Leu
            100                 105                 110

Leu Lys Asp Gly Asn Pro Ile Pro
        115                 120


<210>  40
<211>  130
<212>  PRT
<213>  Enterobacteria phage MS2

<400>  40

Met Ala Ser Asn Phe Thr Gln Phe Val Leu Val Asp Asn Gly Gly Thr
1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Leu Pro Lys Val Ala Thr Gln Thr Val Gly Gly Val Glu Leu Pro Val
65                  70                  75                  80

Ala Ala Trp Arg Ser Tyr Leu Asn Met Glu Leu Thr Ile Pro Ile Phe
                85                  90                  95

Ala Thr Asn Ser Asp Cys Glu Leu Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Tyr
    130

<210>  41
<211>  130
<212>  PRT
<213>  Enterobacteria phage MS2

<400>  41

Met Ala Ser Asn Phe Thr Gln Phe Val Leu Val Asp Asn Gly Gly Thr
1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Val Pro Lys Val Ala Thr Gln Thr Val Gly Gly Val Glu Leu Pro Val
65                  70                  75                  80

Ala Ala Trp Arg Ser Tyr Leu Asn Val Glu Leu Thr Ile Pro Ile Phe
                85                  90                  95

Ala Thr Asn Ser Asp Cys Glu Leu Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Tyr
    130

<210>  42
<211>  130
<212>  PRT
<213>  Enterobacteria phage MS2

<400>  42

Met Ala Ser Asn Phe Thr Gln Phe Val Leu Val Asp Asn Gly Gly Thr
1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Val Pro Lys Val Ala Thr Gln Thr Val Gly Gly Val Glu Leu Pro Val
65                  70                  75                  80

Ala Ala Trp Arg Ser Tyr Leu Asn Met Glu Leu Thr Ile Pro Ile Phe
                85                  90                  95

Ala Thr Asn Pro Asp Cys Glu Leu Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Tyr
    130

<210>  43
<211>  130
<212>  PRT
<213>  Enterobacteria phage MS2

<400>  43

Met Ala Ser Asn Phe Thr Gln Phe Val Leu Val Asp Asn Gly Gly Thr
1               5                   10                  15

Gly Asp Val Ala Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Val Pro Lys Val Ala Thr Gln Thr Val Gly Gly Val Glu Leu Pro Val

65                  70                  75                  80

Ala Ala Trp Arg Ser Tyr Leu Asn Met Glu Leu Thr Ile Pro Ile Phe
                85                  90                  95

Ala Thr Asn Ser Asp Cys Glu Leu Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Tyr
    130

<210>  44
<211>  130
<212>  PRT
<213>  Enterobacteria phage MS2

<220>
<221>  misc_feature
<222>  (50)..(50)
<223>  Xaa can be any naturally occurring amino acid

<400>  44

Met Ala Ser Asn Phe Thr Gln Phe Val Leu Val Asp Asn Gly Gly Thr
1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Xaa Gln Ser Ser Ala Gln Asn Arg Lys Tyr Thr Ile Lys Val Glu
    50                  55                  60

Val Pro Lys Val Ala Thr Gln Thr Val Gly Gly Val Glu Leu Pro Val
65                  70                  75                  80

Ala Ala Trp Arg Ser Tyr Leu Asn Met Glu Leu Thr Ile Pro Ile Phe
                85                  90                  95

Ala Thr Asn Ser Asp Cys Glu Leu Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Tyr
    130

<210>  45
<211>  130
<212>  PRT
<213>  Enterobacteria phage MS2

<220>
<221>  misc_feature
<222>  (7)..(7)
<223>  Xaa can be any naturally occurring amino acid

<400>  45

Met Ala Ser Asn Phe Thr Xaa Phe Val Leu Val Asp Asn Gly Gly Thr
1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Val Pro Lys Val Ala Thr Gln Thr Val Gly Gly Val Glu Leu Pro Val
65                  70                  75                  80

Ala Ala Trp Arg Ser Tyr Leu Asn Met Glu Leu Thr Ile Pro Ile Phe
                85                  90                  95

Ala Thr Asn Ser Asp Cys Glu Leu Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Tyr
    130

<210>  46
<211>  130
<212>  PRT
<213>  Enterobacteria phage BO1

<400>  46

Met Ala Ser Asn Phe Thr Gln Phe Val Leu Val Asp Asn Gly Gly Thr
1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Val Pro Lys Val Ala Thr Gln Thr Val Gly Gly Val Glu Leu Pro Val
65                  70                  75                  80

Ala Ala Trp Arg Ser Tyr Leu Asn Met Glu Leu Thr Ile Pro Ile Phe
                85                  90                  95

Ala Thr Asn Ser Asp Cys Glu Leu Ile Val Lys Ala Met Gln Gly Pro
            100                 105                 110

Leu Lys Asp Gly Asn Pro Ile Pro Ser Ala Ile Ala Ala Asn Ser Gly
        115                 120                 125

Ile Tyr
    130

<210>  47
<211>  130
<212>  PRT
<213>  Enterobacteria phage MS2

<400>  47

Met Ala Ser Asn Phe Thr Gln Phe Val Leu Val Asp Asn Asp Gly Thr
1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Val Pro Lys Val Ala Thr Gln Thr Val Gly Gly Val Glu Leu Pro Val
65                  70                  75                  80

Ala Ala Trp Arg Ser Tyr Leu Asn Met Glu Leu Thr Ile Pro Ile Phe
                85                  90                  95

Ala Thr Asn Ser Asp Cys Glu Leu Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Tyr
    130

<210> 48
<211> 130
<212> PRT
<213> Enterobacteria phage MS2

<220>
<221> misc_feature
<222> (18)..(18)
<223> Xaa can be any naturally occurring amino acid

<400> 48

Met Ala Ser Asn Phe Thr Gln Phe Val Leu Val Asp Asn Gly Gly Thr
1               5                   10                  15

Gly Xaa Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Val Pro Lys Val Ala Thr Gln Thr Val Gly Gly Val Glu Leu Pro Val
65                  70                  75                  80

Ala Ala Trp Arg Ser Tyr Leu Asn Met Glu Leu Thr Ile Pro Ile Phe
                85                  90                  95

Ala Thr Asn Ser Asp Cys Glu Leu Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Tyr
    130

<210>  49
<211>  130
<212>  PRT
<213>  Enterobacteria phage MS2

<400>  49

Met Ala Ser Asn Phe Thr Gln Phe Val Leu Val Asp Asn Gly Gly Thr
1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Val Pro Lys Val Ala Thr Gln Thr Val Gly Gly Val Glu Leu Pro Val
65                  70                  75                  80

Ala Ala Trp Arg Ser Tyr Leu Asn Leu Glu Leu Thr Ile Pro Ile Phe
                85                  90                  95

Ala Thr Asn Pro Asp Cys Glu Leu Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Tyr
    130


<210>  50
<211>  130
<212>  PRT
<213>  Enterobacteria phage MS2

<400>  50

Met Ala Ser Asn Phe Thr Gln Phe Val Leu Val Asp Asn Gly Gly Thr
1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu

50                  55                  60

Val Pro Lys Val Ala Thr Gln Thr Val Gly Gly Val Glu Leu Pro Val
65                  70                  75                  80

Ala Ala Trp Arg Ser Tyr Leu Asn Met Glu Leu Thr Ile Pro Ile Phe
                85                  90                  95

Ala Thr Asn Ser Asp Cys Glu Leu Ile Val Lys Ala Met Gln Gly Leu
            100                 105                 110

Leu Lys Asp Gly Asn Pro Ile Ser Ser Ala Ile Ala Ala Asn Ser Gly
        115                 120                 125

Ile Tyr
    130

<210>  51
<211>  130
<212>  PRT
<213>  Enterobacteria phage MS2

<220>
<221>  misc_feature
<222>  (104)..(104)
<223>  Xaa can be any naturally occurring amino acid

<400>  51

Met Ala Ser Asn Phe Thr Gln Phe Val Leu Val Asp Asn Gly Gly Thr
1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Val Pro Lys Val Ala Thr Gln Thr Val Gly Gly Val Glu Leu Pro Val
65                  70                  75                  80

Ala Ala Trp Arg Ser Tyr Leu Asn Val Glu Leu Thr Ile Pro Ile Phe
                85                  90                  95

Ala Thr Asn Ser Asp Cys Glu Xaa Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Tyr
    130

<210>  52
<211>  130
<212>  PRT
<213>  Enterobacteria phage MS12

<220>
<221>  misc_feature
<222>  (22)..(22)
<223>  Xaa can be any naturally occurring amino acid

<400>  52

Met Ala Ser Asn Phe Thr Gln Phe Val Leu Val Asp Asn Gly Gly Thr
1               5                   10                  15

Gly Asp Val Thr Val Xaa Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Val Pro Lys Val Ala Thr Gln Thr Val Gly Gly Val Glu Leu Pro Val
65                  70                  75                  80

Ala Ala Trp Arg Ser Tyr Leu Asn Met Glu Leu Thr Ile Pro Ile Phe
                85                  90                  95

Ala Thr Asn Ser Asp Cys Ala Leu Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Tyr
    130

<210>  53
<211>  130
<212>  PRT
<213>  Enterobacteria phage MS2

<220>
<221>  misc_feature
<222>  (130)..(130)
<223>  Xaa can be any naturally occurring amino acid

<400>  53

Met Ala Ser Asn Phe Thr Gln Phe Val Leu Val Asp Asn Gly Gly Thr
1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Val Pro Lys Val Ala Thr Gln Thr Val Gly Gly Val Glu Leu Pro Val
65                  70                  75                  80

Ala Ala Trp Arg Ser Tyr Leu Asn Val Glu Leu Thr Ile Pro Ile Phe
                85                  90                  95

Ala Thr Asn Ser Asp Cys Glu Leu Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Xaa
    130

<210>  54
<211>  130
<212>  PRT
<213>  Enterobacteria phage MS2

<220>
<221>  misc_feature
<222>  (130)..(130)
<223>  Xaa can be any naturally occurring amino acid

<400>  54

Met Ala Ser Asn Phe Thr Gln Phe Val Leu Val Asp Asn Gly Gly Thr
1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Val Pro Lys Val Ala Thr Gln Thr Val Gly Gly Val Glu Leu Pro Val
65                  70                  75                  80

Ala Ala Trp Arg Ser Tyr Leu Asn Val Glu Leu Thr Ile Pro Ile Phe
                85                  90                  95

Ala Thr Asn Ser Asp Cys Glu Leu Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Xaa
    130

<210>  55
<211>  130
<212>  PRT
<213>  Enterobacteria phage MS2

<400>  55

Met Ala Ser Asn Phe Thr Gln Phe Val Leu Val Asp Asn Gly Gly Thr
1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Val Pro Lys Val Ala Thr Gln Thr Val Gly Gly Val Gln Leu Pro Val
65                  70                  75                  80

Ala Ala Trp Arg Ser Tyr Leu Asn Met Glu Leu Thr Ile Pro Ile Phe
                85                  90                  95

Ala Thr Asn Asp Asp Cys Ala Leu Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Tyr
    130

<210>  56
<211>  130
<212>  PRT
<213>  Enterobacteria phage MS2

<400>  56

Met Ala Ser Asn Phe Thr Gln Phe Val Leu Val Asp Asn Gly Gly Thr
1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Val Pro Lys Val Ala Thr Gln Thr Val Gly Gly Val Gln Leu Pro Val
65                  70                  75                  80

Ala Ala Trp Arg Ser Tyr Leu Asn Met Glu Leu Thr Ile Pro Ile Phe
                85                  90                  95

Ala Thr Asn Asp Asp Cys Ala Leu Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Tyr
    130

<210>  57
<211>  127
<212>  PRT
<213>  Enterobacteria phage MS2

<400>  57

Asn Phe Thr Gln Phe Val Leu Val Asp Asn Gly Gly Thr Gly Asp Val
1               5                   10                  15

Thr Val Ala Pro Ser Asn Phe Ala Asn Gly Val Ala Glu Trp Ile Ser
            20                  25                  30

Ser Asn Ser Arg Ser Gln Ala Tyr Lys Val Thr Cys Ser Val Arg Gln
        35                  40                  45

Ser Ser Ala Gln Asn Arg Lys Tyr Thr Ile Lys Val Glu Val Pro Lys
    50                  55                  60

Val Ala Thr Gln Thr Val Gly Gly Val Glu Leu Pro Val Ala Ala Trp
65                  70                  75                  80

Arg Ser Tyr Leu Asn Val Glu Leu Thr Ile Pro Ile Phe Ala Thr Asn
                85                  90                  95

Ser Asp Cys Glu Leu Ile Val Lys Ala Met Gln Gly Leu Leu Lys Asp
            100                 105                 110

Gly Asn Pro Ile Pro Ser Ala Ile Ala Ala Asn Ser Gly Ile Tyr
        115                 120                 125

<210>  58
<211>  129
<212>  PRT
<213>  Enterobacteria phage ZR

<400>  58

Ala Ser Asn Phe Thr Gln Phe Val Leu Val Asn Asp Gly Gly Thr Gly
1               5                   10                  15

Asn Val Thr Val Ala Pro Ser Asn Phe Ala Asn Gly Val Ala Glu Trp
            20                  25                  30

Ile Ser Ser Asn Ser Arg Ser Gln Ala Tyr Lys Val Thr Cys Ser Val
        35                  40                  45

Arg Gln Ser Ser Ala Gln Asn Arg Lys Tyr Thr Ile Lys Val Glu Val
    50                  55                  60

Pro Lys Val Ala Thr Gln Thr Val Gly Gly Val Glu Leu Pro Val Ala
65                  70                  75                  80

Ala Trp Arg Ser Tyr Leu Asn Met Glu Leu Thr Ile Pro Ile Phe Ala
                85                  90                  95

Thr Asn Ser Asp Cys Glu Leu Ile Val Lys Ala Met Gln Gly Leu Leu
            100                 105                 110

Lys Asp Gly Asn Pro Ile Pro Ser Ala Ile Ala Ala Asn Ser Gly Ile
        115                 120                 125

Tyr

<210>  59
<211>  129
<212>  PRT
<213>  Enterobacteria phage R17

<400>  59

Ala Ser Asn Phe Thr Gln Phe Val Leu Val Asn Asp Gly Gly Thr Gly
1               5                   10                  15

Asn Val Thr Val Ala Pro Ser Asn Phe Ala Asn Gly Val Ala Glu Trp
            20                  25                  30

Ile Ser Ser Asn Ser Arg Ser Gln Ala Tyr Lys Val Thr Cys Ser Val
        35                  40                  45

Arg Gln Ser Ser Ala Gln Asn Arg Lys Tyr Thr Ile Lys Val Glu Val
    50                  55                  60

Pro Lys Val Ala Thr Gln Thr Val Gly Gly Val Glu Leu Pro Val Ala
65                  70                  75                  80

Ala Trp Arg Ser Tyr Leu Asn Met Glu Leu Thr Ile Pro Ile Phe Ala
                85                  90                  95

Thr Asn Ser Asp Cys Glu Leu Ile Val Lys Ala Met Gln Gly Leu Leu
            100                 105                 110

Lys Asp Gly Asn Pro Ile Pro Ser Ala Ile Ala Ala Asn Ser Gly Ile
        115                 120                 125

Tyr

<210>  60
<211>  130
<212>  PRT
<213>  Enterobacteria phage MS2

<400>  60

Met Ala Ser Asn Phe Thr Gln Phe Val Leu Val Asp Asn Gly Gly Thr
1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Val Pro Lys Val Ala Thr Gln Thr Val Gly Gly Val Glu Leu Pro Val
65                  70                  75                  80

Ala Ala Trp Arg Ser Tyr Leu Asn Met Glu Leu Thr Ile Pro Ile Phe
                85                  90                  95

Ala Thr Asn Ser Asp Cys Glu Leu Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Tyr
    130

<210>  61
<211>  130
<212>  PRT
<213>  Enterobacteria phage MS2

<400>  61

Met Ala Ser Asn Phe Thr Gln Phe Val Leu Val Asp Asn Gly Gly Thr
1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Val Pro Lys Val Ala Thr Gln Thr Val Gly Gly Val Glu Leu Pro Val
65                  70                  75                  80

Ala Ala Trp Arg Ser Tyr Leu Asn Met Glu Leu Thr Ile Pro Ile Phe
                85                  90                  95

Ala Thr Asn Ser Asp Cys Glu Leu Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Tyr
    130

<210>  62
<211>  130
<212>  PRT
<213>  Enterobacteria phage MS2

<400>  62

Met Ala Ser Asn Phe Thr Gln Phe Val Leu Val Asp Asn Gly Gly Thr
1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Val Pro Lys Val Ala Thr Gln Thr Val Gly Gly Val Glu Leu Pro Val
65                  70                  75                  80

Ala Ala Trp Arg Ser Tyr Leu Asn Met Glu Leu Thr Ile Pro Ile Phe
                85                  90                  95

Ala Thr Asn Ser Asp Cys Glu Leu Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Tyr
    130

<210>  63
<211>  130
<212>  PRT
<213>  Enterobacteria phage ZR

<400>  63

Met Ala Ser Asn Phe Thr Gln Phe Val Leu Val Asp Asn Gly Gly Thr

1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Val Pro Lys Val Ala Thr Gln Thr Val Gly Gly Val Glu Leu Pro Val
65                  70                  75                  80

Ala Ala Trp Arg Ser Tyr Leu Asn Met Glu Leu Thr Ile Pro Ile Phe
                85                  90                  95

Ala Thr Asn Ser Asp Cys Glu Leu Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Tyr
    130

<210>  64
<211>  130
<212>  PRT
<213>  Enterobacteria phage JP501

<400>  64

Met Ala Ser Asn Phe Thr Glu Phe Val Leu Val Asp Asn Gly Glu Thr
1               5                   10                  15

Gly Asn Val Thr Val Ala Pro Ser Asn Phe Ala Asn Gly Val Ala Glu
            20                  25                  30

Trp Ile Ser Ser Asp Ser Arg Ser Gln Ala Tyr Lys Val Thr Cys Ser
        35                  40                  45

Val Arg Gln Ser Ser Ala Gln Asn Arg Lys Tyr Thr Ile Lys Val Ala
    50                  55                  60

Val Pro Lys Val Ala Thr Gln Thr Val Gly Gly Val Glu Leu Pro Val
65                  70                  75                  80

Ala Ala Trp Arg Ser Tyr Leu Asn Met Glu Leu Thr Ile Pro Ile Phe

85                  90                  95

Ala Thr Asn Ser Asp Cys Ala Leu Ile Val Lys Ala Met Gln Gly Leu
            100                 105                 110

Leu Lys Asp Gly Asn Pro Ile Pro Ser Ala Ile Ala Ala Asn Ser Gly
        115                 120                 125

Ile Tyr
    130

<210>  65
<211>  129
<212>  PRT
<213>  Enterobacteria phage F2

<400>  65

Ala Ser Asn Phe Thr Gln Phe Val Leu Val Asn Asp Gly Gly Thr Gly
1               5                   10                  15

Asn Val Thr Val Ala Pro Ser Asn Phe Ala Asn Gly Val Ala Glu Trp
            20                  25                  30

Ile Ser Ser Asn Ser Arg Ser Gln Ala Tyr Lys Val Thr Cys Ser Val
        35                  40                  45

Arg Gln Ser Ser Ala Gln Asn Arg Lys Tyr Thr Ile Lys Val Glu Val
    50                  55                  60

Pro Lys Val Ala Thr Gln Thr Val Gly Gly Val Glu Leu Pro Val Ala
65                  70                  75                  80

Ala Trp Arg Ser Tyr Leu Asn Leu Glu Leu Thr Ile Pro Ile Phe Ala
                85                  90                  95

Thr Asn Ser Asp Cys Glu Leu Ile Val Lys Ala Met Gln Gly Leu Leu
            100                 105                 110

Lys Asp Gly Asn Pro Ile Pro Ser Ala Ile Ala Ala Asn Ser Gly Ile
        115                 120                 125

Tyr

<210>  66
<211>  130
<212>  PRT
<213>  Enterobacteria phage JP34

<400>  66

Met Ala Thr Leu Arg Ser Phe Val Leu Val Asp Asn Gly Gly Thr Gly
1               5                   10                  15

Asp Val Thr Val Val Pro Val Ser Asn Ala Asn Gly Val Ala Glu Trp
            20                  25                  30

Leu Ser Asn Asn Ser Arg Ser Gln Ala Tyr Arg Val Thr Ala Ser Tyr
        35                  40                  45

Arg Ala Ser Gly Ala Asp Lys Arg Lys Tyr Thr Ile Lys Leu Glu Val
    50                  55                  60

Pro Lys Ile Val Thr Gln Val Val Asn Gly Val Glu Leu Pro Val Ser
65                  70                  75                  80

Ala Trp Lys Ala Tyr Ala Ser Ile Asp Leu Thr Ile Pro Ile Phe Ala
                85                  90                  95

Ala Thr Asp Asp Val Thr Val Ile Ser Lys Ser Leu Ala Gly Leu Phe
            100                 105                 110

Lys Val Gly Asn Pro Ile Ala Asp Ala Ile Ser Ser Gln Ser Gly Phe
        115                 120                 125

Tyr Ala
    130


<210>  67
<211>  130
<212>  PRT
<213>  Enterobacteria phage JP500

<400>  67

Met Ala Thr Leu Arg Ser Phe Val Leu Val Asp Asn Gly Gly Thr Gly
1               5                   10                  15

Asp Val Thr Val Val Pro Val Ser Asn Ala Asn Gly Val Ala Glu Trp
            20                  25                  30

Leu Ser Asn Asn Ser Arg Ser Gln Ala Tyr Arg Val Thr Ala Ser Tyr
        35                  40                  45

Arg Ala Ser Gly Ala Asp Lys Arg Lys Tyr Thr Ile Lys Leu Glu Val
    50                  55                  60

Pro Lys Ile Val Thr Gln Val Val Asn Gly Val Glu Leu Pro Val Ser
65                  70                  75                  80

Ala Trp Lys Ala Tyr Ala Ser Ile Asp Leu Thr Ile Pro Ile Phe Ala
                85                  90                  95

Ala Thr Asp Asp Val Thr Val Ile Ser Lys Ser Leu Ala Gly Leu Phe
            100                 105                 110

Lys Val Gly Asn Pro Ile Ala Asp Ala Ile Ser Ser Gln Ser Gly Phe
        115                 120                 125

Tyr Ala
    130

<210>  68
<211>  130
<212>  PRT
<213>  Enterobacteria phage SD

<400>  68

Met Ala Thr Leu Arg Ser Phe Val Leu Val Asp Asn Gly Gly Thr Gly
1               5                   10                  15

Asn Val Thr Val Val Pro Val Ser Asn Ala Asn Gly Val Ala Glu Trp
            20                  25                  30

Leu Ser Asn Asn Ser Arg Ser Gln Ala Tyr Arg Val Thr Ala Ser Tyr
        35                  40                  45

Arg Ala Ser Gly Ala Asp Lys Arg Lys Tyr Thr Ile Lys Leu Glu Val
    50                  55                  60

Pro Lys Ile Val Thr Gln Val Val Asn Gly Val Glu Leu Pro Ile Ser
65                  70                  75                  80

Ala Trp Lys Ala Tyr Ala Ser Ile Asp Leu Thr Ile Pro Ile Phe Ala
                85                  90                  95

Ala Thr Asp Asp Val Thr Thr Ile Ser Lys Ser Leu Ala Gly Leu Phe
            100                 105                 110

Lys Val Gly Asn Pro Ile Ala Asp Ala Ile Ser Ser Gln Ser Gly Phe
        115                 120                 125

Tyr Ala
    130

<210>  69
<211>  130

<212>   PRT
<213>   Enterobacteria phage KU1

<400>   69

Met Ala Thr Leu Arg Ser Phe Val Leu Val Asp Asn Gly Gly Thr Gly
1               5                   10                  15

Asn Val Thr Val Val Pro Val Ser Asn Ala Asn Gly Val Ala Glu Trp
            20                  25                  30

Leu Ser Asn Asn Ser Arg Ser Gln Ala Tyr Arg Val Thr Ala Ser Tyr
        35                  40                  45

Arg Ala Ser Gly Ala Asp Lys Arg Lys Tyr Thr Ile Lys Leu Glu Val
    50                  55                  60

Pro Lys Ile Val Thr Gln Ser Val Asn Gly Val Glu Leu Pro Val Ser
65                  70                  75                  80

Ala Trp Lys Ala Phe Ala Ser Ile Asp Leu Thr Ile Pro Ile Phe Ala
                85                  90                  95

Ala Thr Asp Asp Val Thr Leu Ile Ser Lys Ser Leu Ala Gly Leu Phe
            100                 105                 110

Lys Ile Gly Asn Pro Val Ala Asp Ala Ile Ser Ser Gln Ser Gly Phe
        115                 120                 125

Tyr Ala
    130


<210>   70
<211>   130
<212>   PRT
<213>   Enterobacteria phage GA

<400>   70

Met Ala Thr Leu Arg Ser Phe Val Leu Val Asp Asn Gly Gly Thr Gly
1               5                   10                  15

Asn Val Thr Val Val Pro Val Ser Asn Ala Asn Gly Val Ala Glu Trp
            20                  25                  30

Leu Ser Asn Asn Ser Arg Ser Gln Ala Tyr Arg Val Thr Ala Ser Tyr
        35                  40                  45

Arg Ala Ser Gly Ala Asp Lys Arg Lys Tyr Ala Ile Lys Leu Glu Val
    50                  55                  60

Pro Lys Ile Val Thr Gln Val Val Asn Gly Val Glu Leu Pro Gly Ser
65                  70                  75                  80

Ala Trp Lys Ala Tyr Ala Ser Ile Asp Leu Thr Ile Pro Ile Phe Ala
                85                  90                  95

Ala Thr Asp Asp Val Thr Val Ile Ser Lys Ser Leu Ala Gly Leu Phe
            100                 105                 110

Lys Val Gly Asn Pro Ile Ala Glu Ala Ile Ser Ser Gln Ser Gly Phe
        115                 120                 125

Tyr Ala
    130

<210>  71
<211>  130
<212>  PRT
<213>  Enterobacteria phage BZ13

<400>  71

Met Ala Thr Leu Arg Ser Phe Val Leu Val Asp Asn Gly Gly Thr Gly
1               5                   10                  15

Asn Val Thr Val Val Pro Val Ser Asn Ala Asn Gly Val Ala Glu Trp
            20                  25                  30

Leu Ser Asn Asn Ser Arg Ser Gln Ala Tyr Arg Val Thr Ala Ser Tyr
        35                  40                  45

Arg Ala Ser Gly Ala Asp Lys Arg Lys Tyr Thr Ile Lys Leu Glu Val
    50                  55                  60

Pro Lys Ile Val Thr Gln Val Val Asn Gly Val Glu Leu Pro Val Ser
65                  70                  75                  80

Ala Trp Lys Ala Tyr Ala Ser Ile Asp Leu Thr Ile Pro Ile Phe Ala
                85                  90                  95

Ala Thr Asp Asp Val Thr Val Ile Ser Lys Ser Leu Ala Gly Leu Phe
            100                 105                 110

Lys Val Gly Asn Pro Ile Ala Glu Ala Ile Ser Ser Gln Ser Gly Phe
        115                 120                 125

Tyr Ala
    130

<210>  72
<211>  130
<212>  PRT
<213>  Enterobacteria phage BZ13

<400>  72

Met Ala Thr Leu Arg Ser Phe Val Leu Val Asp Asn Gly Gly Thr Gly
1               5                   10                  15

Asn Val Thr Val Val Pro Val Ser Asn Ala Asn Gly Val Ala Glu Trp
            20                  25                  30

Leu Ser Asn Asn Ser Arg Ser Gln Ala Tyr Arg Val Thr Ala Ser Tyr
        35                  40                  45

Arg Ala Ser Gly Ala Asp Lys Arg Lys Tyr Thr Ile Lys Leu Glu Val
    50                  55                  60

Pro Lys Ile Val Thr Gln Thr Val Asn Gly Val Glu Leu Pro Val Ser
65                  70                  75                  80

Ala Trp Lys Ala Tyr Ala Ser Ile Asp Leu Thr Ile Pro Ile Phe Ala
                85                  90                  95

Ala Thr Asp Asp Val Thr Leu Ile Ser Lys Ser Leu Ala Gly Leu Phe
            100                 105                 110

Lys Ile Gly Asn Pro Val Ala Asp Ala Ile Ser Ser Gln Ser Gly Phe
        115                 120                 125

Tyr Ala
    130


<210>  73
<211>  130
<212>  PRT
<213>  Enterobacteria phage BZ13

<400>  73

Met Ala Thr Leu Arg Ser Phe Val Leu Val Asp Asn Gly Gly Thr Gly
1               5                   10                  15

Asn Val Thr Val Val Pro Val Ser Asn Ala Asn Gly Val Ala Glu Trp
            20                  25                  30

Leu Ser Asn Asn Ser Arg Ser Gln Ala Tyr Arg Val Thr Ala Ser Tyr
        35                  40                  45

Arg Ala Ser Gly Ala Asp Lys Arg Lys Tyr Thr Ile Lys Leu Glu Val
    50                  55                  60

Pro Lys Ile Val Thr Gln Val Val Asn Gly Val Glu Leu Pro Val Ser
65                  70                  75                  80

Ala Trp Lys Ala Tyr Ala Ser Ile Asp Leu Thr Ile Pro Ile Phe Ala
                85                  90                  95

Ala Thr Asp Asp Val Thr Val Ile Ser Lys Ser Leu Ala Gly Leu Phe
            100                 105                 110

Lys Val Gly Asp Pro Ile Ala Asp Ala Ile Ser Ser Gln Ser Gly Phe
        115                 120                 125

Tyr Ala
    130

<210>  74
<211>  130
<212>  PRT
<213>  Enterobacteria phage TH1

<400>  74

Met Ala Thr Leu Arg Ser Phe Val Leu Val Asp Asn Gly Gly Thr Gly
1               5                   10                  15

Asn Val Thr Val Val Pro Val Ser Asn Ala Asn Gly Val Ala Glu Trp
            20                  25                  30

Leu Ser Asn Asn Ser Arg Ser Gln Ala Tyr Arg Val Thr Ala Ser Tyr
        35                  40                  45

Arg Ala Ser Gly Ala Asp Lys Arg Lys Tyr Thr Ile Lys Leu Glu Val
    50                  55                  60

Pro Lys Ile Val Thr Gln Val Val Asn Gly Val Glu Leu Pro Val Ser
65                  70                  75                  80

Ala Trp Lys Ala Tyr Ala Ser Ile Asp Leu Thr Ile Pro Ile Phe Ala
                85                  90                  95

Ala Thr Asp Asp Val Thr Val Ile Ser Lys Ser Leu Ala Gly Leu Phe
            100                 105                 110

Lys Val Gly Asn Pro Ile Ala Asp Ala Ile Ser Ser Gln Ser Gly Phe
        115                 120                 125

Tyr Ala
    130

<210>  75
<211>  130
<212>  PRT
<213>  Enterobacteria phage T12

<400>  75

Met Ala Thr Leu Arg Ser Phe Val Leu Val Asp Asn Gly Gly Thr Gly
1               5                   10                  15

Asn Val Thr Val Val Pro Val Ser Asn Ala Asn Gly Val Ala Glu Trp
            20                  25                  30

Leu Ser Asn Asn Ser Arg Ser Gln Ala Tyr Arg Val Thr Ala Ser Tyr
        35                  40                  45

Arg Ala Ser Gly Ala Asp Lys Arg Lys Tyr Thr Ile Lys Leu Glu Val
    50                  55                  60

Pro Lys Ile Val Thr Gln Val Val Asn Gly Val Glu Leu Pro Val Ser
65                  70                  75                  80

Ala Trp Lys Ala Tyr Ala Ser Ile Asp Leu Thr Ile Pro Ile Phe Ala
                85                  90                  95

Ala Thr Asp Asp Val Thr Val Ile Ser Lys Ser Leu Ala Gly Leu Phe
            100                 105                 110

Lys Val Gly Asn Pro Ile Ala Asp Ala Ile Ser Ser Gln Ser Gly Phe
        115                 120                 125

Tyr Ala
    130

<210>  76
<211>  130
<212>  PRT
<213>  Enterobacteria phage FR

<400>  76

Met Ala Ser Asn Phe Glu Glu Phe Val Leu Val Asp Asn Gly Gly Thr
1               5                   10                  15

Gly Asp Val Lys Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser

35                  40                  45

Val Arg Gln Ser Ser Ala Asn Asn Arg Lys Tyr Thr Val Lys Val Glu
    50                  55                  60

Val Pro Lys Val Ala Thr Gln Val Gln Gly Gly Val Glu Leu Pro Val
65                  70                  75                  80

Ala Ala Trp Arg Ser Tyr Met Asn Met Glu Leu Thr Ile Pro Val Phe
                85                  90                  95

Ala Thr Asn Asp Asp Cys Ala Leu Ile Val Lys Ala Leu Gln Gly Thr
            100                 105                 110

Phe Lys Thr Gly Asn Pro Ile Ala Thr Ala Ile Ala Ala Asn Ser Gly
        115                 120                 125

Ile Tyr
    130

<210>  77
<211>  130
<212>  PRT
<213>  Enterobacteria phage R17

<400>  77

Met Ala Ser Asn Phe Thr Gln Phe Val Leu Val Asp Asn Gly Gly Thr
1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45

Val Arg Gln Ser Ser Ala Gln Asn Arg Lys Tyr Thr Ile Lys Val Glu
    50                  55                  60

Val Pro Lys Val Ala Thr Gln Thr Val Gly Gly Val Glu Leu Pro Val
65                  70                  75                  80

Ala Ala Trp Arg Ser Tyr Leu Asn Met Glu Leu Thr Ile Pro Ile Phe
                85                  90                  95

Ala Thr Asn Ser Asp Cys Glu Leu Ile Val Lys Ala Met Gln Gly Leu
            100                 105                 110

Leu Lys Asp Gly Asn Pro Ile Pro Ser Ala Ile Ala Ala Asn Ser Gly

115                 120                 125

Ile Tyr
    130

<210>  78
<211>  132
<212>  PRT
<213>  Enterobacteria phage Qbeta

<400>  78

Ala Lys Leu Glu Thr Val Thr Leu Gly Asn Ile Gly Lys Asp Gly Lys
1               5                   10                  15

Gln Thr Leu Val Leu Asn Pro Arg Gly Val Asn Pro Thr Asn Gly Val
            20                  25                  30

Ala Ser Leu Ser Gln Ala Gly Ala Val Pro Ala Leu Glu Lys Arg Val
        35                  40                  45

Thr Val Ser Val Ser Gln Pro Ser Arg Asn Arg Lys Asn Tyr Lys Val
    50                  55                  60

Gln Val Lys Ile Gln Asn Pro Thr Ala Cys Thr Ala Asn Gly Ser Cys
65                  70                  75                  80

Asp Pro Ser Val Thr Arg Gln Ala Tyr Ala Asp Val Thr Phe Ser Phe
                85                  90                  95

Thr Gln Tyr Ser Thr Asp Glu Glu Arg Ala Phe Val Arg Thr Glu Leu
            100                 105                 110

Ala Ala Leu Leu Ala Ser Pro Leu Leu Ile Asp Ala Ile Asp Gln Leu
        115                 120                 125

Asn Pro Ala Tyr
    130